(19) Europäisches Patentamt
European Patent Office
Office européen des brevets

(11) **EP 4 394 048 A2**

(12) **EUROPEAN PATENT APPLICATION**

(43) Date of publication:
**03.07.2024 Bulletin 2024/27**

(21) Application number: **24175406.8**

(22) Date of filing: **10.12.2021**

(51) International Patent Classification (IPC):
***C12Q 1/6886*** (2018.01)

(52) Cooperative Patent Classification (CPC):
**C12Q 1/6886**; C12Q 2600/106; C12Q 2600/112;
C12Q 2600/118; C12Q 2600/158

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.12.2020 US 202063124617 P**

(62) Document number(s) of the earlier application(s) in
accordance with Art. 76 EPC:
**21843825.7 / 4 244 394**

(71) Applicant: **BostonGene Corporation
Waltham, MA 02453 (US)**

(72) Inventors:
• **MEERSON, Mark
Waltham, 02453 (US)**
• **KOTLOV, Nikita
4041 Limassol (CY)**
• **KUDRYASHOVA, Olga
109341 Moscow (RU)**
• **BAGAEV, Alexander
Waltham, 02451 (US)**

(74) Representative: **Mewburn Ellis LLP
Aurora Building
Counterslip
Bristol BS1 6BX (GB)**

Remarks:
This application was filed on 13-05-2024 as a
divisional application to the application mentioned
under INID code 62.

(54) **TECHNIQUES FOR IDENTIFYING FOLLICULAR LYMPHOMA TYPES**

(57) Aspects of the disclosure relate to methods, systems, computer-readable storage media, and graphical user interfaces (GUIs) that are useful for characterizing subjects having certain cancers, for example lymphomas. The disclosure is based, in part, on methods for determining the tumor microenvironment (TME) type of a lymphoma (e.g., follicular lymphoma) subject and identifying the subject's prognosis based upon the TME type determination.

FIG. 1

**Description**

CROSS REFERENCE TO RELATED APPLICATIONS

This application claims the benefit under 35 U.S.C. § 119(e) of U.S. Provisional Application No. 63/124,617, filed December 11, 2020, titled "TECHNIQUES FOR IDENTIFYING FOLLICULAR LYMPHOMA TYPES," which is incorporated by reference herein in its entirety.

BACKGROUND

Correctly characterizing the type or types of cancer a patient or subject has and, potentially, selecting one or more effective therapies for the patient can be crucial for the survival and overall wellbeing of that patient. Advances in characterizing cancers, predicting prognoses, identifying effective therapies, and otherwise aiding in personalized care of patients with cancer are needed.

SUMMARY

Aspects of the disclosure relate to methods, systems, and computer-readable storage media that can be used for determining a follicular lymphoma (FL) tumor microenvironment (TME) type for a subject. In some aspects, the disclosure provides a method for determining a follicular lymphoma (FL) tumor microenvironment (TME) type for a subject having, suspected of having, or at risk of having a follicular lymphoma (FL), comprising: using at least one computer hardware processor to perform: (a) obtaining RNA expression data for the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells; (b) generating an FL TME signature for the subject using the RNA expression data, the FL TME signature comprising a first gene expression signature comprising first gene group expression scores for respective gene groups in the first plurality of gene groups, and a second gene expression signature comprising second gene group expression scores for respective gene groups in the second plurality of gene groups associated with B cells; and (c) identifying, using the FL TME signature and from among a plurality of FL TME types, an FL TME type for the subject.

Aspects of the present disclosure include a system, comprising: at least one computer hardware processor; and at least one computer-readable storage medium storing processor-executable instructions that, when executed by the at least one computer hardware processor, cause the at least one computer hardware processor to perform a method for determining a follicular lymphoma (FL) tumor microenvironment (TME) type for a subject having, suspected of having, or at risk of having a follicular lymphoma (FL), the method comprising: (a) obtaining RNA expression data for the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells; (b) generating an FL TME signature for the subject using the RNA expression data, the FL TME signature comprising: a first gene expression signature comprising first gene group expression scores for respective gene groups in the first plurality of gene groups, and a second gene expression signature comprising second gene group expression scores for respective gene groups in the second plurality of gene groups associated with B cells, the generating comprising: determining the first gene expression signature by determining the first gene group expression scores using the first RNA expression levels, and determining the second gene expression signature by determining the second gene group expression scores using the second RNA expression levels; and (c) identifying, using the FL TME signature and from among a plurality of FL TME types, an FL TME type for the subject.

Aspects of the present disclosure include at least one computer-readable storage medium storing processor-executable instructions that, when executed by at least one computer hardware processor, cause the at least one computer hardware processor to perform a method for determining a follicular lymphoma (FL) tumor microenvironment (TME) type for a subject having, suspected of having, or at risk of having a follicular lymphoma (FL), the method comprising: (a) obtaining RNA expression data for the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells; (b) generating an FL TME signature for the subject using the RNA expression data, the FL TME signature comprising: a first gene expression signature comprising first gene group expression scores for respective gene groups in the first plurality of gene groups, and a second gene expression signature comprising second gene group expression scores for respective gene groups in the second plurality of gene groups associated with B cells, the generating comprising: determining the first gene expression signature by determining the first gene group expression scores using the first RNA expression levels, and determining the second gene expression signature by determining the second gene group expression scores using the second RNA expression levels; and (c) identifying, using the FL TME signature and from among a plurality of FL TME types, an FL TME type for the subject.

In some embodiments, the generating comprises determining the first gene expression signature by determining the first gene group expression scores using the first RNA expression levels and determining the second gene expression signature by determining the second gene group expression scores using the second RNA expression levels.

In some embodiments, obtaining the RNA expression data for the subject comprises obtaining bulk sequencing RNA data previously obtained by sequencing a biological sample obtained from the subject.

In some embodiments, the bulk sequencing data comprises at least 1 million reads, at least 5 million reads, at least 10 million reads, at least 20 million reads, at least 50 million reads, or at least 100 million reads.

In some embodiments, the sequencing data comprises bulk RNA sequencing (RNA-seq) data, single cell RNA sequencing (scRNA-seq) data, or next generation sequencing (NGS) data. In some embodiments, the sequencing data comprises microarray data.

In some embodiments, obtaining the RNA expression for the subject comprises sequencing a biological sample obtained from the subject.

In some embodiments, the method described herein further comprises normalizing the RNA expression data to transcripts per million (TPM) units prior to generating the FL TME signature.

In some embodiments, the biological sample comprises lymph node tissue of the subject. In some embodiments, the sample comprises tumor tissue of the subject.

In some embodiments, the first RNA expression levels for genes in the first plurality of gene groups comprise RNA expression levels for at least three genes from each of at least two of the following gene groups: (a) MHC II group: *HLA-DRA, HLA-DRB1, HLA-DMA, HLA-DPA1, HLA-DPB1, HLA-DMB, HLA-DQB1, HLA-DQA1, CIITA;* (b) Effector cells group: *IFNG, GZMA, GZMB, PRF1, GZMK, ZAP70,* GNLY, *FASLG, TBX21, EOMES, CD8A, CD8B;* and (c) Follicular Dendritic Cells (FDC) group: *PDPN, LTBR, FDCSP, CLU, PRNP, C4A, BST1, SERPINE2, C1S, TNFRSF1A.*

In some embodiments, the first RNA expression levels for genes in the first plurality of gene groups further comprise RNA expression levels for at least three genes from each of at least two of the following gene groups: (d) Treg cells group: *FOXP3, CTLA4, IL10, TNFRSF18, CCR8, IKZF4, IKZF2;* (e) T helper cells (Follicular B Helper T cells) group: *CXCR5, IL6, ICOS, CD40LG, CD84, IL21, BCL6, MAF, SH2D1A, IL4;* (f) Effector cells group: *IFNG, GZMA, GZMB, PRF1, GZMK, ZAP70,* GNLY, *FASLG, TBX21, EOMES, CD8A, CD8B;* (g) Follicular Dendritic Cells (FDC) group: *PDPN, LTBR, FDCSP, CLU, PRNP, C4A, BST1, SERPINE2, C1S, TNFRSF1A;* (h) Lymphatic endothelial cells group: *CCL21, CXCL12, SOX18, PPP1R13B, FLT4, PROX1, PDPN, LYVE1, FOXC2, CXADR, EDNRB, JAM2, JAM3;* (i) Proliferation rate group: *MKI67, ESCO2, CETN3, CDK2, CCND1, CCNE1, AURKA, AURKB, E2F1, MYBL2, BUB1, PLK1, CCNB1, MCM2, MCM6;* (j) M2 group: *IL10, VEGFA, TGFB1, IDO1, PTGES, MRC1, CSF1, LRP1, ARG1, PTGS1, MSR1, CD163, CSF1R;* and (k) MHC II group: *HLA-DRA, HLA-DRB1, HLA-DMA, HLA-DPA1, HLA-DPB1, HLA-DMB, HLA-DQB1, HLA-DQA1, CIITA.*

In some embodiments, the first RNA expression levels for genes in the first plurality of gene groups further comprise RNA expression levels for at least three genes from each of at least two of the following gene groups: (1) CD4<sup>+</sup> T cells group: *CD4, TRAT1, CD40LG, TRAC, CD28;* (m) CD8<sup>+</sup> T cells group: *PRF1, GZMA, CD8B, KLRK1, CD8A, ZAP70, GZMK, TBX21, GZMB, NKG7, EOMES, CD160, KLRC2, TRAT1;* and (n) Macrophages group: *CMKLR1, IL4I1, OLR1, ADAMDEC1, FPR3, CSF1R, MRC1, SIGLEC1, MS4A7, APOC2, APOE, CD163, SPP1, CCL7, LILRB4, C3AR1, SLAMF8, C1QC, MS4A4A, CLEC10A, C5AR1, RAB7B, CLEC5A, CD14, KMO, VSIG4, ADORA3, IL10, CD4, TREM2, ADAP2, CD68, IFI30, MMP9, PLA2G7, MSR1, C1QA, CYBB, CCR1, CD33.*

In some embodiments, the second RNA expression levels for genes in the second plurality of gene groups comprises RNA expression levels for at least three genes from each of at least two of the following gene groups associated with B cells: (a) Naive B cells group: *CD200, CD27, DPPA4, NAAA, XBP1, MNS1, SIGLEC6, PDE8B, BCL2, IRF4, RHOBTB3, CD1A, ENTPD1,* and *KIF18A;* (b) Centrocyte group: *DHRS9, EGR3, FCER2, DPPA4, ENTPD1, FGD6, DNAJB9, ELL2, ERN1, EIF4E3, AHNAK,* and *FEZ1;* (c) Centroblast group: *KANK2, POU2AF1, PDE8B, SLAMF7, TCL1A, RBM47, MNS1, UEVLD, RASGRF1, NDE1, KIF13A, JUN,* and *NEK2;* (d) Memory B cells group: *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1;* and/or (e) Plasmacyte group: *FKBP11, EGR3, EIF4E3, DPPA4, DNER, ELL2, ELOVL6, FNDC3A, DNAJB9, PRDM1, DLGAP5, FGD6, DHRS9, FNDC3B,* and *ZNF677.*

In some embodiments, determining the first gene group expression scores comprises: determining a respective gene expression score for each of at least two of the three following gene groups, using, for a particular gene group, first RNA expression levels for at least three genes in the particular gene group to determine the gene expression score for the particular group, the three gene groups including: (a) MHC II group: *HLA-DRA, HLA-DRB1, HLA-DMA, HEA-DPA1, HLA-DPB1, HLA-DMB, HLA-DQB1, HLA-DQA1, CIITA;* (b) Effector cells group: *IFNG, GZMA, GZMB, PRF1, GZMK, ZAP70,* GNLY, *FASLG, TBX21, EOMES, CD8A, CD8B;* and (c) Follicular Dendritic Cells (FDC) group: *PDPN, LTBR, FDCSP, CLU, PRNP, C4A, BST1, SERPINE2, C1S, TNFRSF1A.*

In some embodiments, determining the first gene expression signature further comprises determining a respective gene expression score for each of at least two of the following gene groups, using, for a particular gene group, first RNA expression levels for at least three genes in the particular gene group to determine the gene expression score for the particular group, the gene groups including: (d) Treg cells group: *FOXP3, CTLA4, IL10, TNFRSF18, CCR8, IKZF4, IKZF2;* (e) T helper cells (Follicular B Helper T cells) group: *CXCR5, IL6, ICOS, CD40LG, CD84, IL21, BCL6, MAF, SH2D1A, IL4;* (f) Effector cells group: *IFNG, GZMA, GZMB, PRF1, GZMK, ZAP70,* GNLY, *FASLG, TBX21, EOMES, CD8A, CD8B;* (g) Follicular Dendritic Cells (FDC) group: *PDPN, LTBR, FDCSP, CLU, PRNP, C4A, BST1, SERPINE2, C1S, TNFRSF1A;* (h) Lymphatic endothelial cells group: *CCL21, CXCL12, SOX18, PPP1R13B, FLT4, PROX1, PDPN, LYVE1, FOXC2, CXADR, EDNRB, JAM2, JAM3;* (i) Proliferation rate group: *MKI67, ESCO2, CETN3, CDK2, CCND1, CCNE1, AURKA, AURKB, E2F1, MYBL2, BUB1, PLK1, CCNB1, MCM2, MCM6;* (j) M2 group: *IL10, VEGFA, TGFB1, IDO1, PTGES, MRC1, CSF1, LRP1, ARG1, PTGS1, MSR1, CD163, CSF1R;* and (k) MHC II group: *HLA-DRA, HLA-DRB1, HLA-DMA, HLA-DPA1, HLA-DPB1, HLA-DMB, HLA-DQB1, HLA-DQA1, CIITA.*

In some embodiments, determining the first gene expression signature further comprises determining a respective gene expression score for each of at least two of the following gene groups, using, for a particular gene group, first RNA expression levels for at least three genes in the particular gene group to determine the gene expression score for the particular group, the gene groups including: (1) CD4$^+$ T cells group: *CD4, TRAT1, CD40LG, TRAC, CD28;* (m) CD8$^+$ T cells group: *PRF1, GZMA, CD8B, KLRK1, CD8A, ZAP70, GZMK, TBX21, GZMB, NKG7, EOMES, CD160, KLRC2, TRAT1;* and (n) Macrophages group: *CMKLR1, IL4I1,OLR1, ADAMDEC1, FPR3, CSF1R, MRC1, SIGLEC1, MS4A7, APOC2, APOE, CD163, SPP1, CCL7, LILRB4, C3AR1, SLAMF8, C1QC, MS4A4A, CLEC10A, C5AR1, RAB7B, CLEC5A, CD14, KMO, VSIG4, ADORA3, IL10, CD4, TREM2, ADAP2, CD68, IFI30, MMP9, PLA2G7, MSR1, C1QA, CYBB, CCR1, CD33.*

In some embodiments, the first gene group expression scores include a first score for a first gene group in the first plurality of gene groups. In some embodiments, determining the first gene group expression scores comprises determining the first score, using a gene set enrichment analysis (GSEA) technique, from RNA expression levels of at least some genes in the first gene group.

In some embodiments, the first score of the first gene group in the first gene expression signature is determined using a single-sample GSEA (ssGSEA) technique from RNA expression levels for at least some of the genes in one of the following gene groups: (a) MHC II group: *HLA-DRA, HLA-DRB1, HLA-DMA, HLA-DPA1, HLA-DPB1, HLA-DMB, HLA-DQB1, HLA-DQA1, CIITA;* (b) Effector cells group: *IFNG, GZMA, GZMB, PRF1, GZMK, ZAP70,* GNLY, *FASLG, TBX21, EOMES, CD8A, CD8B;* or (c) Follicular Dendritic Cells (FDC) group: *PDPN, LTBR, FDCSP, CLU, PRNP, C4A, BST1, SERPINE2, C1S, TNFRSF1A.*

In some embodiments, determining the second gene expression signature comprises determining a respective gene expression score for each of at least two of the following gene groups associated with B cells including, using, for a particular gene group associated with B cells, second RNA expression levels for at least three genes in the particular gene group associated with B cells to determine the gene expression score for the particular group, the gene groups associated with B cells including (a) Naive B cells group: *CD200, CD27, DPPA4, NAAA, XBP1, MNS1, SIGLEC6, PDE8B, BCL2, IRF4, RHOBTB3, CD1A, ENTPD1,* and *KIF18A;* (b) Centrocyte group: *DHRS9, EGR3, FCER2, DPPA4, ENTPD1, FGD6, DNAJB9, ELL2, ERN1, EIF4E3, AHNAK,* and *FEZ1;* (c) Centroblast group: *KANK2, POU2AF1, PDE8B, SLAMF7, TCL1A, RBM47, MNS1, UEVLD, RASGRF1, NDE1, KIF13A, JUN,* and *NEK2;* (d) Memory B cells group: *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1;* and (e) Plasmacyte group: *FKBP11, EGR3, EIF4E3, DPPA4, DNER, ELL2, ELOVL6, FNDC3A, DNAJB9, PRDM1, DLGAP5, FGD6, DHRS9, FNDC3B,* and *ZNF677.*

In some embodiments, the second plurality of gene groups associated with B cells comprises a first B-cell gene group, and determining the second gene expression scores comprises: determining, using RNA expression levels of at least some genes in the first B-cell gene group and coefficients of a first statistical model associated with the first B-cell gene group, a first score for the first B-cell gene group in the second gene expression signature, wherein, the coefficients of the first statistical model were previously estimated by training the first statistical model to generate, from the RNA expression levels of the at least some genes in the first B-cell gene group, an output indicative of whether the subject is to be associated with the first B-cell gene group.

In some embodiments, determining the first score for the first B-cell gene group comprises: determining an initial score as a dot product between a vector of the coefficients of the first statistical model and a vector of the RNA expression levels of the at least some of the genes in the first B-cell gene group; and determining the score by adjusting the initial score to compensate for batch effects in a process used to obtain the RNA expression levels from the biological sample.

In some embodiments, adjusting the initial score is performed by median scaling.

In some embodiments, the second plurality of gene groups associated with B cells comprises a second B-cell gene group, wherein determining the second gene expression scores comprises: determining, using RNA expression levels of at least some genes in the second B-cell gene group and coefficients of a second statistical model associated with the second B-cell gene group, a second score for the second B-cell gene group in the second gene expression signature, wherein the coefficients of the second statistical model were previously estimated by training the second statistical model to generate, from the RNA expression levels of the at least some genes in the second B-cell gene group, an output indicative of whether the subject is to be associated with the second B-cell gene group.

In some embodiments, the second plurality of gene groups associated with B cells comprises a third B-cell gene group, wherein determining the second gene expression scores comprises: determining, using RNA expression levels of at least some genes in the third B-cell gene group and coefficients of a third statistical model associated with the second B-cell gene group, a third score for the third B-cell gene group in the second gene expression signature, wherein the coefficients of the third statistical model were previously estimated by training the third statistical model to generate, from the RNA expression levels of the at least some genes in the third B-cell gene group, an output indicative of whether the subject is to be associated with the third B-cell gene group.

In some embodiments, the second plurality of gene groups associated with B cells comprises a fourth B-cell gene group, wherein determining the second gene expression scores comprises: determining, using RNA expression levels of at least some genes in the fourth B-cell gene group and coefficients of a fourth statistical model associated with the fourth B-cell gene group, a fourth score for the fourth B-cell gene group in the second gene expression signature, wherein the coefficients of the fourth statistical model were previously estimated by training the fourth statistical model to generate, from the RNA expression levels of the at least some genes in the fourth B-cell gene group, an output indicative of whether the subject is to be associated with the fourth B-cell gene group.

In some embodiments, the second plurality of gene groups associated with B cells comprises a fifth B-cell gene group, wherein determining the second gene expression scores comprises: determining, using RNA expression levels of at least some genes in the fifth B-cell gene group and coefficients of a fifth statistical model associated with the fifth B-cell gene group, a fifth score for the fifth B-cell gene group in the second gene expression signature, wherein the coefficients of the fifth statistical model were previously estimated by training the fifth statistical model to generate, from the RNA expression levels of the at least some genes in the fifth B-cell gene group, an output indicative of whether the subject is to be associated with the fifth B-cell gene group.

In some embodiments, the first B-cell gene group is the Naive B cells group: *CD200, CD27, DPPA4, NAAA, XBP1, MNS1, SIGLEC6, PDE8B, BCL2, IRF4, RHOBTB3, CD1A, ENTPD1,* and *KIF18A.* In some embodiments, the second B-cell gene group is the Centrocyte group: *DHRS9, EGR3, FCER2, DPPA4, ENTPD1, FGD6, DNAJB9, ELL2, ERN1, EIF4E3, AHNAK,* and *FEZ1.* In some embodiments, the third B-cell gene group is the Centroblast group: *KANK2, POU2AF1, PDE8B, SLAMF7, TCL1A, RBM47, MNS1, UEVLD, RASGRF1, NDE1, KIF13A, JUN,* and *NEK2.* In some embodiments, the fourth B-cell gene group is the Memory B cells group: *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1.* In some embodiments, the fifth B-cell gene group is the Plasmacyte group: *FKBP11, EGR3, EIF4E3, DPPA4, DNER, ELL2, ELOVL6, FNDC3A, DNAJB9, PRDM1, DLGAP5, FGD6, DHRS9, FNDC3B,* and *ZNF677.*

In some embodiments, each of the first, second, third, fourth, and fifth B-cell gene groups of the second plurality of gene groups is selected from the B-cell gene groups listed in Table 2.

In some embodiments, each of the first statistical model, second statistical model, third statistical model, fourth statistical model, and fifth statistical model is a logistic regression model with a respective set of coefficients.

In some embodiments, determining the second gene expression scores comprises, for each particular B-cell gene group in the second plurality of gene groups: determining, using RNA expression levels of genes in the particular B-cell gene group and coefficients of a respective statistical model associated with the particular B-cell gene group, a respective score for the respective B-cell gene group in the second gene expression signature.

In some embodiments, the first statistical model comprises a generalized linear model. In some embodiments, the statistical model comprises a generalized linear model. In some embodiments, the generalized linear model comprises a logistic regression model.

In some embodiments, generating the FL TME signature further comprises performing median scaling on the first gene expression signature and the second gene expression signature.

In some embodiments, the second gene expression signature comprises a plurality of BAGS scores for a respective plurality of gene groups. In some embodiments, generating the second gene expression signature comprises determining a first BAGS score for a first of the plurality of gene groups, wherein determining the first BAGS score is performed using RNA gene expression levels of at least some of the genes in the first gene group and coefficients of a BAGS classifier associated with the first group.

In some embodiments, the plurality of FL TME types is associated with a respective plurality of FL TME signature clusters. In some embodiments, identifying, using the FL TME signature and from among a plurality of FL TME types, the FL TME type for the subject comprises: associating the FL TME signature of the subject with a particular one of the plurality of FL TME signature clusters; and, identifying the FL TME type for the subject as the FL TME type corresponding to the particular one of the plurality of FL TME signature clusters to which the FL TME signature of the subject is associated.

In some embodiments, the methods disclosed herein further comprise generating a plurality of FL TME signature clusters, the generating comprising: obtaining multiple sets of RNA expression data obtained by sequencing biological samples from multiple respective subjects, each of the multiple sets of RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells; generating multiple FL TME signatures from the multiple sets of RNA expression data, each of the multiple FL TME signatures comprising first gene group expression scores for respective gene groups in the first plurality of gene groups and second gene group expression scores for respective gene groups in the second plurality of gene groups associated with B cells, the generating comprising, for each particular one of the multiple TME signatures: determining the first gene group expression scores using the first RNA expression levels in the particular set of RNA expression data from which the particular one TME signature is being generated, and determining the second gene group expression scores using the second RNA expression levels in the particular set of RNA expression data form which the particular one TME signature is being generated; and clustering the multiple TME signatures to obtain the plurality of FL TME signature clusters.

In some embodiments, the method as disclosed herein further comprises updating the plurality of FL TME signature clusters using the FL TME signature of the subject. In some embodiments, the FL TME signature of the subject is one of a threshold number FL TME signatures for a threshold number of subjects. In some embodiments, when the threshold number of FL TME signatures is generated the FL TME signature clusters are updated.

In some embodiments, the threshold number of FL TME signatures is at least 50, at least 75, at least 100, at least 200, at least 500, at least 1000, or at least 5000 FL TME signatures.

In some embodiments, the clustering is performed using a clustering algorithm. In some embodiments, the clustering algorithm is a dense clustering algorithm, spectral clustering algorithm, k-means clustering algorithm, hierarchical clustering algorithm, and/or an agglomerative clustering algorithm.

In some embodiments, the method of the present disclosure further comprises determining an FL TME type of a second subject, wherein the FL TME type of the second subject is identified using the updated FL TME signature clusters, wherein the identifying comprises: determining an FL TME signature of the second subject from RNA expression data obtained by sequencing a biological sample obtained from the second subject; associating the FL TME signature of the second subject with a particular one of the plurality of the updated FL TME signature clusters; and identifying the FL TME type for the second subject as the FL TME type corresponding to the particular one of the plurality of updated FL TME signature clusters to which the FL TME signature of the second subject is associated.

In some embodiments, the plurality of a plurality of FL TME types comprises a Normal-like type, a Plasma-cell (PC)-like type, a Light Zone (LZ)-like type, and a Dark Zone (DZ)-like type.

In some embodiments, the FL TME signature further comprises a third gene expression signature, wherein the third gene expression signature comprises one or more PROGENy signatures. In some embodiments, the one or more PROGENy signatures comprise NF-kB and/or PI3K PROGENy signatures.

In some embodiments, the method as disclosed herein further comprises identifying the subject as not having transformed follicular lymphoma (tFL) when the identified FL-TME type for the subject is the Normal-like type.

In some embodiments, the method as disclosed herein further comprises identifying the subject as having a high risk of progression and/or an increased risk of lacking response to R-CHOP when the identified FL-TME type for the subject is the DZ-like type.

In some embodiments, the method as disclosed herein further comprises further comprising: identifying one or more anti-cancer therapies for the subject based upon the identified FL-TME type for the subject; and administering the one or more identified anti-cancer therapies to the subject.

In some embodiments, the one or more anti-cancer therapies comprises rituximab, cyclophosphamide, doxorubicin hydrochloride, vincristine sulfate, and prednisone (R-CHOP) when the subject is identified as having an FL TME type other than DZ-like type.

Aspects of the present disclosure provide a method for treating follicular lymphoma, the method comprising administering one or more therapeutic agents to a subject identified as having a particular FL TME type, wherein the FL TME type of the subject has been identified by method comprising: using at least one computer hardware processor to perform: (a) obtaining RNA expression data for the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells; (b) generating an FL TME signature for the subject using the RNA expression data, the FL TME signature comprising: a first gene expression signature comprising first gene group expression scores for respective gene groups in the first plurality of gene groups, and a second gene expression signature comprising second gene group expression scores for respective gene groups in the second plurality of gene groups associated with B cells, the generating comprising: determining the first gene expression signature by determining the first gene group expression scores using the first RNA expression levels, and determining the second gene expression signature by determining the second gene group expression scores using the second RNA expression levels; and (c) identifying, using the FL TME signature and from among a plurality of FL TME types, an FL TME type for the subject.

In some embodiments, the subject has been identified as having an FL TME type selected from a Normal-like type, a Plasma cell (PC)-like type, a Light Zone (LZ)-like type, and a Dark Zone (DZ)-like type.

In some embodiments, the therapeutic agent comprises R-CHOP when the subject has been identified as having a Normal-like type, a PC-like type, or a Light Zone (LZ)-like type.

In some embodiments, the R-CHOP is administered to the subject on more than one occasion. In some embodiments, the R-CHOP is administered to the subject on between 3 and 6 occasions.

In some embodiments, the therapeutic agent is not R-CHOP when the subject has been identified as having a Dark zone-like type.

BRIEF DESCRIPTION OF DRAWINGS

FIG. 1 is a diagram depicting a flowchart of an illustrative process 100 for determining a follicular lymphoma (FL) tumor microenvironment (TME) type for a subject having, suspected of having, or at risk of having a follicular lymphoma (FL), according to some embodiments of the technology as described herein.

FIG. 2 is a diagram depicting a flowchart of an illustrative process for processing sequencing data to obtain RNA expression data, according to some embodiments of the technology as described herein.

FIG. 3 is a diagram depicting an illustrative technique for determining a first gene expression signature, according to some embodiments of the technology as described herein.

FIG. 4 is a diagram depicting an illustrative technique for determining a second gene expression signature associated with B cells, according to some embodiments of the technology as described herein.

FIG. 5 is a diagram depicting an example of a follicular lymphoma (FL) tumor microenvironment (TME) signature 520, according to some embodiments of the technology as described herein.

FIG. 6 is a diagram depicting an illustrative technique for identifying a follicular lymphoma (FL) tumor microenvironment (TME) type using an FL TME signature, according to some embodiments of the technology as described herein.

FIG. 7 shows representative data indicating cell composition of each FL TME type is consistent with the origin of the identified FL clusters, in accordance with some embodiments of the technology as described herein.

FIG. 8 shows representative data for enrichment of transformed follicular lymphoma (tFL) in DZ-like FL TME type, in accordance with some embodiments of the technology as described herein. Shown top to bottom on the bars are: Plasma cell (PC)-type (also referred to as TH-depleted type), Normal-like (absent from right bar), Light Zone (LZ)-like, and Dark Zone (DZ)-like.

FIG. 9 shows distribution of Stage, Grade, and Progression Risk across FL TME types, in accordance with some embodiments of the technology as described herein. Shown top to bottom on the bars are: PC-type (also referred to as TH-depleted type), Normal-like, LZ-like, and DZ-like.

FIG. 10 shows representative data for survival and progression analysis across different FL TME types. OS=overall survival; FFS= failure free survival, in accordance with some embodiments of the technology as described herein.

FIG. 11 shows FL TME types in normal lymph node (LN), FL, and other B cell lymphoma samples, in accordance with some embodiments of the technology as described herein. Shown top to bottom and left to right: Normal bar comprises Normal-like and PC-like (also referred to as TH-depleted type), Chronic Lymphocytic Leukemia comprises DZ-like, Normal-like, and PC-like, Burkitt Lymphoma comprises DZ-like and PC-like, and FL comprises DZ-like, LZ-like, Normal-like, and PC-like.

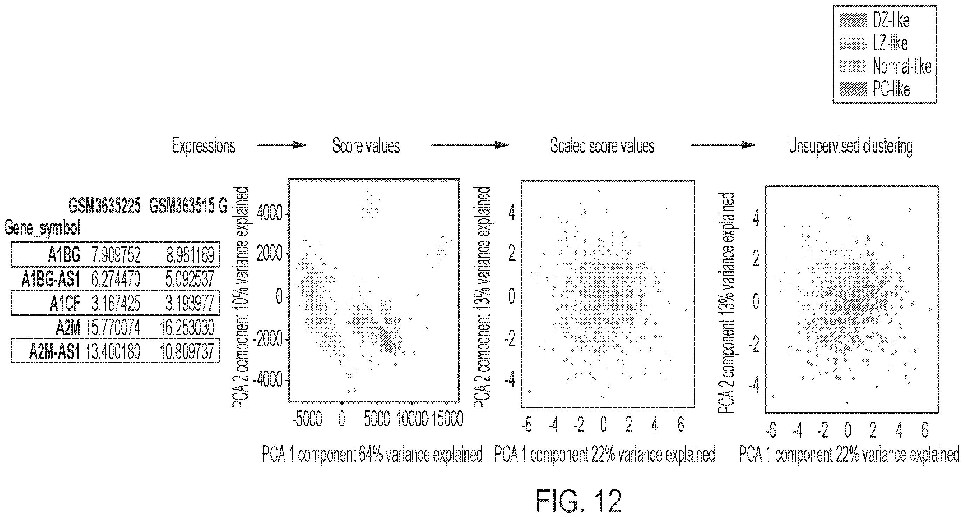

FIG. 12 provides an exemplary illustration to present the process of gene expression data analysis. FIG. 12, left panel, shows a principal component analysis (PCA) projection of gene signature values of all initial cohorts before scaling. Each dot represents a sample, and each different shade represents a dataset. FIG. 12, middle panel, shows a PCA projection after median scaling. FIG. 12, right panel shows a PCA projection with labels obtained by unsupervised clustering. Four distinct FL TME types are shown: DZ-like type, LZ-like type, normal-like type, and PC-like type (also referred to as TH-depleted type).

FIG. 13 provides an exemplary heatmap of FL samples that show the noisy signatures caused by addition of gene groups (e.g., M1 and MHC I gene groups), in accordance with some embodiments of the technology as described herein.

FIG. 14 provides an exemplary heatmap of FL samples that show the correlations between CD4$^+$ T cell group, CD8$^+$ T cell group, and Effector T cells group, in accordance with some embodiments of the technology as described herein.

FIG. 15 shows a heatmap of FL samples classified into four distinct FL TME types based on unsupervised dense clustering of gene expression signatures, in accordance with some embodiments of the technology as described herein. Each column represents one sample. Panel on the top corresponds to the sample annotation: Dataset and FL type. Heatmap at the bottom part represents the signal of each of the used signatures or ratios; "Pathways" module is based on PROGENy signatures.

FIG. 16 depicts an illustrative implementation of a computer system that may be used in connection with some embodiments of the technology described herein.

## DETAILED DESCRIPTION

Aspects of the disclosure relate to methods for characterizing subjects having certain cancers, for example lymphomas. The disclosure is based, in part, on methods for determining the tumor microenvironment (TME) type of a subject's lymphoma (e.g., follicular lymphoma). In some embodiments the methods comprise identifying a subject as having a particular follicular lymphoma (FL) TME type based upon a FL TME signature computed for the subject from their RNA expression data. The FL TME signature may comprise two sub-signatures: a first gene expression signature and a second gene expression signature. The first gene expression signature may include gene group expression scores for gene groups that are associated with lymphatic tissue and/or follicular lymphoma. The second gene expression signature may include gene group expression scores for gene groups that are associated with B cells. The FL TME type identified for the subject may have various prognostic, diagnostic, and/or therapeutic applications. For example, in some embodiments, methods developed by the inventors and described herein are useful for identifying a subject's prognosis, such as a therapeutic response prognosis, based upon the FL TME type identified for the subject.

Follicular lymphoma (FL) is a form of non-Hodgkin lymphoma that arises from B-lymphocytes, and affects the lymph nodes, bone marrow and blood. FL may account for up to 40% of all non-Hodgkin lymphomas, and is typically characterized as an indolent cancer. However, more than 25% (and up to 60%) of FL patients have been observed to undergo transformation from indolent FL to more highly aggressive lymphomas, for example diffuse large B-cell lymphoma. Moreover, a significant percentage of FL patients are resistant to the first line FL chemotherapeutic regimen, R-CHOP (rituximab, cyclophosphamide, doxorubicin hydrochloride (hydroxydaunorubicin), vincristine sulfate (Oncovin), and prednisone).

In the context of FL diagnosis and treatment, clinical prognostic markers, such as the Follicular Lymphoma International Prognostic Index (FLIPI), are considered to be unreliable for individual patient prognosis. Such clinical measures are also of limited value to guide selection of individual therapeutics.

Previously developed molecular biomarker signatures for FL have also suffered from challenges, for example as described by Liu et al. Annals of Lymphoma. 2021 Jun;5:11, the entire contents of which are incorporated by reference herein. Certain previously described molecular biomarkers are highly unpredictable due to factors such as highly variable biology across FL tumors, heterogeneous treatment of subjects used to create the biomarkers, and a failure to adequately identify immune cell subsets that are associated with follicular and intrafollicular areas. Additionally, characterization of the FL tumor microenvironment (TME) has traditionally been based upon immunohistochemistry assays, which typically do not resolve immune cell (e.g., T cell) populations at a resolution that is sufficient to assess tumor microenvironment biology. Accordingly, the inventors have recognized that there is a need to develop methods for molecular characterization of FL types specifically based upon the underlying biology of the lymphatic tumor microenvironment, rather than more broadly defined cancer biomarkers.

Aspects of the disclosure relate to statistical techniques for analyzing expression data (e.g., RNA expression data), which was obtained from a biological sample obtained from a subject that has follicular lymphoma (FL), is suspected of having FL, or is at risk of developing FL, in order to generate a gene expression signature for the subject (termed an "FL TME signature" herein) and use this signature to identify a particular FL type that the subject may have.

The inventors have recognized that a combination of certain gene expression signatures (e.g., a first gene expression signature comprising scores for the gene groups listed in Table 1 and a second gene expression signature comprising scores for gene groups associated with B cells) may be combined to form a FL TME signature that characterizes patients having FL more accurately than previously developed methods. The combination of these two sub-signatures, in turn, may be used to identify the subject as having a particular follicular lymphoma (FL) tumor microenvironment type.

The use of two sub-signatures to generate an FL TME signature represents an improvement over previously described FL molecular biomarkers or tumor microenvironment analyses because the specific groups of genes used to produce the sub-signatures described herein better reflect the molecular tumor microenvironments of FL because these gene groups are associated with 1) lymphatic tissue and/or follicular lymphoma, and 2) a gene expression signature relating to groups of genes that are associated with B cells. These focused combinations of gene groups (e.g., gene groups consisting of only the genes listed in Tables 1 and 2) are unconventional, and differ from previously described molecular signatures, which attempt to incorporate expression data from very large numbers of genes.

Indeed, one important distinguishing characteristic of the FL TME signatures is the smaller number of genes used to determine the FL TME signature as compared to conventional techniques (e.g., the BAGS technique described in Dybkaer et al. J Clin Oncol. 2015 Apr 20; 33(12): 1379-1388, and used for associating B-cell subset phenotypes with DLBCL prognosis, which is incorporated by reference herein in its entirety). Using fewer genes is also an improvement in the efficiency with which such a FL TME signature may be constructed. In addition, fewer computations need to be performed to compute the FL TME signature described herein than would need to be performed to compute signatures for very large numbers of genes, as is the case for BAGS technique.

The FL TME typing methods described herein have several utilities. For example, identifying a subject's FL TME type using methods described herein may allow for the subject to be diagnosed as having (or being at a high risk of developing) an aggressive form of FL at a timepoint that is not possible with previously described FL characterization methods. Since the majority of FL tumors are initially indolent (and are often detected only at an advanced stage), earlier detection of aggressive FL types, enabled by the FL TME signatures described herein, improve the patient diagnostic technology o by enabling earlier chemotherapeutic intervention for patients than currently possible for patients tested for FL using other methods.

Methods described by the disclosure are also useful for determining a therapeutic regimen for a subject having FL. As described herein, the inventors have determined that subjects identified by methods described herein as having Dark Zone (DZ)-like FL have an increased likelihood of responding poorly (or lacking a response) to R-CHOP therapy. Identifying a subject as having "DZ-type" FL using methods described herein, prior to the start of chemotherapy, allows the subject to avoid being prescribed R-CHOP therapy in exchange for a less toxic therapy. Thus, the techniques developed by the inventors and described herein improve patient treatment and associated outcomes by increasing patient comfort, and avoiding toxic side effects of chemotherapy that is not expected to be effective for the subject.

*Follicular Lymphoma*

Aspects of the disclosure relate to methods of determining the follicular lymphoma (FL) TME type of a subject having, suspected of having, or at risk of having FL. A subject may be any mammal, for example a human, non-human primate, rodent (e.g., rat, mouse, guinea pig, etc.), dog, cat, horse etc. In some embodiments, a subject is a human. As used herein, "follicular lymphoma" or "FL" refers to a B cell lymphoma caused by an uncontrolled division of abnormal B lymphocytes in the body of a subject.

A subject having FL may exhibit one or more signs or symptoms of FL, for example night sweats, unexpected loss of weight, fever, asthenia, and adenopathy. In some embodiments, a subject having FL does not exhibit one or more signs or symptoms of FL. In some embodiments, a subject having FL has been diagnosed by a medical professional (e.g., a licensed physician) as having FL based upon one or more assays (e.g., clinical assays, molecular diagnostics, etc.) that indicate that the subject has FL, even in the absence of one or more signs or symptoms.

A subject suspected of having FL typically exhibits one or more signs or symptoms of FL. In some embodiments, a subject suspected of having FL exhibits one or more signs or symptoms of FL but has not been diagnosed by a medical professional (e.g., a licensed physician) and/or has not received a test result (e.g., a clinical assay, molecular diagnostic, etc.) indicating that the subject has FL.

A subject a risk of having FL may or may not exhibit one or more signs or symptoms of FL. In some embodiments, a subject at risk of having FL comprises one or more risk factors that increase the likelihood that the subject will develop FL. Examples of risk factors include the presence of pre-cancerous cells in a clinical sample, having one or more genetic mutations that predispose the subject to developing cancer (e.g., FL), taking one or more medications that increase the likelihood that the subject will develop cancer (e.g., FL), family history of FL, and the like.

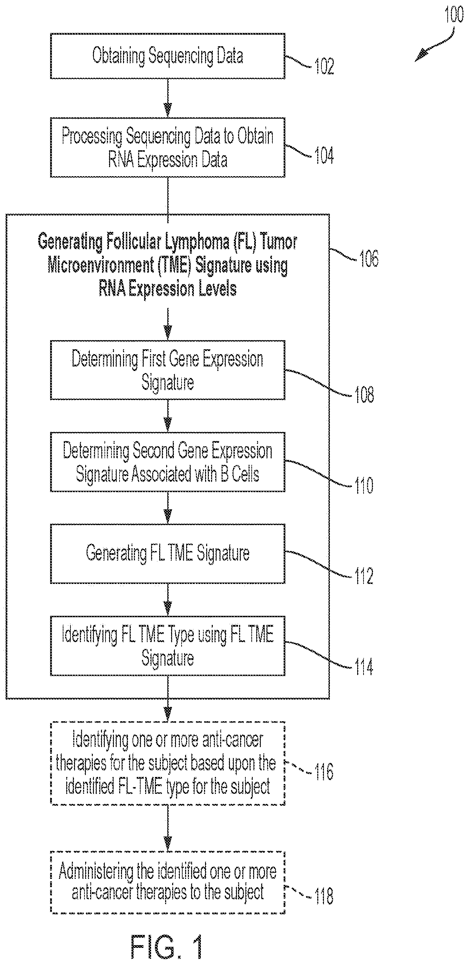

FIG. 1 is a flowchart of an illustrative process 100 for determining an FL TME signature for a subject and using the determined FL TME signature to identify the FL TME type for the subject.

Various acts of process 100 may be implemented using any suitable computing device(s). For example, in some embodiments, one or more acts of the illustrative process 100 may be implemented in a clinical or laboratory setting. For example, one or more acts of the process 100 may be implemented on a computing device that is located within the clinical or laboratory setting. In some embodiments, the computing device may directly obtain RNA expression data from a sequencing platform located within the clinical or laboratory setting. For example, a computing device included in the sequencing platform may directly obtain the RNA expression data from the sequencing platform. In some embodiments, the computing device may indirectly obtain RNA expression data from a sequencing platform that is located within or external to the clinical or laboratory setting. For example, a computing device that is located within the clinical or laboratory setting may obtain expression data via a communication network, such as Internet or any other suitable network, as aspects of the technology described herein are not limited to any particular communication network.

Additionally or alternatively, one or more acts of the illustrative process 100 may be implemented in a setting that is remote from a clinical or laboratory setting. For example, the one or more acts of process 100 may be implemented on a computing device that is located externally from a clinical or laboratory setting. In this case, the computing device may indirectly obtain RNA expression data that is generated using a sequencing platform located within or external to a clinical or laboratory setting. For example, the expression data may be provided to computing device via a communication network, such as Internet or any other suitable network.

It should be appreciated that not all acts of process 100, as illustrated in FIG. 1, may be implemented using one or more computing devices. For example, one or both of the acts 116 and 118, described, herein may be implemented manually. For example, the act 116 of identifying one or more anti-cancer therapies may be implemented manually (e.g., by a clinician), automatically (e.g., by software identifying one or more anti-cancer therapies), or in part manually and in part automatically (e.g., a clinician may select one or more anti-cancer therapies in part using recommendations for one or more cancer therapies generated by the software, for example, using the techniques described herein). As another example, the act 118 of administering one or more anti-cancer therapies may be manually performed (e.g., by a clinician).

Process 100 begins at act 102 where sequencing data for a subject is obtained. In some embodiments, the sequencing data may be obtained by sequencing a biological sample (e.g., lymph node tissue and/or tumor tissue) obtained from the subject using any suitable sequencing technique. The sequencing data may include sequencing data of any suitable type, from any suitable source, and be in any suitable format. Examples of sequencing data, sources of sequencing data, and formats of sequencing data are described herein including in the section called "Obtaining RNA Expression Data".

As one illustrative example, in some embodiments, the sequencing data may comprise bulk sequencing data. The bulk sequencing data may comprise at least 1 million reads, at least 5 million reads, at least 10 million reads, at least 20 million reads, at least 50 million reads, or at least 100 million reads. In some embodiments, the sequencing data comprises bulk RNA sequencing (RNA-seq) data, single cell RNA sequencing (scRNA-seq) data, or next generation sequencing (NGS) data. In some embodiments, the sequencing data comprises microarray data.

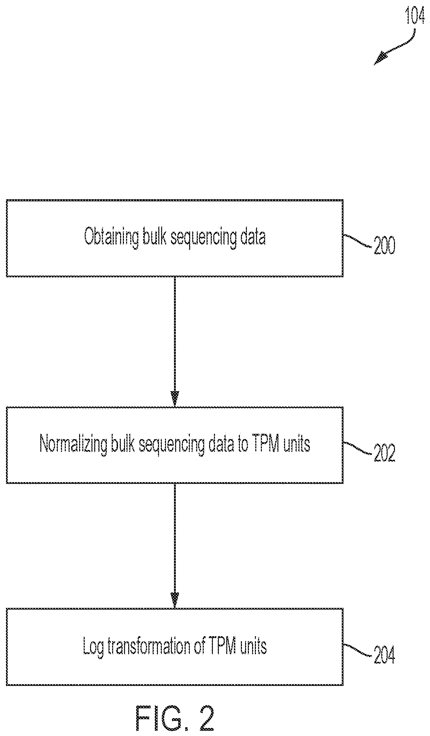

Next, process 100 proceeds to act 104, where the sequencing data obtained at act 102 is processed to obtain RNA expression data. This may be done in any suitable way and may involve normalizing bulk sequencing data to transcripts-per-million (TPM) units (or other units) and/or log transforming the RNA expression levels in TPM units. Converting the data to TPM units and normalization are described herein including with reference to FIG. 2.

Next, process 100 proceeds to act 106, where a follicular lymphoma (FL) tumor microenvironment (TME) signature is generated for the subject using the RNA expression data generated at act 104 (e.g., from bulk-sequencing data, converted to TPM units and subsequently log-normalized, as described herein including with reference to FIG. 2).

As described herein, in some embodiments, an FL TME signature comprises two sub-signatures: a first gene expression signature and a second gene expression signature. The first gene expression signature comprises gene scores for a first set of gene groups (e.g., one or more of the gene groups shown in Table 1). The second gene expression signature comprises gene scores for a second set of gene groups (e.g., one or more gene groups shown in Table 2).

Accordingly, act 106 comprises: act 108 where the first gene expression signature is determined, act 110 where the second gene expression signature is determined, and act 112 where the first and second gene signatures (and, optionally, one or more other signatures such as the ones based on PROGENy and/or ratios of gene group scores) are combined to generate the FL TME signature.

In some embodiments, determining the first gene expression signature comprises determining, for each of multiple gene groups listed in Table 1 (and/or one or more gene groups), a respective gene score. The gene score for a particular gene group may be determined using RNA expression levels for at least some of the genes in the gene group (e.g. the expression levels obtained at act 104). The RNA expression levels may be processed using a gene set enrichment analysis (GSEA) technique to determine the score for the particular gene group.

For example, in some embodiments, determining the first gene expression signature comprises: determining a respective gene expression score for each of at least two of the three following gene groups, using, for a particular gene group, first RNA expression levels for at least three genes in the particular gene group to determine the gene expression score for the particular group, the three gene groups including: (a) MHC II group: HLA-DRA, HLA-DRB1, HLA-DMA, HLA-DPA1, HLA-DPB1, HLA-DMB, HLA-DQB1, HLA-DQA1, CIITA; (b) Effector cells group: IFNG, GZMA, GZMB, PRF1, GZMK, ZAP70, GNLY, FASLG, TBX21, EOMES, CD8A, CD8B; and (c) Follicular Dendritic Cells (FDC) group: PDPN, LTBR, FDCSP, CLU, PRNP, C4A, BST1, SERPINE2, C1S, TNFRSF1A.

As another example, in some embodiments, determining the first gene expression signature further comprises determining a respective gene expression score for each of at least two of the following gene groups, using, for a particular gene group, first RNA expression levels for at least three genes in the particular gene group to determine the gene expression score for the particular group, the gene groups including: (d) Treg cells group: FOXP3, CTLA4, IL10, TNFRSF18, CCR8, IKZF4, IKZF2; (e) T helper cells (Follicular B Helper T cells) group: CXCR5, IL6, ICOS, CD40LG, CD84, IL21, BCL6, MAF, SH2D1A, IL4; (f) Effector cells group: IFNG, GZMA, GZMB, PRF1, GZMK, ZAP70, GNLY, FASLG, TBX21, EOMES, CD8A, CD8B; (g) Follicular Dendritic Cells (FDC) group: PDPN, LTBR, FDCSP, CLU, PRNP, C4A, BST1, SERPINE2, C1S, TNFRSF1A; (h) Lymphatic endothelial cells group: CCL21, CXCL12, SOX18, PPP1R13B, FLT4, PROX1, PDPN, LYVE1, FOXC2, CXADR, EDNRB, JAM2, JAM3; (i) Proliferation rate group: MKI67, ESCO2, CETN3, CDK2, CCND1, CCNE1, AURKA, AURKB, E2F1, MYBL2, BUB1, PLK1, CCNB1, MCM2, MCM6; (j) M2 group: IL10, VEGFA, TGFB1, IDO1, PTGES, MRC1, CSF1, LRP1, ARG1, PTGS1, MSR1, CD163, CSF1R; and (k) MHC II group: HLA-DRA, HLA-DRB1, HLA-DMA, HLA-DPA1, HLA-DPB1, HLA-DMB, HLA-DQB1, HLA-DQA1, CIITA.

As yet another example, in some embodiments, determining the first gene expression signature further comprises determining a respective gene expression score for each of at least two of the following gene groups, using, for a particular gene group, first RNA expression levels for at least three genes in the particular gene group to determine the gene expression score for the particular group, the gene groups including: (1) CD4+ T cells group: CD4, TRAT1, CD40LG, TRAC, CD28; (m) CD8+ T cells group: PRF1, GZMA, CD8B, KLRK1, CD8A, ZAP70, GZMK, TBX21, GZMB, NKG7, EOMES, CD160, KLRC2, TRAT1; and (n) Macrophages group: CMKLR1, IL4I1, OLR1, ADAMDEC1, FPR3, CSF1R, MRC1, SIGLEC1, MS4A7, APOC2, APOE, CD163, SPP1, CCL7, LILRB4, C3AR1, SLAMF8, C1QC, MS4A4A, CLEC10A, C5AR1, RAB7B, CLEC5A, CD14, KMO, VSIG4, ADORA3, IL10, CD4, TREM2, ADAP2, CD68, IFI30, MMP9, PLA2G7, MSR1, C1QA, CYBB, CCR1, CD33.

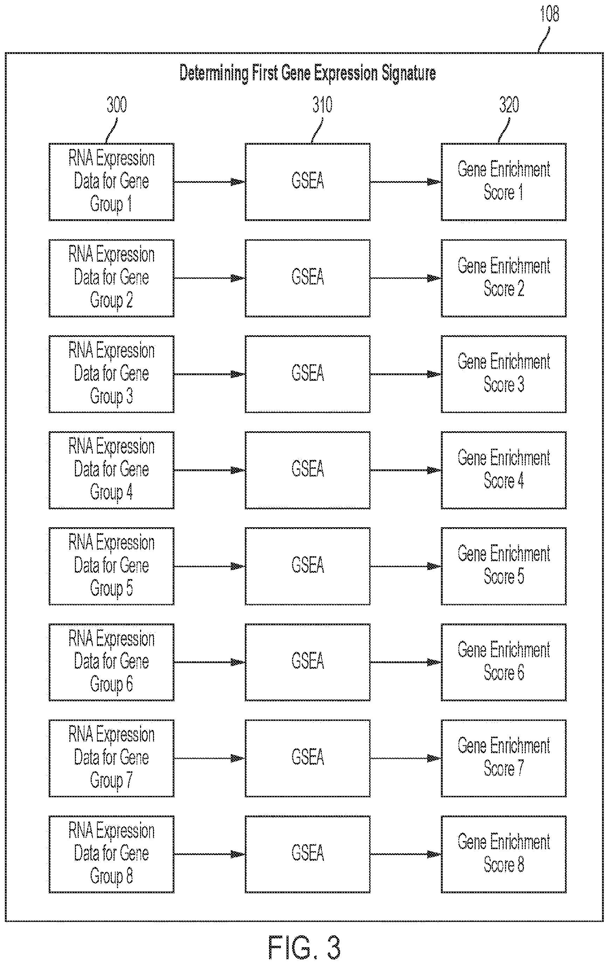

Aspects of determining the first gene expression signature are described herein, including with reference to FIG. 3 and in the Section titled "Gene Expression Signatures".

Turning to the second gene expression signature, in some embodiments, determining the second gene expression signature comprises determining, for each of multiple gene groups listed in Table 2 (and/or one or more gene groups), a respective gene score. The gene score for a particular gene group may be determined using RNA expression levels for at least some of the genes in the gene group (e.g. the expression levels obtained at act 104). The RNA expression levels may be combined with coefficients of a statistical model (e.g., a logistic regression model) trained to distinguish among different B-cell phenotypes (e.g., between a particular B-cell phenotype listed in Table 2 and one or more (or all as a group) other B-cell phenotypes).

In some embodiments, determining the second gene expression signature comprises determining a respective gene expression score for each of at least two of the following gene groups associated with B cells including, using, for a particular gene group associated with B cells, second RNA expression levels for at least three genes in the particular gene group associated with B cells to determine the gene expression score for the particular group, the gene groups associated with B cells including: (a) Naive B cells group: CD200, CD27, DPPA4, NAAA, XBP1, MNS1, SIGLEC6, PDE8B, BCL2, IRF4, RHOBTB3, CD1A, ENTPD1, and KIF18A; (b) Centrocyte group: DHRS9, EGR3, FCER2, DPPA4, ENTPD1, FGD6, DNAJB9, ELL2, ERN1, EIF4E3, AHNAK, and FEZ1; (c) Centroblast group: KANK2, POU2AF1, PDE8B, SLAMF7, TCL1A, RBM47, MNS1, UEVLD, RASGRF1, NDE1, KIF13A, JUN, and NEK2; (d) Memory B cells group: SLC39A8, IL21R, CCR1, TCL1A, BHLHE41, NAAA, ITGAM, EGR3, FCGR2A, RHOBTB3, DPPA4, CD27, RCBTB2, ELOVL6, and ABCB1; and (e) Plasmacyte group: FKBP11, EGR3, EIF4E3, DPPA4, DNER, ELL2, ELOVL6, FNDC3A, DNAJB9, PRDM1, DLGAP5, FGD6, DHRS9, FNDC3B, and ZNF677.

In some embodiments, determining the second gene expression signature comprises determining, using RNA expression levels of at least some genes in the first B-cell gene group and coefficients of a first statistical model associated with the first B-cell gene group, a first score for the first B-cell gene group in the second gene expression signature, wherein the coefficients of the first statistical model were previously estimated by training the first statistical model to generate, from the RNA expression levels of the at least some genes in the first B-cell gene group, an output indicative of whether the subject is to be associated with the first B-cell gene group.

In some embodiments, determining the first score for the first B-cell gene group comprises: determining an initial score as a dot product between a vector of the coefficients of the first statistical model (e.g., a logistic regression model) and a vector of the RNA expression levels of the at least some of the genes in the first B-cell gene group; and determining the score by adjusting the initial score (e.g., using median scaling) to compensate for batch effects in a process used to obtain the RNA expression levels from the biological sample.

In some embodiments, in lieu of determining the second gene expression signature using scores for one or more of the gene groups listed in Table 2, the second gene expression signature may comprise scores for one or more BAGS gene groups, which are defined in Dybkaer et al. J Clin Oncol. 2015 Apr 20; 33(12): 1379-1388, which is incorporated by reference herein in its entirety.

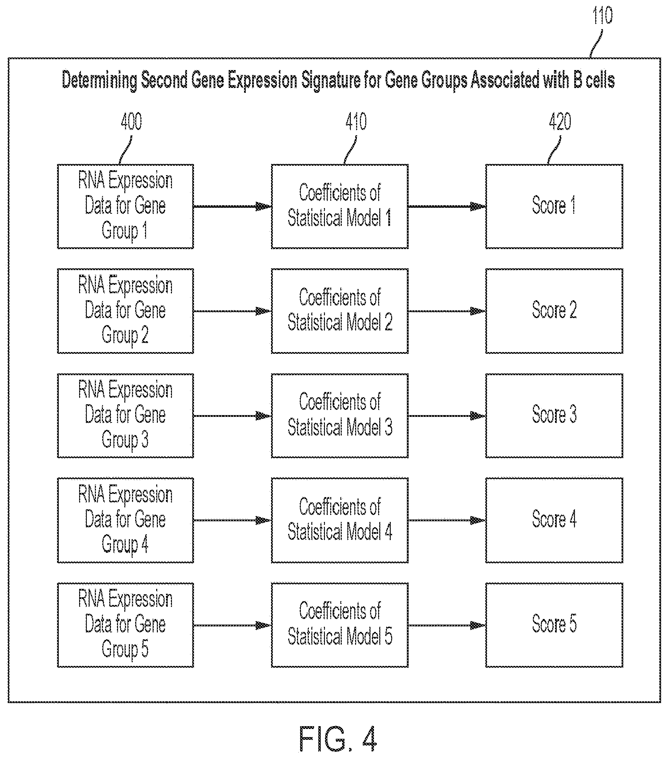

Aspects of determining the second gene expression signature are described herein, including with reference to FIG. 4 and in the Section titled "Gene Expression Signatures".

Acts 108 and 110 may be performed serially or in parallel, as aspects of the technology described herein are not limited in this respect.

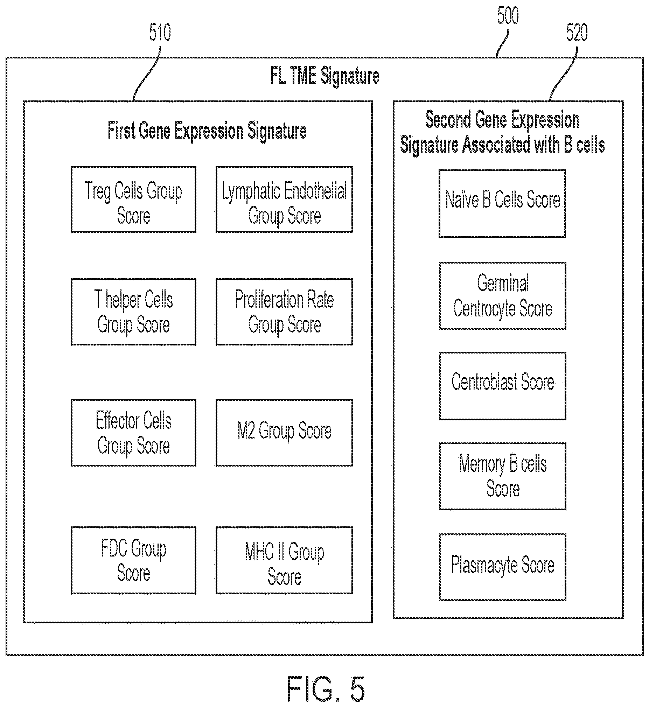

As described above, at act 112, the first and second gene expression signatures (determined during acts 108 and 110, respectively) may be combined to generate the FL TME signature. An example of such an FL TME signature is shown in FIG. 5. In some embodiments, the FL TME signature consists of only the first and second gene expression signatures. In other embodiments, the FL TME signature includes one or more other components in addition to the first and second gene expression signatures. For example, in some embodiments, the FL TME signature includes a third signature comprising one or more PROGENy signatures and/or ratios of gene group scores, as described herein.

Next, process 100 proceeds to act 114, where an FL TME type is identified for the subject using the FL TME signature generated at act 112. This may be done in any suitable way. For example, in some embodiments, the each of the possible FL TME types is associated with a respective cluster of FL TME signatures. In such embodiments, an FL TME type for the subject may be identified by associating the FL TME signature of the subject with a particular one of the plurality of FL TME signature clusters; and identifying the FL TME type for the subject as the FL TME type corresponding to the particular one of the plurality of FL TME signature clusters to which the FL TME signature of the subject is associated. Examples of FL TME types are described herein. Aspects of identifying an FL TME type for a subject are described herein including in the section below titled "Identifying FL TME Type".

In some embodiments, process 100 completes after act 114 completes. In some such embodiments the determined FL TME signature and/or identified FL TME Type may be stored for subsequent use, provided to one or more recipients (e.g., a clinician, a researcher, etc.), and/or used to update the FL TME signature clusters (as described hereinbelow).

However, in some embodiments, one or more other acts are performed after act 114. For example, in the illustrated embodiment, one or more anti-cancer therapies may be identified for the subject based on the FL TME type determined for the subject. For example, in some embodiments, the one or more anti-cancer therapies identified at act 116 comprise: rituximab, cyclophosphamide, doxorubicin hydrochloride, vincristine sulfate, and prednisone (R-CHOP) when the subject is identified (at act 114) as having an FL TME type other than DZ-like type. In some embodiments, at act 116, the subject may be determined as having a high risk of progression and/or an increased risk of lacking response to R-CHOP when the identified FL-TME type for the subject is the DZ-like type.

At act 118, one or more of the identified anti-cancer therapies may be administered in a therapeutically effective manner to the subject.

*Biological Samples*

Aspects of the disclosure relate to methods for determining a FL TME type of a subject by obtaining sequencing data from a biological sample that has been obtained from the subject.

The biological sample may be from any source in the subject's body including, but not limited to, any fluid such as blood (*e.g.,* whole blood, blood serum, or blood plasma), lymph nodes, and tonsils.

The biological sample may be any type of sample including, for example, a sample of a bodily fluid, one or more cells, one or more pieces of tissue(s) or organ(s). In some embodiments, the biological sample comprises lymph node tissue of the subject. In some embodiments, the biological sample comprises tumor cells of the subject, for example follicular lymphoma cells of the subject.

In some embodiments, a lymph node tissue sample may be obtained from a subject using a needle to draw fluid (*e.g.,* aspirate) from the lymph node or biopsy a lymph node.

A sample of lymph node or blood, in some embodiments, refers to a sample comprising cells, e.g., cells from a blood sample or lymph node sample. In some embodiments, the sample comprises non-cancerous cells. In some embodiments, the sample comprises pre-cancerous cells. In some embodiments, the sample comprises cancerous cells. In some embodiments, the sample comprises blood cells. In some embodiments, the sample comprises lymph node cells. In some embodiments, the sample comprises lymph node cells and blood cells. Examples of cancerous blood cells include, but are not limited to, cancerous FL cells.

A sample of blood may be a sample of whole blood or a sample of fractionated blood. In some embodiments, the sample of blood comprises whole blood. In some embodiments, the sample of blood comprises fractionated blood. In some embodiments, the sample of blood comprises buffy coat. In some embodiments, the sample of blood comprises serum. In some embodiments, the sample of blood comprises plasma. In some embodiments, the sample of blood comprises a blood clot.

In some embodiments, a sample of blood is collected to obtain the cell-free nucleic acid (e.g., cell-free DNA) in the blood.

In some embodiments, the sample may be from a cancerous tissue or an organ or a tissue or organ suspected of having one or more cancerous cells. In some embodiments, the sample may be from a healthy (*e.g.,* non-cancerous) tissue or organ. In some embodiments, the sample from a healthy (*e.g.,* non-cancerous) tissue or organ may be from a subject who is at risk or suspected of having the risk of developing cancer. In some embodiments, the sample from a healthy (*e.g.,* non-cancerous) tissue or organ may be from tissues surrounding one or more cancerous cells. In some embodiments, a sample from a subject (*e.g.,* a biopsy from a subject) may include both healthy and cancerous cells and/or tissue. In certain embodiments, one sample will be taken from a subject for analysis. In some embodiments, more than one (*e.g.,* 2, 3, 4, 5, 6, 7, 8, 9, 10, 11, 12, 13, 14, 15, 16, 17, 18, 19, 20, or more) samples may be taken from a subject for analysis. In some embodiments, one sample from a subject will be analyzed. In certain embodiments, more than one (*e.g.,* 2, 3, 4, 5, 6, 7, 8, 9, 10, 11, 12, 13, 14, 15, 16, 17, 18, 19, 20, or more) samples may be analyzed. If more than one sample from a subject is analyzed, the samples may be procured at the same time (*e.g.,* more than one sample may be taken in the same procedure), or the samples may be taken at different times (*e.g.,* during a different procedure including a procedure 1, 2, 3, 4, 5, 6, 7, 8, 9, 10 days; 1, 2, 3, 4, 5, 6, 7, 8, 9, 10 weeks; 1, 2, 3, 4, 5, 6, 7, 8, 9, 10 months, 1, 2, 3, 4, 5, 6, 7, 8, 9, 10 years, or 1, 2, 3, 4, 5, 6, 7, 8, 9, 10 decades after a first procedure). A second or subsequent sample may be taken or obtained from the same region (*e.g.,* from the same tumor or area of tissue) or a different region (including, *e.g.,* a different tumor). A second or subsequent sample may be taken or obtained from the subject after one or more treatments, and may be taken from the same region or a different region. A second or subsequent sample may be taken or obtained from the subject when the first sample from the subject was taken. For example, two separate samples can be taken during the same procurement. These two separate samples can be pooled or compared for the analysis as disclosed herein. As a non-limiting example, the second or subsequent sample may be useful in determining whether the cancer in each sample has different characteristics (*e.g.,* in the case of samples taken from two physically separate tumors in a patient) or whether the cancer has responded to one or more treatments (*e.g.,* in the case of two or more samples from the same tumor prior to and subsequent to a treatment).

Any of the biological samples described herein may be obtained from the subject using any known technique. See, for example, the following publications on collecting, processing, and storing biological samples, each of which is incorporated by reference herein in its entirety: Biospecimens and biorepositories: from afterthought to science by Vaught et al. (Cancer Epidemiol Biomarkers Prev. 2012 Feb;21(2):253-5), and Biological sample collection, processing, storage and information management by Vaught and Henderson (IARC Sci Publ. 2011;(163):23-42).

In some embodiments, the biological sample may be obtained from a surgical procedure (*e.g.,* laparoscopic surgery, microscopically controlled surgery, or endoscopy), bone marrow biopsy, punch biopsy, endoscopic biopsy, or needle biopsy (e.g., a fine-needle aspiration, core needle biopsy, vacuum-assisted biopsy, or image-guided biopsy). In some embodiments, each of the at least one biological sample is a bodily fluid sample such as whole blood sample, a cell sample, or a tissue biopsy.

Any of the biological samples from a subject described herein may be stored using any method that preserves stability of the biological sample. In some embodiments, preserving the stability of the biological sample means inhibiting components (e.g., DNA, RNA, protein, or tissue structure or morphology) of the biological sample from degrading until they are measured so that when measured, the measurements represent the state of the sample at the time of obtaining it from the subject. In some embodiments, a biological sample is stored in a composition that is able to penetrate the same and protect components (e.g., DNA, RNA, protein, or tissue structure or morphology) of the biological sample from degrading. As used herein, degradation is the transformation of a component from one form to another form such that the first form is no longer detected at the same level as before degradation.

In some embodiments, the biological sample is stored using cryopreservation. Non-limiting examples of cryopreservation include, but are not limited to, step-down freezing, blast freezing, direct plunge freezing, snap freezing, slow freezing using a programmable freezer, and vitrification. In some embodiments, the biological sample is stored using lyophilisation. In some embodiments, a biological sample is placed into a container that already contains a preservant (e.g., RNALater to preserve RNA) and then frozen (e.g., by snap-freezing), after the collection of the biological sample from the subject. In some embodiments, such storage in frozen state is done immediately after collection of the biological sample. In some embodiments, a biological sample may be kept at either room temperature or 4°C for some time (e.g., up to an hour, up to 8 h, or up to 1 day, or a few days) in a preservant or in a buffer without a preservant, before being frozen.

Non-limiting examples of preservants include formalin solutions, formaldehyde solutions, RNALater or other equivalent solutions, TriZol or other equivalent solutions, DNA/RNA Shield or equivalent solutions, EDTA (e.g., Buffer AE (10 mM Tris·Cl; 0.5 mM EDTA, pH 9.0)) and other coagulants, and Acids Citrate Dextrose (e.g., for blood specimens).

In some embodiments, special containers may be used for collecting and/or storing a biological sample. For example, a vacutainer may be used to store blood. In some embodiments, a vacutainer may comprise a preservant (e.g., a coagulant, or an anticoagulant). In some embodiments, a container in which a biological sample is preserved may be contained in a secondary container, for the purpose of better preservation, or for the purpose of avoid contamination.

Any of the biological samples from a subject described herein may be stored under any condition that preserves stability of the biological sample. In some embodiments, the biological sample is stored at a temperature that preserves stability of the biological sample. In some embodiments, the sample is stored at room temperature (e.g., 25 °C). In some embodiments, the sample is stored under refrigeration *(e.g.,* 4 °C). In some embodiments, the sample is stored under freezing conditions (e.g., -20 °C). In some embodiments, the sample is stored under ultralow temperature conditions (e.g., -50 °C to -800 °C). In some embodiments, the sample is stored under liquid nitrogen (e.g., -1700 °C). In some embodiments, a biological sample is stored at -60 °C to -8-°C (e.g., -70°C) for up to 5 years (e.g., up to 1 month, up to 2 months, up to 3 months, up to 4 months, up to 5 months, up to 6 months, up to 7 months, up to 8 months, up to 9 months, up to 10 months, up to 11 months, up to 1 year, up to 2 years, up to 3 years, up to 4 years, or up to 5 years). In some embodiments, a biological sample is stored as described by any of the methods described herein for up to 20 years (e.g., up to 5 years, up to 10 years, up to 15 years, or up to 20 years).

*Obtaining RNA Expression Data*

Aspects of the disclosure relate to methods of determining a FL TME type of a subject using RNA expression data obtained from a biological sample obtained from the subject.

The RNA expression data used in methods described herein typically is derived from sequencing data obtained from the biological sample. After the sequencing data is obtained, it is processed in order to obtain the RNA expression data. RNA expression data may be acquired using any method known in the art including, but not limited to: whole transcriptome sequencing, total RNA sequencing, mRNA sequencing, targeted RNA sequencing, RNA exome capture sequencing, next generation sequencing, and/or deep RNA sequencing. In some embodiments, RNA expression data may be obtained using a microarray assay.

In some embodiments, the sequencing data is processed to produce RNA expression data. In some embodiments, sequencing data is processed by one or more bioinformatics methods or software tools, for example RNA sequence quantification tools (e.g., Kallisto) and genome annotation tools (e.g., Gencode v23), in order to produce the RNA expression data. The Kallisto software is described in Nicolas L Bray, Harold Pimentel, Páll Melsted and Lior Pachter, Near-optimal probabilistic RNA-seq quantification, Nature Biotechnology 34, 525-527 (2016), doi:10.1038/nbt.3519, which is incorporated by reference in its entirety herein.

In some embodiments, microarray expression data is processed using a bioinformatics R package, such as "affy" or "limma", in order to produce expression data. The "affy" software is described in Bioinformatics. 2004 Feb 12;20(3):307-15. doi: 10.1093/bioinformatics/btg405. "affy--analysis of Affymetrix GeneChip data at the probe level" by Laurent Gautier 1, Leslie Cope, Benjamin M Bolstad, Rafael A Irizarry PMID: 14960456 DOI: 10.1093/bioinformatics/btg405, which is incorporated by reference herein in its entirety. The "limma" software is described in Ritchie ME, Phipson B, Wu D, Hu Y, Law CW, Shi W, Smyth GK "limma powers differential expression analyses for RNA-sequencing and microarray studies." Nucleic Acids Res. 2015 Apr 20;43(7):e47. 20. https://doi.org/10.1093/nar/gkv007 PMID: 25605792, PMCID: PMC4402510, which is incorporated by reference herein its entirety.

In some embodiments, sequencing data and/or expression data comprises more than 5 kilobases (kb). In some embodiments, the size of the obtained RNA data is at least 10 kb. In some embodiments, the size of the obtained RNA sequencing data is at least 100 kb. In some embodiments, the size of the obtained RNA sequencing data is at least 500 kb. In some embodiments, the size of the obtained RNA sequencing data is at least 1 megabase (Mb). In some embodiments, the size of the obtained RNA sequencing data is at least 10 Mb. In some embodiments, the size of the obtained RNA sequencing data is at least 100 Mb. In some embodiments, the size of the obtained RNA sequencing data is at least 500 Mb. In some embodiments, the size of the obtained RNA sequencing data is at least 1 gigabase (Gb). In some embodiments, the size of the obtained RNA sequencing data is at least 10 Gb. In some embodiments, the size of the obtained RNA sequencing data is at least 100 Gb. In some embodiments, the size of the obtained RNA sequencing data is at least 500 Gb.

In some embodiments, the expression data is acquired through bulk RNA sequencing. Bulk RNA sequencing may include obtaining expression levels for each gene across RNA extracted from a large population of input cells (e.g., a mixture of different cell types.) In some embodiments, the expression data is acquired through single cell sequencing (e.g., scRNA-seq). Single cell sequencing may include sequencing individual cells.

In some embodiments, bulk sequencing data comprises at least 1 million reads, at least 5 million reads, at least 10 million reads, at least 20 million reads, at least 50 million reads, or at least 100 million reads. In some embodiments, bulk sequencing data comprises between 1 million reads and 5 million reads, 3 million reads and 10 million reads, 5 million reads and 20 million reads, 10 million reads and 50 million reads, 30 million reads and 100 million reads, or 1 million reads and 100 million reads (or any number of reads including, and between).

In some embodiments, the expression data comprises next-generation sequencing (NGS) data. In some embodiments, the expression data comprises microarray data.

Expression data (e.g., indicating expression levels) for a plurality of genes may be used for any of the methods or compositions described herein. The number of genes which may be examined may be up to and inclusive of all the genes of the subject. In some embodiments, expression levels may be determined for all of the genes of a subject. As a non-limiting example, four or more, five or more, six or more, seven or more, eight or more, nine or more, ten or more, eleven or more, twelve or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more, 35 or more, 40 or more, 50 or more, 60 or more, 70 or more, 80 or more, 90 or more, 100 or more, 125 or more, 150 or more, 175 or more, 200 or more, 225 or more, 250 or more, 275 or more, or 300 or more genes may be used for any evaluation described herein. As another set of non-limiting examples, the expression data may include, for each gene group listed in Tables 1 and 2, expression data for at least 5, at least 10, at least 15, at least 20, at least 25, at least 35, at least 50, at least 75, at least 100 genes selected from each gene group.

In some embodiments, RNA expression data is obtained by accessing the RNA expression data from at least one computer storage medium on which the RNA expression data is stored. Additionally or alternatively, in some embodiments, RNA expression data may be received from one or more sources via a communication network of any suitable type. For example, in some embodiment, the RNA expression data may be received from a server (e.g., a SFTP server, or Illumina BaseSpace).

The RNA expression data obtained may be in any suitable format, as aspects of the technology described herein are not limited in this respect. For example, in some embodiments, the RNA expression data may be obtained in a text-based file (e.g., in a FASTQ, FASTA, BAM, or SAM format). In some embodiments, a file in which sequencing data is stored may contains quality scores of the sequencing data. In some embodiments, a file in which sequencing data is stored may contain sequence identifier information.

Expression data, in some embodiments, includes gene expression levels. Gene expression levels may be detected by detecting a product of gene expression such as mRNA and/or protein. In some embodiments, gene expression levels are determined by detecting a level of a mRNA in a sample. As used herein, the terms "determining" or "detecting" may include assessing the presence, absence, quantity and/or amount (which can be an effective amount) of a substance within a sample, including the derivation of qualitative or quantitative concentration levels of such substances, or otherwise evaluating the values and/or categorization of such substances in a sample from a subject.

FIG. 2 shows an exemplary process 104 for processing sequencing data to obtain RNA expression data from sequencing data. Process 104 may be performed by any suitable computing device or devices, as aspects of the technology described herein are not limited in this respect. For example, process 104 may be performed by a computing device part of a sequencing platform. In other embodiments, process 104 may be performed by one or more computing devices external to the sequencing platform.

Process 104 begins at act 200, where bulk sequencing data is obtained from a biological sample obtained from a subject. The bulk sequencing data is obtained by any suitable method, for example, using any of the methods described herein including in the Section titled "Biological Samples".

In some embodiments, the bulk sequencing data obtained at act 104 comprises RNA-seq data. In some embodiments, the biological sample comprises blood or tissue. In some embodiments, the biological sample comprises one or more tumor cells, for example, one or more FL tumor cells.

Next, process 104 proceeds to act 202 where the sequencing data obtained at act 200 is normalized to transcripts per kilobase million (TPM) units. The normalization may be performed using any suitable software and in any suitable way. For example, in some embodiments, TPM normalization may be performed according to the techniques described in Wagner et al. (Theory Biosci. (2012) 131:281-285), which is incorporated by reference herein in its entirety. In some embodiments, the TPM normalization may be performed using a software package, such as, for example, the gcrma package. Aspects of the gcrma package are described in Wu J, Gentry RIwcfJMJ (2021). "gcrma: Background Adjustment Using Sequence Information. R package version 2.66.0.", which is incorporated by reference in its entirety herein. In some embodiments, RNA expression level in TPM units for a particular gene may be calculated according to the following formula:

$$A \cdot \frac{1}{\sum(A)} \cdot 10^6$$

$$A = \frac{total\ reads\ mapped\ to\ gene \cdot 10^3}{gene\ length\ in\ bp}$$

Where

Next, process 104 proceeds to act 204, where the RNA expression levels in TPM units (as determined at act 202) may be log transformed. Although, in some embodiments, the log transformation is optional and may be omitted, in some embodiments, the log transformation is an important transformation to employ for calculating gene scores for gene groups associated with B cells (e.g., the gene scores that constitute the second sub-signature of a subject's FL TME signature) as it reduces the range of variability of the RNA expression levels thereby improving the resulting FL TME signature by making it more informative and effective at identifying the FL TME type for the subject.

Process 104 is illustrative and there are variations. For example, in some embodiments, one or both of acts 202 and 204 may be omitted. Thus, in some embodiments, the RNA expression levels may not be normalized to transcripts per million units and may, instead, be converted to another type of unit (e.g., reads per kilobase million (RPKM) or fragments per kilobase million (FPKM) or any other suitable unit). Additionally or alternatively, in some embodiments, the log transformation may be omitted. Instead, no transformation may be applied in some embodiments, or one or more other transformations may be applied in lieu of the log transformation.

Expression data obtained by process 104 can include the sequence data generated by a sequencing protocol (e.g., the series of nucleotides in a nucleic acid molecule identified by next-generation sequencing, sanger sequencing, etc.) as well as information contained therein (e.g., information indicative of source, tissue type, etc.) which may also be considered information that can be inferred or determined from the sequence data. In some embodiments, expression data obtained by process 104 can include information included in a FASTA file, a description and/or quality scores included in a FASTQ file, an aligned position included in a BAM file, and/or any other suitable information obtained from any suitable file.

*Gene Expression Signatures*

Aspects of the disclosure relate to processing of expression data to determine one or more gene expression signatures. In some embodiments, expression data (e.g., RNA expression data) is processed using a computing device to determine the one or more gene expression signatures. In some embodiments, the computing device may be operated by a user such as a doctor, clinician, researcher, patient, or other individual. For example, the user may provide the expression data as input to the computing device (e.g., by uploading a file), and/or may provide user input specifying processing or other methods to be performed using the expression data.

In some embodiments, expression data may be processed by one or more software programs running on computing device.

The disclosure is based, in part, on the recognition that a combination of certain gene expression signatures (e.g., a first gene expression signature comprising the gene groups listed in Table 1 and a second gene expression signature associated with B cells) may be combined to produce a FL TME signature that characterizes patients having FL more accurately than previously developed methods.

In some embodiments, methods described herein comprise an act of determining a first gene expression signature comprising first gene group expression scores for respective gene groups in a first plurality of gene groups. This first gene expression signature may be a sub-signature of a subject's overall FL TME signature (see e.g., FIG. 5). In some embodiments, the first gene group expression signature comprises first gene group expression scores having a gene group score for at least one (e.g., 1, 2, 3, 4, 5, 6, 7, or 8) of the gene groups listed in Table 1.

The number of genes in a gene group used to determine a gene group expression score may vary. In some embodiments, all RNA expression levels for all genes in a particular gene group may be used to determine a gene group score for the particular gene group. In other embodiments, RNA expression data for fewer than all genes may be used (e.g., RNA expression levels for at least two genes, at least three genes, at least five genes, between 2 and 10 genes, between 5 and 15 genes, or any other suitable range within these ranges).

In some embodiments, the first gene group expression signature comprises a score for the Treg cells gene group. In some embodiments, this score may be calculated using RNA expression levels of at least two genes (e.g., at least two genes, at least three genes, at least four genes, at least five genes, at least six genes, or at least seven genes) in the Treg cells gene group, which is defined by its constituent genes: *FOXP3, CTLA4, IL10, TNFRSF18, CCR8, IKZF4,* and *IKZF2.*

In some embodiments, a first gene group expression signature comprises a score for the T helper cells gene group. In some embodiments, this score may be calculated using RNA expression levels of at least two genes (e.g., at least two genes, at least three genes, at least four genes, at least five genes, at least six genes, at least seven genes, at least eight genes, at least nine genes, at least ten genes, or more than ten genes) in the T helper cells (Follicular B Helper T cells) gene group, which is defined by its constituent gene: *CXCR5, IL6, ICOS, CD40LG, CD84, IL21, BCL6, MAF, SH2D1A,* and *IL4.*

In some embodiments, a first gene group expression signature comprises a score for the MHC II group. In some embodiments, this score may be calculated using RNA expression levels of at least two genes (e.g., at least two genes, at least three genes, at least four genes, at least five genes, at least six genes, at least seven genes, at least eight genes, or at least nine genes) in the MHC II group, which is defined by its constituent genes: *HLA-DRA, HLA-DRB1, HLA-DMA, HLA-DPA1, HLA-DPB1, HLA-DMB, HLA-DQB1, HLA-DQA1,* and *CIITA.*

In some embodiments, a first gene group expression signature comprises a score for the Effector cells group. In some embodiments, this score may be calculated using RNA expression levels of at least two genes (e.g., at least two genes, at least three genes, at least four genes, at least five genes, at least six genes, at least seven genes, at least eight genes, at least nine genes, at least ten genes, or more than ten genes) in the Effector cells group, which is defined by its constituent genes: *IFNG, GZMA, GZMB, PRF1, GZMK, ZAP70,* GNLY, *FASLG, TBX21, EOMES, CD8A,* and *CD8B.*

In some embodiments, a first gene group expression signature comprises a score for the Follicular Dendritic Cells group. In some embodiments, this score may be calculated using RNA expression levels of at least two genes (e.g., at least two genes, at least three genes, at least four genes, at least five genes, at least six genes, at least seven genes, at least eight genes, at least nine genes, or at least ten genes) in the Follicular Dendritic Cells (FDC) group, which is defined by its constituent genes: *PDPN, LTBR, FDCSP, CLU, PRNP, C4A, BST1, SERPINE2, C1S,* and *TNFRSF1A.*

In some embodiments, a first gene group expression signature comprises a score for the Lymphatic endothelial cells group. In some embodiments, this score may be calculated using RNA expression levels of at least two genes (e.g., at least two genes, at least three genes, at least four genes, at least five genes, at least six genes, at least seven genes, at least eight genes, at least nine genes, at least ten genes, or more than ten genes) in the Lymphatic endothelial cells group, which is defined by its constituent genes: *CCE21, CXCE12, SOX18, PPP1R13B, FLT4, PROX1, PDPN, LYVE1, FOXC2, CXADR, EDNRB, JAM2,* and *JAM3.*

In some embodiments, a first gene group expression signature comprises a score for the Proliferation rate group. In some embodiments, this score may be calculated using RNA expression levels of at least two genes (e.g., at least two genes, at least three genes, at least four genes, at least five genes, at least six genes, at least seven genes, at least eight genes, at least nine genes, at least ten genes, or more than ten genes) in the Proliferation rate group, which is defined by its constituent genes: *MKI67, ESCO2, CETN3, CDK2, CCND1, CCNE1, AURKA, AURKB, E2F1, MYBL2, BUB1, PLK1, CCNB1, MCM2,* and *MCM6.*

In some embodiments, a first gene group expression signature comprises a score for the M2 group. In some embodiments, this score may be calculated using RNA expression levels of at least two genes (e.g., at least two genes, at least three genes, at least four genes, at least five genes, at least six genes, at least seven genes, at least eight genes, at least nine genes, at least ten genes, or more than ten genes) in the M2 group, which is defined by its constituent genes: *IL10, VEGFA, TGFB1, IDO1, PTGES, MRC1, CSF1, LRP1, ARG1, PTGS1, MSR1, CD163,* and *CSF1R.*

In some embodiments, determining a first gene expression signature comprises determining a respective gene expression score for each of at least two of the following gene groups, using, for a particular gene group, first RNA expression levels for at least three genes in the particular gene group to determine the gene expression score for the particular group, the gene groups including: MHC II group: *HLA-DRA, HLA-DRB1, HLA-DMA, HLA-DPA1, HLA-DPB1, HLA-DMB, HLA-DQB1, HLA-DQA1, CIITA*; Effector cells group: *IFNG, GZMA, GZMB, PRF1, GZMK, ZAP70,* GNLY, *FASLG, TBX21, EOMES, CD8A, CD8B;* and Follicular Dendritic Cells (FDC) group: *PDPN, LTBR, FDCSP, CLU, PRNP, C4A, BST1, SERPINE2, C1S,* and *TNFRSF1A.*

In some embodiments, determining a first gene expression signature comprises determining a respective gene expression score for each of at least two of the following gene groups, using, for a particular gene group, first RNA expression levels for at least three genes in the particular gene group to determine the gene expression score for the particular group, the gene groups including: Treg cells group: *FOXP3, CTLA4, IL10, TNFRSF18, CCR8, IKZF4, IKZF2*; T helper cells (Follicular B Helper T cells) group: *CXCR5, IL6, ICOS, CD40LG, CD84, IL21, BCL6, MAF, SH2D1A, IL4;* Effector cells group: *IFNG, GZMA, GZMB, PRF1, GZMK, ZAP70,* GNLY, *FASLG, TBX21, EOMES, CD8A, CD8B;* Follicular Dendritic Cells (FDC) group: *PDPN, LTBR, FDCSP, CLU, PRNP, C4A, BST1, SERPINE2, C1S, TNFRSF1A*; Lymphatic endothelial cells group: *CCL21, CXCL12, SOX18, PPP1R13B, FLT4, PROX1, PDPN, LYVE1, FOXC2, CXADR, EDNRB, JAM2, JAM3*; Proliferation rate group: *MKI67, ESCO2, CETN3, CDK2, CCND1, CCNE1, AURKA, AURKB, E2F1, MYBL2, BUB1, PLK1, CCNB1, MCM2, MCM6;* M2 group: *IL10, VEGFA, TGFB1, IDO1, PTGES, MRC1, CSF1, LRP1, ARG1, PTGS1, MSR1, CD163, CSF1R;* and MHC II group: *HLA-DRA, HLA-DRB1, HLA-DMA, HLA-DPA1, HLA-DPB1, HLA-DMB, HLA-DQB1, HLA-DQA1, CIITA.*

In some embodiments, determining a first gene expression signature comprises determining a respective gene group score for each of the following gene groups: Treg cells group: *FOXP3, CTLA4, IL10, TNFRSF18, CCR8, IKZF4, IKZF2;* T helper cells (Follicular B Helper T cells) group: *CXCR5, IL6, ICOS, CD40LG, CD84, IL21, BCL6, MAF, SH2D1A, IL4*; Effector cells group: *IFNG, GZMA, GZMB, PRF1, GZMK, ZAP70,* GNLY, *FASLG, TBX21, EOMES, CD8A, CD8B*; Follicular Dendritic Cells (FDC) group: *PDPN, LTBR, FDCSP, CLU, PRNP, C4A, BST1, SERPINE2, C1S, TNFRSF1A*; Lymphatic endothelial cells group: *CCL21, CXCL12, SOX18, PPP1R13B, FLT4, PROX1, PDPN, LYVE1, FOXC2, CXADR, EDNRB, JAM2, JAM3*; Proliferation rate group: *MKI67, ESCO2, CETN3, CDK2, CCND1, CCNE1, AURKA, AURKB, E2F1, MYBL2, BUB1, PLK1, CCNB1, MCM2, MCM6;* M2 group: *IL10, VEGFA, TGFB1, IDO1, PTGES, MRC1, CSF1, LRP1, ARG1, PTGS1, MSR1, CD163, CSF1R;* and MHC II group: *HLA-DRA, HLA-DRB1, HLA-DMA, HLA-DPA1, HLA-DPB1, HLA-DMB, HLA-DQB1, HLA-DQA1, CIITA.* Each gene group score may be determined using RNA expression levels for one or more (e.g., at least three, at least four, at least five, at least six, etc., all) genes in the gene group.

In some embodiments, determining a first gene expression signature further comprises determining a respective gene group score for each of the following gene groups: CD4+ T cells group: *CD4, TRAT1, CD40LG, TRAC, CD28;* CD8+ T cells group: *PRF1, GZMA, CD8B, KLRK1, CD8A, ZAP70, GZMK, TBX21, GZMB, NKG7, EOMES, CD160, KLRC2, TRAT1;* and Macrophages group: *CMKLR1, IL4I1,OLR1, ADAMDEC1, FPR3, CSF1R, MRC1, SIGLEC1, MS4A7, APOC2, APOE, CD163, SPP1, CCL7, LILRB4, C3AR1, SLAMF8, C1QC, MS4A4A, CLEC10A, C5AR1, RAB7B, CLEC5A, CD14, KMO, VSIG4, ADORA3, IL10, CD4, TREM2, ADAP2, CD68, IFI30, MMP9, PLA2G7, MSR1, C1QA, CYBB, CCR1, CD33.* Each gene group score may be determined using RNA expression levels for one or more (e.g., at least three, at least four, at least five, at least six, etc., all) genes in the gene group.

A list of gene groups is provided in Table 1 below:

Table 1: List of Gene Groups, the left column providing the name of the Gene Group and the right column providing examples of genes in the Gene Group.

| Gene Group Name | Constituent Genes |
|---|---|
| Treg cells | *FOXP3, CTLA4, IL10, TNFRSF18, CCR8, IKZF4, IKZF2* |

(continued)

| Gene Group Name | Constituent Genes |
|---|---|
| T helper cells (Follicular B Helper T cells) | *CXCR5, IL6, ICOS, CD40LG, CD84, IL21, BCL6, MAF, SH2D1A, IL4* |
| MHC II | *HLA-DRA, HLA-DRB1, HLA-DMA, HLA-DPA1, HLA-DPB1, HLA-DMB, HLA-DQB1, HLA-DQA1, CIITA* |
| Effector cells | *IFNG, GZMA, GZMB, PRF1, GZMK, ZAP70, GNLY, FASLG, TBX21, EOMES, CD8A, CD8B* |
| Follicular Dendritic Cells (FDC) | *PDPN, LTBR, FDCSP, CLU, PRNP, C4A, BST1, SERPINE2, C1S, TNFRSF1A* |
| M2 | *IL10, VEGFA, TGFB1, IDO1, PTGES, MRC1, CSF1, LRP1, ARG1, PTGS1, MSR1, CD163, CSF1R* |
| Lymphatic endothelial cells | *CCL21, CXCL12, SOX18, PPP1R13B, FLT4, PROX1, PDPN, LYVE1, FOXC2, CXADR, EDNRB, JAM2, JAM3* |
| Proliferation rate | *MKI67, ESCO2, CETN3, CDK2, CCND1, CCNE1, AURKA, AURKB, E2F1, MYBL2, BUB1, PLK1, CCNB1, MCM2, MCM6* |

As described above, aspects of the disclosure relate to determining an FL TME signature for a subject. That signature may include two sub-signatures: a first gene expression signature (e.g., generated using RNA expression data for gene groups listed in Table 1) and a second gene expression signature (e.g., generated using RNA expression data for gene groups listed in Table 2). Aspects of determining of these sub-signatures is described next with reference to FIGs. 3 and 4.

In some embodiments, the first gene expression signature may be determined by using a gene set enrichment analysis (GSEA) technique to determine a gene enrichment score for one or more (e.g., one, two, three, four, five, six, seven, or all eight) gene groups listed in Table 1.

In some embodiments, the first gene expression signature includes a first score for a first gene group in the first plurality of gene groups, and determining the first score, using a gene set enrichment analysis (GSEA) technique, from RNA expression levels of at least some genes in the first gene group. In some embodiments, using a GSEA technique comprises using single-sample GSEA. Aspects of single sample GSEA (ssGSEA) are described in Barbie et al. Nature. 2009 Nov 5; 462(7269): 108-112, the entire contents of which are incorporated by reference herein. In some embodiments, ssGSEA is performed according to the following formula:

$$ssGSEA\ score\ =\ \frac{\sum\limits_{i}^{N} r_i^{1.25}}{\sum\limits_{i}^{N} r_i^{0.25}}\ -\ \frac{(M - N + 1)}{2}$$

where $r_i$ represents the rank of the ith gene in expression matrix, where N represents the number of genes in the gene set (e.g., the number of genes in the first gene group when ssGSEA is being used to determine a score for the first gene group using expression levels of the genes in the first gene group), and where M represents total number of genes in expression matrix. Additional, suitable techniques of performing GSEA are known in the art and are contemplated for use in the methods described herein without limitation.

FIG. 3 depicts an illustrative process 108 for determining a first gene expression signature, according to some embodiments of the technology as described herein. As shown in FIG. 3, the first gene expression signature comprises multiple gene group scores 320 determined for respective multiple gene groups. Each gene group score, for a particular gene group, is computed by performing GSEA 310 (e.g., using ssGSEA) on RNA expression data for one or more (e.g., at least two, at least three, at least four, at least five, at least six, etc., all) genes in the particular gene group.

For example, as shown in FIG. 3, a gene group score (labelled "Gene Enrichment Score 1") for gene group 1 (e.g., the Treg cells group) is computed from RNA expression data for one or more genes in gene group 1. As another example, a gene group score (labelled "Gene Enrichment Score 2") for gene group 2 (e.g., the T helper cells group) is computed from RNA expression data for one or more genes in gene group 2. As another example, a gene group score (labelled "Gene Enrichment Score 3") for gene group 3 (e.g., the MHC II group) is computed from RNA expression data for one or more genes in gene group 3. As another example, a gene group score (labelled "Gene Enrichment Score 4") for gene group 4 (e.g., the Effector cells group) is computed from RNA expression data for one or more genes in gene group 4. As another example, a gene group score (labelled "Gene Enrichment Score 5") for gene group 5 (e.g., the Follicular Dendritic Cells group) is computed from RNA expression data for one or more genes in gene group 5. As another example, a gene group score (labelled "Gene Enrichment Score 6") for gene group 6 (e.g., the M2 group) is computed from RNA expression data for one or more genes in gene group 6. As another example, a gene group score (labelled "Gene Enrichment Score 7") for gene group 7 (e.g., the Lymphatic endothelial cells group) is computed from RNA expression data for one or more genes in gene group 7. As another example, a gene group score (labelled "Gene Enrichment Score 8") for gene group 8 (e.g., the Proliferation group) is computed from RNA expression data for one or more genes in gene group 8.

Although the example of FIG. 3 shows that the first gene expression signature includes eight gene group scores for a respective set of eight gene groups, it should be appreciated that in other embodiments, the first gene expression signature may include scores for any suitable number of groups (e.g., not just 8), as aspects of the technology described herein are not limited in this respect. For example, the first gene expression signature may include scores for only a subset of the gene groups listed in Table 1 above. As another example, the first gene expression signature may include one or more scores for one or more gene groups other than those gene groups listed in Table 1 (either in addition to the score(s) for the groups in Table 1 or instead of one or more of the scores for the groups in Table 1).

In some embodiments, RNA expression levels for a particular gene group may be embodied in at least one data structure having fields storing the expression levels. The data structure or data structures may be provided as input to software comprising code that implements a GSEA technique (e.g., the ssGSEA technique) and processes the expression levels in the at least one data structure to compute a score for the particular gene group.

As described above, in addition to the first gene expression signature, an FL TME signature for a subject may include a second gene expression signature (e.g., generated using RNA expression data for gene groups listed in Table 2).

In some embodiments, the second gene expression signature may comprise a plurality of gene group scores for a respective plurality of gene groups. In some embodiments, the gene groups of the second plurality of gene groups are associated with B cells. A gene group associated with B cells refers to a gene group (and genes in that group) that are known or predicted to be expressed by cell types that interact with B cells and/or are known or predicted to be expressed by B cells. Non-limiting examples of gene groups associated with B cells, and their constituent genes, are listed in Table 2. Accordingly, the plurality of gene group scores may be determined for each of one or more of the gene groups listed in Table 2. Additionally or alternatively to the gene groups named in Table 2, the plurality of gene groups (for which respective gene group scores are determined) may include one or more other gene groups associated with B-cells, which are not listed in Table 2. In some embodiments, a gene group score for a gene group associated with B cells may be determined by using RNA expression data for at least one (e.g., one, two, three, four, etc., all) gene in the gene group and coefficients of a statistical model (e.g., a generalized linear model, such as, for example, a logistic regression model) trained to predict whether a biological sample has a particular B-cell phenotype.

The number of genes in a gene group used to determine a gene group expression score may vary. In some embodiments, all RNA expression levels for all genes in a particular gene group may be used to determine a gene group score for the particular gene group. In other embodiments, RNA expression data for fewer than all genes may be used (e.g., RNA expression levels for at least two genes, at least three genes, at least five genes, between 2 and 10 genes, between 5 and 15 genes, or any other suitable range within these ranges).

In some embodiments, determining a second gene expression signature comprises determining a respective gene expression score for each of at least two of the following gene groups associated with B cells including, using, for a particular gene group associated with B cells, second RNA expression levels for at least three genes in the particular gene group associated with B cells to determine the gene expression score for the particular group, the gene groups associated with B cells including: Naive B cells: *CD200, CD27, DPPA4, NAAA, XBP1, MNS1, SIGLEC6, PDE8B, BCL2, IRF4, RHOBTB3, CD1A, ENTPD1,* and *KIF18A;* Centrocyte: *DHRS9, EGR3, FCER2, DPPA4, ENTPD1, FGD6, DNAJB9, ELL2, ERN1, EIF4E3, AHNAK,* and *FEZ1;* Centroblast: *KANK2, POU2AF1, PDE8B, SLAMF7, TCL1A, RBM47, MNS1, UEVLD, RASGRF1, NDE1, KIF13A, JUN,* and *NEK2;* Memory B cells: *SLC39A8, IL21R, CCR1, TCL1A, BHEHE41, NAAA, ITGAM, EGR3, FCGR2A, RHOBTB3, DPPA4, CD27, RCBTB2, ELOVL6,* and *ABCB1;* and Plasmacyte: *FKBP11, EGR3, EIF4E3, DPPA4, DNER, ELL2, ELOVL6, FNDC3A, DNAJB9, PRDM1, DLGAP5, FGD6, DHRS9, FNDC3B,* and *ZNF677.*

In some embodiments, determining a second gene expression signature comprises determining a respective gene expression score for each gene in each of the following gene groups associated with B cells including, using, for a particular gene group associated with B cells, second RNA expression levels for each gene in the particular gene group associated with B cells to determine the gene expression score for the particular group, the gene groups associated with B cells including: Naive B cells: *CD200, CD27, DPPA4, NAAA, XBP1, MNS1, SIGLEC6, PDE8B, BCL2, IRF4,*

*RHOBTB3, CD1A, ENTPD1,* and *KIF18A;* Centrocyte: *DHRS9, EGR3, FCER2, DPPA4, ENTPD1, FGD6, DNAJB9, ELL2, ERN1, EIF4E3, AHNAK,* and *FEZ1;* Centroblast: *KANK2, POU2AF1, PDE8B, SLAMF7, TCL1A, RBM47, MNS1, UEVLD, RASGRF1, NDE1, KIF13A, JUN,* and *NEK2;* Memory B cells: *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1;* and Plasmacyte: *FKBP11, EGR3, EIF4E3, DPPA4, DNER, ELL2, ELOVL6, FNDC3A, DNAJB9, PRDM1, DLGAP5, FGD6, DHRS9, FNDC3B,* and *ZNF677.*

In some embodiments, a second gene expression signature is produced using a technique other than GSEA or ssGSEA. In some embodiments, a second gene expression signature is determined using a B cell associated gene signature (BAGS) classification system. BAGS classification is known, and described for example in Dybkær K et al., Diffuse large B-cell lymphoma classification system that associates normal B-cell subset phenotypes with prognosis. J Clin Oncol. 2015;33(12): 1379-1388, which is incorporated by reference herein in its entirety. In some embodiments, a second gene expression signature comprises a plurality of BAGS scores for a respective plurality of gene groups, wherein generating the second gene expression signature comprises determining a first BAGS score for a first of the plurality of gene groups, wherein determining the first BAGS score is performed using RNA gene expression levels of at least some of the genes in the first gene group and coefficients of a BAGS classifier associated with the first group. In some embodiments, determining the first BAGS score comprises: determining an initial BAGS score as a dot product between a vector of the coefficients of the first BAGS classifier and a vector of the RNA expression levels of the at least some of the genes in the first gene group; and determining the BAGS score by adjusting the initial BAGS score to compensate for batch effects in a process used to obtain the RNA expression levels from the biological sample.

Aspects of how a gene group score, part of a second gene expression signature, is determined are described next with reference to FIG. 4.

FIG. 4 depicts an illustrative technique process 108 for determining a second gene expression signature, according to some embodiments of the technology as described herein.

As shown in FIG. 4, the second gene expression signature comprises multiple gene group scores 420 determined for respective multiple gene groups. A gene group score, for a particular gene group, is computed by using: (1) coefficients 410 of a statistical model associated with the particular gene group; and (2) RNA expression data for one or more (e.g., at least two, at least three, at least four, at least five, at least six, etc., all) genes in the particular gene group.

For example, as shown in FIG. 4, a gene group score (labelled "Score 1") for gene group 1 (e.g., the Naive B cells group) is computed using RNA expression data for one or more genes in gene group 1 and coefficients of a statistical model (e.g., a linear regression model) associated with this gene group. As another example, a gene group score (labelled "Score 2") for gene group 2 (e.g., the Centrocyte group) is computed using RNA expression data for one or more genes in gene group 2 and coefficients of a statistical model (e.g., a linear regression model) associated with this gene group. As another example, a gene group score (labelled "Score 3") for gene group 3 (e.g., the Centroblast group) is computed using RNA expression data for one or more genes in gene group 3 and coefficients of a statistical model (e.g., a linear regression model) associated with this gene group. As another example, a gene group score (labelled "Score 4") for gene group 4 (e.g., the Memory B cells group) is computed using RNA expression data for one or more genes in gene group 4 and coefficients of a statistical model (e.g., a linear regression model) associated with this gene group. As another example, a gene group score (labelled "Score 5") for gene group 5 (e.g., the Plasmacyte (Plasma) group) is computed using RNA expression data for one or more genes in gene group 5 and coefficients of a statistical model (e.g., a linear regression model) associated with this gene group.

In some embodiments, determining a gene group score for a particular gene group associated with B cells (e.g., for any one group listed in Table 2) from: (1) RNA expression levels for at least some of the genes in the particular gene group and (2) coefficients of a statistical model associated with the particular gene group, involves: (a) determining an initial score as a dot product between a vector of the coefficients of the statistical model and a vector of the RNA expression levels of the at least some of the genes in the particular gene group; and (b) determining the gene group score by adjusting the initial score to compensate for batch effects in a process used to obtain the RNA expression levels from the biological sample.

In some embodiments, adjusting the initial score may be performed by using median scaling with respect to a dataset of scores derived from a batch of biological samples that were sequenced using the same process that was used to sequence the subject's (the subject for whom the FL TME signature is being calculated and in particular for whom the second sub-signature is being calculated from RNA data for genes in the gene groups associated with B cells) biological sample. In some embodiments, median scaling involves estimating median and MAD (median absolute deviation) for each signature within such a dataset, and applying the formula $x_i$-median(x)/MAD(x). Other scaling techniques may be used to compensate for batch effects in addition to or instead of median scaling, as aspects of the technology described herein are not limited in this respect.

In some embodiments, RNA expression levels for a particular gene group may be embodied in at least one data structure having fields storing the expression levels. The data structure or data structures may be provided as input to software comprising code that is configured to access coefficients of a statistical model (e.g., a logistic regression model) associated with the particular gene group, determine a dot product between the gene expression levels and the coefficients, and perform suitable scaling (e.g., median scaling) to produce a score for the particular gene group.

Although the example of FIG. 4 shows that the second gene expression signature includes five gene group scores for a respective set of five gene groups, it should be appreciated that in other embodiments, the second gene expression signature may include scores for any suitable number of groups (e.g., not just 5), as aspects of the technology described herein are not limited in this respect. For example, the second gene expression signature may include scores for only a subset of the gene groups listed in Table 2. As another example, the second gene expression signature may include one or more scores for one or more gene groups other than those gene groups listed in Table 2 (either in addition to the score(s) for the groups in Table 2 or instead of one or more of the scores for the groups in Table 2).

A list of B-cell associated gene groups is provided in Table 2 below:

Table 2: List of B-cell associated Gene Groups, the left column providing the name of the Gene Group and the right column providing examples of genes in the Gene Group.

| Gene Group Name | Constituent Genes |
|---|---|
| Naive B cells | *CD200, CD27, DPPA4, NAAA, XBP1, MNS1, SIGLEC6, PDE8B, BCL2, IRF4, RHOBTB3, CD1A, ENTPD1,* and *KIF18A* |
| Centrocyte | *DHRS9, EGR3, FCER2, DPPA4, ENTPD1, FGD6, DNAJB9, ELL2, ERN1, EIF4E3, AHNAK,* and *FEZ1* |
| Centroblast | *KANK2, POU2AF1, PDE8B, SLAMF7, TCL1A, RBM47, MNS1, UEVLD, RASGRF1, NDE1, KIF13A, JUN,* and *NEK2* |
| Memory B cells | *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1* |
| Plasmacyte (Plasma) | *FKBP11, EGR3, EIF4E3, DPPA4, DNER, ELL2, ELOVL6, FNDC3A, DNAJB9, PRDM1, DLGAP5, FGD6, DHRS9, FNDC3B,* and *ZNF677* |

In some embodiments, a FL TME signature comprises one or more additional gene expression signatures (e.g., in addition to the first gene expression signature and second gene expression signature described above). In some embodiments, an FL TME signature may comprise at least two (e.g., at least two, at least three, at least four, at least five, at least six, at least seven, at least eight, at least nine, at least ten, or more than ten) PROGENy signatures. In some embodiments, the PROGENy signatures comprise an NF-kB score and/or a Phosphoinositide 3-kinase (PI3K) score [*e.g.,* as described by doi.org/10.1038/s41467-017-02391-6, the entire contents of which are incorporated by reference herein].

In some embodiments, a CD4$^+$ group to CD8$^+$ group gene expression signature ratio may be used in calculating a FL TME signature. In some embodiments, a gene expression signature is obtained by using RNA expression levels for at least three genes in the each of the CD4$^+$ group to CD8$^+$ group to determine the gene expression signature for each group. The ratio of the two gene expression signatures is then calculated. The use of gene expression signature (GES) ratios, such as ratios of gene group expression signatures, may improve conventional GESs-based approaches in the determination of follicular lymphoma types.

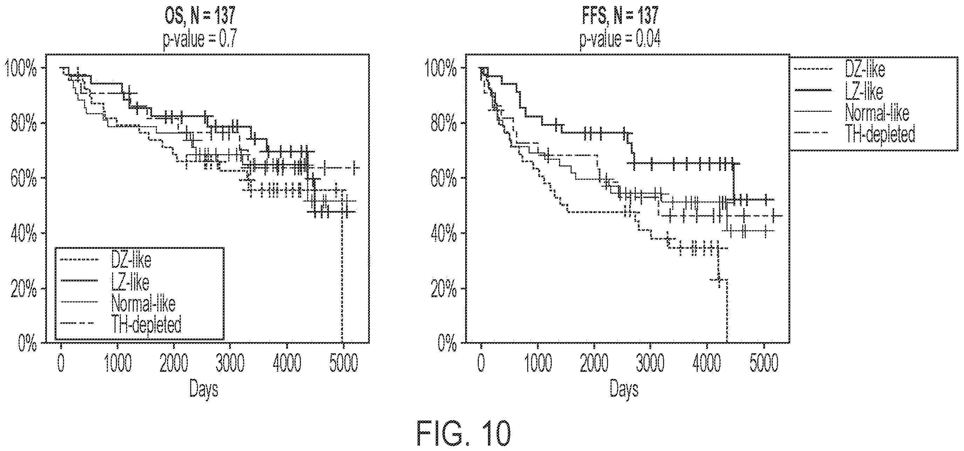

In some embodiments, the CD4$^+$ group to CD8$^+$ T-cell group expression score ratio may be used as gene signatures that are separate from other gene signatures when clustering. For example, the CD4$^+$ group to CD8$^+$ group T-cell signal ratio can be a standalone gene signature for determining the FL TME. As shown in FIG. 10, the CD4$^+$ group and CD8$^+$ group gene signatures are highly correlated to the Effector cell gene signatures. Accordingly, the use of the CD4$^+$ group and CD8$^+$ group gene signatures and/or their ratios are optional when clustering. In some embodiments, the CD4$^+$ group to CD8$^+$ group signature ratio may be included in the group of other gene signatures when clustering. The calculation of the CD4$^+$ group to CD8$^+$ group signature ratio is known by a skilled person in the art. For example, the respective gene group expression scores of the CD4$^+$ group and the CD8$^+$ group are first determined. The value of the gene group expression score of CD4$^+$ group is then divided by the value of the gene group expression score of CD8$^+$ group to obtain the CD4$^+$ to CD8$^+$ T-cell signal ratio. In some embodiments, the CD4$^+$ T-cell and the CD8$^+$ T-cell signatures can be used as standalone signatures (e.g., no ratios are calculated).

Some aspects of determining gene group scores for gene groups are also described in U.S. Patent Publication No. 2020-0273543, entitled "SYSTEMS AND METHODS FOR GENERATING, VISUALIZING AND CLASSIFYING MOLECULAR FUNCTIONAL PROFILES", the entire contents of which are incorporated by reference herein.

*Generating FL TME Signature and Identifying TME Type*

FIG. 5 shows an illustrative FL TME signature 500. The FL TME signature comprises a first expression signature 510 and a second gene expression signature associated with B cells 520. As shown, the first expression signature 510 comprises eight gene group scores for the following gene groups: Treg cells group, T helper cells group, Effector Cells group, FDC group, Lymphatic endothelial group, Proliferation rate group, M2 group, and the MHC II group. Also, as shown, the second expression signature 520 comprises five gene group scores for the following gene groups associated with B cells: Naive B cells group, Centrocyte group, Centroblast group, Memory B cells group, and the Plasmacyte group.

As can be appreciated, the example FL TME signature 500 comprises thirteen scores including a score for each of the gene groups in Table 1 and a score for each of the gene groups in Table 2. However, it should be appreciated, that an FL TME signature may include fewer scores than the number of scores shown in FIG. 5 (e.g., by omitting scores for one or more of the gene groups listed in Table 1 and/or Table 2) or more scores than the number of scores shown in FIG. 5 (e.g., by including scores for one or more other gene groups in addition to or instead of the gene groups listed in Table 1 and/or Table 2, such as, for example, scores associated with the CD4+ T cells group, the CD8+ T cells group and/or the macrophages group, described herein).

In some embodiments, an FL TME signature may be embodied in at least one data structure comprising fields storing the gene group scores part of the FL TME signature.

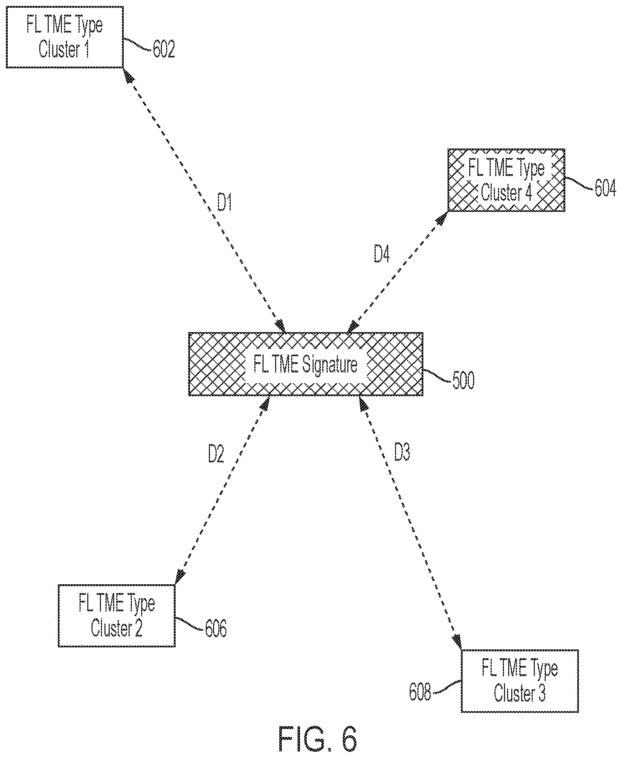

FIG. 6 is a diagram illustrating how an FL TME type may be identified for a subject by using the FL TME signature determined for the subject using the techniques described herein.

As described herein, in some embodiments, one of a plurality of different FL TME types may be identified for the subject using the FL TME signature determined for the subject using the techniques described herein. In some embodiments, the TME types comprise normal-like type, PC-like (or T Helper (TH)-depleted) type, light Zone (LZ)-like type, and dark Zone (DZ)-like type, as described herein and further below. In some embodiments, each of the plurality of FL TME types is associated with a respective FL TME signature cluster in a plurality of FL TME signature clusters. The FL TME type for a subject may be determined by: (1) associating the FL TME signature of the subject with a particular one of the plurality of FL TME signature clusters; and (2) identifying the FL TME type for the subject as the FL TME type corresponding to the particular one of the plurality of FL TME signature clusters to which the FL TME signature of the subject is associated.

For example, as shown in FIG. 6, a subject's FL TME signature 500 may be associated with one of four TME clusters: 602, 604, 606, and 608. Each of the clusters 602, 604, 608, and 610 may be associated with respect FL TME type. In this example, the FL TME signature 500 is compared to each cluster (e.g., using a distance-based comparison or any other suitable metric) and, based on the result of the comparison, the FL TME signature 500 is associated with the closest FL signature cluster (when a distance-based comparison is performed, or the "closest" in the sense of whatever metric or measure of distance is used). In this example, FL TME signature 500 is associated with FL TME Type Cluster 4 604 (as shown by the consistent shading) because the measure of distance D4 between the FL TME signature 500 and (e.g., a centroid or other point representative of) cluster 604 is smaller than the measures of the distance D1, D2, and D3 between the FL TME signature 500 and (e.g., a centroid or other point(s) representative of) clusters 602, 606, and 608, respectively.

In some embodiments, a subject's FL TME signature may be associated with one of four FL TME signature clusters by using a machine learning technique (e.g., such as k-nearest neighbors (KNN) or any other suitable classifier) to assign the FL TME signature to one of the four FL TME signature clusters. The machine learning technique may be trained to assign FL TME signatures on the metacohorts represented by the signatures in the clusters.

In some embodiments, the FL TME signature clusters may be generated by: (1) obtaining FL TME signatures (using the techniques described herein) for a plurality of subjects; and (2) clustering the FL TME signatures so obtained into the plurality of clusters. Any suitable clustering technique may be used for this purpose including, but not limited to, a dense clustering algorithm, spectral clustering algorithm, k-means clustering algorithm, hierarchical clustering algorithm, and/or an agglomerative clustering algorithm.

Accordingly, in some embodiments, generating the FL TME signature clusters involves: (A) obtaining multiple sets of RNA expression data obtained by sequencing biological samples from multiple respective subjects, each of the multiple sets of RNA expression data indicating first RNA expression levels for genes in a first plurality of gene groups (e.g., one or more of the gene groups in Table 1) and second RNA expression levels for genes in a second plurality of gene groups different from the first plurality of gene groups (e.g., one or more of the gene groups in Table 2), wherein genes in the second plurality of gene groups are associated with B cells; (B) generating multiple FL TME signatures from the multiple sets of RNA expression data, each of the multiple FL TME signatures comprising first gene group expression scores for respective gene groups in the first plurality of gene groups and second gene group expression scores for respective gene groups in the second plurality of gene groups associated with B cells, the generating comprising, for each particular one of the multiple TME signatures: (i) determining the first gene group expression scores using the first RNA expression levels in the particular set of RNA expression data from which the particular one TME signature is being generated, and (ii) determining the second gene group expression scores using the second RNA expression levels in the particular set of RNA expression data form which the particular one TME signature is being generated; and (C) clustering the multiple TME signatures to obtain the plurality of FL TME signature clusters.

The resulting FL TME signature clusters may each contain any suitable number of FL TME signatures (e.g., at least 10, at least 100, at least 500, at least 500, at least 1000, at least 5000, between 100 and 10,000, between 500 and 20,000, or any other suitable range within these ranges), as aspects of the technology described herein are not limited in this respect.

The number of FL TME signature clusters in this example is four. And although, in some embodiments, it may be possible that the number of clusters is different, it should be appreciated that an important aspect of the present disclosure is the inventors' discovery that FL may be characterized into four types based upon the generation of FL TME signatures using methods described herein. In some embodiments, FL TME types include normal-like type, PC-like (or T Helper (TH)-depleted) type, light Zone (LZ)-like type, and dark Zone (DZ)-like type.

The FL TME types described herein may be described by qualitative characteristics, for example high signals for certain gene expression signatures or scores or low signals for certain other gene expression signatures or scores. In some embodiments, a "high" signal refers to a gene expression signal or score (e.g., an enrichment score, or score produced using B cell associated gene groups) that is at least 1-fold, 2-fold, 3-fold, 4-fold, 5-fold, 6-fold, 7-fold, 8-fold, 9-fold, 10-fold, 20-fold, 50-fold, 100-fold, 1000-fold, or more increased relative to the score of the same gene or gene group in a subject having a different type of FL. In some embodiments, a "low" signal refers to a gene expression signal or score (e.g., an enrichment score, or score produced using B cell associated gene groups) that is at least 1-fold, 2-fold, 3-fold, 4-fold, 5-fold, 6-fold, 7-fold, 8-fold, 9-fold, 10-fold, 20-fold, 50-fold, 100-fold, 1000-fold, or more decreased relative to the score of the same gene or gene group in a subject having a different type of FL TME.

Without wishing to be bound by any theory, the tumor microenvironment of FL may contain variable numbers of immune cells, stromal cells, blood vessels and extracellular matrix. In some embodiments, normal-like type of FL TME is characterized by the highest stromal signal and high effector cell signal, relative to other types of FL TME, as measured by a first gene expression signal or second gene expression signal. High signal of Memory, Naive and Plasma cell signatures are determined from scores of B-cell related gene groups using the techniques described herein. In some embodiments, normal-like type of FL TME is characterized by the highest signal of NF-kB signature. Normal-like type of FL TME is most similar to a normal lymph node in the selected signature space. For example, most normal lymph node and tonsil samples are categorized as normal-like FL TME type when this classification type is used. In another example, transformed samples such as cancerous tissues cannot be categorized in normal-like type. In some embodiments, normal-like FL TME type is associated with the best prognosis on R-CHOP.

In some embodiments, PC-like type (or T Helper-depleted) of FL TME is characterized by the lowest CD4 to CD8 T-cell signal ratio and highest T-reg to T follicular helper ratio. In some embodiments, PC-like type FL TME has high effector cell signal. The inventors of the present disclosure identified that the CD4/CD8 ratio is strongly correlated with the effector cell signature. Accordingly, these two signatures may be used interchangeably. In some embodiments, PC-like type TME has high Plasma cell signal. In some embodiments, PC-like type FL TME is associated with intermediate prognosis on R-CHOP (e.g., a better prognosis than DZ-type and a worse prognosis than normal-type).

In some embodiments, light zone (LZ)-like type FL TME is characterized by the highest centrocyte and MHC-II signal (i.e., light zone phenotype). In some embodiments, LZ-like type FL TME has low effector cells signal. In some embodiments, LZ-like type FL TME is associated with intermediate prognosis on R-CHOP (e.g., a better prognosis than DZ-type FL TME and a worse prognosis than normal-type FL TME).

In some embodiments, dark zone (DZ)-like type FL TME is characterized by the highest centroblast and proliferation rate signal (i.e., dark zone phenotype). In some embodiments, DZ-like type FL TME has high PI3K signal. In some embodiments, DZ-like type FL TME has low Effector cell group signal. In some embodiments, DZ-like type FL TME is associated with worst prognosis on R-CHOP.

In some embodiments, the prediction of prognosis on R-CHOP can be based on Kaplan Meier (KM)-curves of a single dataset. The methods and analyses associated with KM-curves on survival prediction are well known in the art.

In some embodiments, progression-risk score can be used for determining the prediction of prognosis on R-CHOP. In some embodiments, progression-risk score can be used for evaluating the progression of FL. The use of progression-risk score is for example, described by Huet *et al.,* "A gene-expression profiling score for outcome prediction disease in patients with follicular lymphoma: a retrospective analysis on three international cohorts", the entire contents of which are incorporated by reference herein. In some embodiments, high progression-risk score is strongly enriched in DZ-like subtype. In some embodiments, low progression-risk score is strongly associated with normal-like subtype. In some embodiments, DZ-like subtype is associated with the most aggressive FL subtype.

In some embodiments, the present disclosure provides methods for providing a prognosis, predicting survival or stratifying patient risk of a subject suspected of having, or at risk of having FL. In some embodiments, the method comprises determining a FL TME type of the subject as described herein.

In some embodiments, the methods comprise identifying the subject as having an increased risk of FL progression relative to other FL TME types when the subject is assigned normal-like type. In some embodiments, "increased risk of FL progression" may indicate poor prognosis of FL or increased likelihood of having advanced disease in a subject. In some embodiments, "increased risk of FL progression" may indicate that the subject who has FL is expected to be less responsive or unresponsive to certain treatments. For instance, "increased risk of FL progression" indicates that a subject is at least 10%, 20%, 30%, 40%, 50%, 60%, 70%, 80%, 90%, or 100% less likely to experience a progression-free survival event (e.g., relapse, retreatment, or death) than another FL patient or population of FL patients (e.g., patients having FL, but not the same FL TME type as the subject).

In some embodiments, the methods further comprise identifying the subject as having a decreased risk of FL progression relative to other FL TME types when the subject is assigned DZ-like type. In some embodiments, "decreased risk of FL progression" may indicate more positive prognosis of FL or decreased likelihood of having advanced disease in a subject. In some embodiments, "decreased risk of FL progression" may indicate that the subject who has FL is expected to be more responsive to certain treatments and show improvements of disease symptoms. For instance, "decreased risk of FL progression" indicates that a subject is at least 10%, 20%, 30%, 40%, 50%, 60%, 70%, 80%, 90%, or 100% more likely to experience a progression-free survival event (e.g., relapse, retreatment, or death) than another FL patient or population of FL patients (e.g., patients having FL, but not the same FL TME type as the subject).

In some embodiments, the methods further comprise identifying the subject as having an increased risk of lacking response to R-CHOP relative to other FL TME types when the subject is assigned DZ-like type. In some embodiments, "increased risk of lacking response to R-CHOP" may indicate the subject who has FL is expected to be less responsive or unresponsive to R-CHOP. For instance, "increased risk of lacking response to R-CHOP" indicates that a subject is at least 10%, 20%, 30%, 40%, 50%, 60%, 70%, 80%, 90%, or 100% less likely to experience the efficacy of R-CHOP treatment and/or improvements on FL symptoms than another FL patient or population of FL patients (e.g., patients having FL, but not the same FL TME type as the subject).

In some embodiments, the methods further comprise providing a recommendation to administer (e.g., identifying for the patient) one or more chemotherapeutic agents to the subject based upon the identifying of the patient's FL TME type. For example, the subject who is determined to have a DZ-like or a PC-like FL TME may be recommended to receive one or more chemotherapeutic agents that are different (e.g., not R-CHOP) than another FL patient or population of FL patients (e.g., patients having FL, but not the same FL TME type(s) as the subject, who may be recommended for R-CHOP therapy). In some embodiments, the methods described herein further comprise administering the identified anti-cancer therapeutic to the subject based on the identifying of the subject's FL TME type.

In some embodiments, the methods described herein comprise the use of at least one computer hardware processor to perform the determination.

In some embodiments, the present disclosure provides a method for providing a prognosis, predicting survival or stratifying patient risk of a subject suspected of having, or at risk of having FL. In some embodiments, the method comprises determining a FL TME type of the subject as described herein.

### Updating FL TME Clusters Based on New Data

Techniques for generating FL TME clusters are described herein. It should be appreciated that the FL TME clusters may be updated as additional FL TME signatures are computed for patients. For example, once a threshold number of new FL TME signatures are obtained (e.g., 1 new signature, 10 new signatures, 100 new signatures, 500 new signatures, any suitable threshold number of signatures in the range of 10-1,000 signatures), the new signatures may be combined with the FL TME signatures previously used to generate the FL TME clusters and the combined set of old and new FL TME signatures may be clustered again (e.g., using any of the clustering algorithms described herein or any other suitable clustering algorithm) to obtain an updated set of FL TME signature clusters.

In this way, data obtained from a future patient may be analyzed in a way that takes advantage of information learned from patients whose FL TME signature was computed prior to that of the future patient. In this sense, the machine learning techniques described herein (e.g., the unsupervised clustering machine learning techniques) are adaptive and learn with the accumulation of new patient data. This facilitates improved characterization of the FL TME type that future patients may have and may improve the selection of treatment for those patients.

### Reports

In some aspects, methods disclosed herein comprise generating a report for assisting with the preparation of recommendation for prognosis and/or treatment. The generated report can provide summary of information, so that the clinician can identify the FL subtypes or suitable therapy. The report as described herein may be a paper report, an electronic record, or a report in any format that is deemed suitable in the art. The report may be shown and/or stored on a computing device known in the art (e.g., handheld device, desktop computer, smart device, website, etc.). The report may be shown and/or stored on any device that is suitable as understood by a skilled person in the art.

In some embodiments, methods disclosed herein can be used for commercial diagnostic purposes. For example, the generated report may include, but is limited to, information concerning expression levels of one or more genes from any of the gene groups described herein, clinical and pathologic factors, patient's prognostic analysis, predicted response to the treatment, classification of the FL TME environment (e.g., as belonging to one of the types described herein), the alternative treatment recommendation, and/or other information. In some embodiments, the methods and reports may include database management for the keeping of the generated reports. For instance, the methods as disclosed herein can create a record in a database for the subject (e.g., subject 1, subject 2, etc.) and populate the specific record with data for the subject. In some embodiments, the generated report can be provided to the subject and/or to the clinicians. In some embodiments, a network connection can be established to a server computer that includes the data and report for receiving or outputting. In some embodiments, the receiving and outputting of the date or report can be requested from the server computer.

*Therapeutic Methods*

Aspects of the disclosure relate to methods of treating a subject having (or suspected or at risk of having) FL based upon a determination of the FL TME type of the subject. In some embodiments, the methods comprise administering one or more (*e.g.,* 1, 2, 3, 4, 5, or more) therapeutic agents to the subject. In some embodiments, the therapeutic agent (or agents) administered to the subject are selected from small molecules, peptides, nucleic acids, radioisotopes, cells (*e.g.,* CAR T-cells, etc.), and combinations thereof. Examples of therapeutic agents include chemotherapies (*e.g.,* cytotoxic agents, etc.), immunotherapies (*e.g.,* immune checkpoint inhibitors, such as PD-1 inhibitors, PD-L1 inhibitors, etc.), antibodies (*e.g.,* anti-HER2 antibodies), cellular therapies (*e.g.* CAR T-cell therapies), gene silencing therapies (*e.g.,* interfering RNAs, CRISPR, etc.), antibody-drug conjugates (ADCs), and combinations thereof.

In some embodiments, a subject is administered an effective amount of a therapeutic agent. "An effective amount" as used herein refers to the amount of each active agent required to confer therapeutic effect on the subject, either alone or in combination with one or more other active agents. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons, or for virtually any other reasons.

Empirical considerations, such as the half-life of a therapeutic compound, generally contribute to the determination of the dosage. For example, antibodies that are compatible with the human immune system, such as humanized antibodies or fully human antibodies, may be used to prolong half-life of the antibody and to prevent the antibody being attacked by the host's immune system. Frequency of administration may be determined and adjusted over the course of therapy, and is generally (but not necessarily) based on treatment, and/or suppression, and/or amelioration, and/or delay of a cancer. Alternatively, sustained continuous release formulations of an anti-cancer therapeutic agent may be appropriate. Various formulations and devices for achieving sustained release are known in the art.

In some embodiments, dosages for an anti-cancer therapeutic agent as described herein may be determined empirically in individuals who have been administered one or more doses of the anti-cancer therapeutic agent. Individuals may be administered incremental dosages of the anti-cancer therapeutic agent. To assess efficacy of an administered anti-cancer therapeutic agent, one or more aspects of a cancer (e.g., tumor microenvironment, tumor formation, tumor growth, or FL TME types, etc.) may be analyzed.

Generally, for administration of any of the anti-cancer antibodies described herein, an initial candidate dosage may be about 2 mg/kg. For the purpose of the present disclosure, a typical daily dosage might range from about any of 0.1 $\mu$g/kg to 3 $\mu$g/kg to 30 $\mu$g/kg to 300 $\mu$g/kg to 3 mg/kg, to 30 mg/kg to 100 mg/kg or more, depending on the factors mentioned above. For repeated administrations over several days or longer, depending on the condition, the treatment is sustained until a desired suppression or amelioration of symptoms occurs or until sufficient therapeutic levels are achieved to alleviate a cancer, or one or more symptoms thereof. An exemplary dosing regimen comprises administering an initial dose of about 2 mg/kg, followed by a weekly maintenance dose of about 1 mg/kg of the antibody, or followed by a maintenance dose of about 1 mg/kg every other week. However, other dosage regimens may be useful, depending on the pattern of pharmacokinetic decay that the practitioner (e.g., a medical doctor) wishes to achieve. For example, dosing from one-four times a week is contemplated. In some embodiments, dosing ranging from about 3 $\mu$g/mg to about 2 mg/kg (such as about 3 $\mu$g/mg, about 10 $\mu$g/mg, about 30 $\mu$g/mg, about 100 $\mu$g/mg, about 300 $\mu$g/mg, about 1 mg/kg, and about 2 mg/kg) may be used. In some embodiments, dosing frequency is once every week, every 2 weeks, every 4 weeks, every 5 weeks, every 6 weeks, every 7 weeks, every 8 weeks, every 9 weeks, or every 10 weeks; or once every month, every 2 months, or every 3 months, or longer. The progress of this therapy may be monitored by conventional techniques and assays and/or by monitoring FL TME types as described herein. The dosing regimen (including the therapeutic used) may vary over time.

When the anti-cancer therapeutic agent is not an antibody, it may be administered at the rate of about 0.1 to 300 mg/kg of the weight of the patient divided into one to three doses, or as disclosed herein. In some embodiments, for an adult patient of normal weight, doses ranging from about 0.3 to 5.00 mg/kg may be administered. The particular dosage regimen, e.g.., dose, timing, and/or repetition, will depend on the particular subject and that individual's medical history, as well as the properties of the individual agents (such as the half-life of the agent, and other considerations well known in the art).

For the purpose of the present disclosure, the appropriate dosage of an anti-cancer therapeutic agent will depend on the specific anti-cancer therapeutic agent(s) (or compositions thereof) employed, the type and severity of cancer, whether the anti-cancer therapeutic agent is administered for preventive or therapeutic purposes, previous therapy, the patient's clinical history and response to the anti-cancer therapeutic agent, and the discretion of the attending physician. Typically, the clinician will administer an anti-cancer therapeutic agent, such as an antibody, until a dosage is reached that achieves the desired result.

Administration of an anti-cancer therapeutic agent can be continuous or intermittent, depending, for example, upon the recipient's physiological condition, whether the purpose of the administration is therapeutic or prophylactic, and other factors known to skilled practitioners. The administration of an anti-cancer therapeutic agent *(e.g.,* an anti-cancer antibody) may be essentially continuous over a preselected period of time or may be in a series of spaced dose, e.g., either before, during, or after developing cancer.

As used herein, the term "treating" refers to the application or administration of a composition including one or more active agents to a subject, who has a cancer, a symptom of a cancer, or a predisposition toward a cancer, with the purpose to cure, heal, alleviate, relieve, alter, remedy, ameliorate, improve, or affect the cancer or one or more symptoms of FL, or the predisposition toward FL.

Alleviating FL includes delaying the development or progression of the disease, or reducing disease severity. Alleviating the disease does not necessarily require curative results. As used therein, "delaying" the development of a disease (e.g., a cancer) means to defer, hinder, slow, retard, stabilize, and/or postpone progression of the disease. This delay can be of varying lengths of time, depending on the history of the disease and/or individuals being treated. A method that "delays" or alleviates the development of a disease, or delays the onset of the disease, is a method that reduces probability of developing one or more symptoms of the disease in a given time frame and/or reduces extent of the symptoms in a given time frame, when compared to not using the method. Such comparisons are typically based on clinical studies, using a number of subjects sufficient to give a statistically significant result.

"Development" or "progression" of a disease means initial manifestations and/or ensuing progression of the disease. Development of the disease can be detected and assessed using clinical techniques known in the art. Alternatively, or in addition to the clinical techniques known in the art, development of the disease may be detectable and assessed based on other criteria. However, development also refers to progression that may be undetectable. For purpose of this disclosure, development or progression refers to the biological course of the symptoms. "Development" includes occurrence, recurrence, and onset. As used herein "onset" or "occurrence" of a cancer includes initial onset and/or recurrence.

Examples of the antibody anti-cancer agents include, but are not limited to, alemtuzumab (Campath), trastuzumab (Herceptin), Ibritumomab tiuxetan (Zevalin), Brentuximab vedotin (Adcetris), Ado-trastuzumab emtansine (Kadcyla), blinatumomab (Blincyto), Bevacizumab (Avastin), Cetuximab (Erbitux), ipilimumab (Yervoy), nivolumab (Opdivo), pembrolizumab (Keytruda), atezolizumab (Tecentriq), avelumab (Bavencio), durvalumab (Imfinzi), and panitumumab (Vectibix).

Examples of an immunotherapy include, but are not limited to, a PD-1 inhibitor or a PD-L1 inhibitor, a CTLA-4 inhibitor, adoptive cell transfer, therapeutic cancer vaccines, oncolytic virus therapy, T-cell therapy, and immune checkpoint inhibitors.

Examples of radiation therapy include, but are not limited to, ionizing radiation, gamma-radiation, neutron beam radiotherapy, electron beam radiotherapy, proton therapy, brachytherapy, systemic radioactive isotopes, and radiosensitizers.

Examples of a surgical therapy include, but are not limited to, a curative surgery (e.g., tumor removal surgery), a preventive surgery, a laparoscopic surgery, and a laser surgery.

Examples of the chemotherapeutic agents include, but are not limited to, R-CHOP, Carboplatin or Cisplatin, Docetaxel, Gemcitabine, Nab-Paclitaxel, Paclitaxel, Pemetrexed, and Vinorelbine. Additional examples of chemotherapy include, but are not limited to, Platinating agents, such as Carboplatin, Oxaliplatin, Cisplatin, Nedaplatin, Satraplatin, Lobaplatin, Triplatin, Tetranitrate, Picoplatin, Prolindac, Aroplatin and other derivatives; Topoisomerase I inhibitors, such as Camptothecin, Topotecan, irinotecan/SN38, rubitecan, Belotecan, and other derivatives; Topoisomerase II inhibitors, such as Etoposide (VP-16), Daunorubicin, a doxorubicin agent (e.g., doxorubicin, doxorubicin hydrochloride, doxorubicin analogs, or doxorubicin and salts or analogs thereof in liposomes), Mitoxantrone, Aclarubicin, Epirubicin, Idarubicin, Amrubicin, Amsacrine, Pirarubicin, Valrubicin, Zorubicin, Teniposide and other derivatives; Antimetabolites, such as Folic family (Methotrexate, Pemetrexed, Raltitrexed, Aminopterin, and relatives or derivatives thereof); Purine antagonists (Thioguanine, Fludarabine, Cladribine, 6-Mercaptopurine, Pentostatin, clofarabine, and relatives or derivatives thereof) and Pyrimidine antagonists (Cytarabine, Floxuridine, Azacitidine, Tegafur, Carmofur, Capacitabine, Gemcitabine, hydroxyurea, 5-Fluorouracil (5FU), and relatives or derivatives thereof); Alkylating agents, such as Nitrogen mustards (e.g., Cyclophosphamide, Melphalan, Chlorambucil, mechlorethamine, Ifosfamide, mechlorethamine, Trofosfamide, Prednimustine, Bendamustine, Uramustine, Estramustine, and relatives or derivatives thereof); nitrosoureas (e.g., Carmustine, Lomustine, Semustine, Fotemustine, Nimustine, Ranimustine, Streptozocin, and relatives or derivatives thereof); Triazenes (e.g., Dacarbazine, Altretamine, Temozolomide, and relatives or derivatives thereof); Alkyl sulphonates (e.g., Busulfan, Mannosulfan, Treosulfan, and relatives or derivatives thereof); Procarbazine; Mitobronitol, and Aziridines (e.g., Carboquone, Triaziquone, ThioTEPA, triethylenemalamine, and relatives or derivatives thereof); Antibiotics, such as Hydroxyurea, Anthracyclines (e.g., doxorubicin agent, daunorubicin, epirubicin and relatives or derivatives thereof); Anthracenediones (e.g., Mitoxantrone and relatives or derivatives thereof); Streptomyces family antibiotics (e.g., Bleomycin, Mitomycin C, Actinomycin, and Plicamycin); and ultraviolet light.

In some aspects, the disclosure provides a method for treating follicular lymphoma, the method comprising administering one or more therapeutic agents (e.g., one or more anti-cancer agents, such as one or more chemotherapeutic agents) to a subject identified as having a particular FL TME type, wherein the FL TME type of the subject has been identified by method comprising: using at least one computer hardware processor to perform obtaining RNA expression data for the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells; generating an FL TME signature for the subject using the RNA expression data, the FL TME signature comprising: a first gene expression signature comprising first gene group expression scores for respective gene groups in the first plurality of gene groups, and a second gene expression signature comprising second gene group expression scores for respective gene groups in the second plurality of gene groups associated with B cells, the generating comprising: determining the first gene expression signature by determining the first gene group expression scores using the first RNA expression levels, and determining the second gene expression signature by determining the second gene group expression scores using the second RNA expression levels; and identifying, using the FL TME signature and from among a plurality of FL TME types, an FL TME type for the subject.

In some embodiments, the subject has been identified as having an FL TME type selected from a Normal-like type, a PC-like type, a Light Zone (LZ)-like type, and a Dark Zone (DZ)-like type.

The disclosure is based, in part, on the inventors' recognition that subjects having certain FL TME types are likely to respond well to R-CHOP (a combination of Rituximab, vincristine, doxorubicin, cyclophosphamide, and prednisolone), the typical first line treatment for FL. Treatment with R-CHOP is well known, for example as described in Cunningham et al. Lancet. 2013 May 25;381(9880):1817-26. doi: 10.1016/S0140-6736(13)60313-X, the entire contents of which are incorporated by reference herein. In some embodiments, the therapeutic agent comprises R-CHOP when the subject has been identified as having Normal-like type, PC-like type, or Light Zone-like type. In some embodiments, the R-CHOP is administered to the subject at the following dosages: Rituximab- 375 mg/m$^2$ IV, vincristine- 1.4 mg/m$^2$ IV, doxorubicin- 50 mg/m$^2$ IV, cyclophosphamide 750 mg/m$^2$ IV, and prednisolone 100 mg PO (orally). In some embodiments, the R-CHOP is administered to the subject every 21 days. In some embodiments, the subject is administered the R-CHOP every 21 days for between 3 and 6 (e.g., 3, 4, 5, or 6) cycles of treatment.

Aspects of the disclosure are based on the inventors' recognition that subjects having certain FL TME types are unlikely to respond well (e.g., have an increased risk of having refractory FL) to certain conventional FL therapies, such as R-CHOP. Thus, in some embodiments, the therapeutic agent comprises a therapeutic agent other than R-CHOP when the subject has been identified as having a Dark Zone-like type (e.g., the subject is not administered R-CHOP). Examples of second-line FL therapies include but are not limited to axicabtagene ciloleucel (Yescarta), bendamustine (Treanda) with or without rituximab (Rituxan), obinutuzumab (Gazyva), Copanlisib (Aliqopa), Copiktra (duvelisib), Fludarabine (Fludara) and rituximab (Rituxan), Idelalisib (Zydelig), Lisocabtagene Maraleucel (liso-cel, Breyanzi), R$^2$ - rituximab and lenalidomide (Rituxan and Revlimid), R-CVP (rituximab, cyclophosphamide, vincristine, and prednisone), R-FND (rituximab, fludarabine, mitoxantrone, and dexamethasone), Rituximab and Hyaluronidase Human (Rituxan Hycela), R-DHAP (Rituximab, dexamethasone, cytarabine, and cisplatin), R-ICE (rituximab, ifosfamide, carboplatin, and etoposide phosphate), R-ESHAP (rituximab, etoposide, solu-medrone, high-dose cytarabine, and cisplatin), Tazemetostat (TAZVERIK), Umbralisib (UKONIQ).

In some embodiments, a subject having Dark-zone type FL is identified as a candidate for, or administered, a stem cell transplant, for example autologous stem cell transplantation or allogeneic stem cell transplantation.

EXAMPLES

*Example 1: Identification of Follicular Lymphoma (FL) Tumor Microenvironment (TME)*

This example describes an illustrative technique for generating an FL TME signature for a subject from RNA expression data for the subject, according to some embodiments of the technology described herein. The produced FL TME signature reflects and/or indicates the abundance of both the malignant and microenvironment (TME) cell subpopulations and the activity of tumor-promoting and tumor-suppressive processes occurring within a tumor, and constitutes a personalized tumor map.

The generated FL TME signature for the subject is used to identify an FL TME type for the subject from among four FL TME types: Normal-like type, PC-like (or T Helper (TH)-depleted) type, Light Zone (LZ)-like type, and the Dark Zone (DZ)-like type.

Aspects of some of the steps of the process described in this example are described in further detail herein including with reference to FIGs. 1-6 above.

Follicular lymphoma (FL) is one of the most frequent indolent B cell lymphomas having a connection with tumor microenvironment (TME). In lymphomagenesis, malignant cells depend on signals from surrounding cells.

RNA expression data (including both RNA-seq and microarray expression data) were obtained from multiple public databases. Data were subjected to basic quality control (QC) measures. For example, outlier samples and samples with signs of RNA degradation were excluded. Preprocessing of expression data included normalization and log-transformation. For microarrays normalization is performed automatically using gcrma package. RNA-seq data was subsequently normalized to TPM (transcript per million) units. TPM normalization techniques are described in Wagner et al. (Theory Biosci. (2012) 131:281-285), which is incorporated by reference herein in its entirety. TPM normalization may be performed using a software package, such as, for example, the gcrma package. Aspects of the gcrma package are described in Wu J, Gentry RIwcfJMJ (2021). "gcrma: Background Adjustment Using Sequence Information. R package version 2.66.0.", which is incorporated by reference in its entirety herein. In some embodiments, RNA expression level in TPM units for a particular gene may be calculated according to:

$$A \cdot \frac{1}{\sum(A)} \cdot 10^6$$

$$A = \frac{total\ reads\ mapped\ to\ gene \cdot 10^3}{gene\ length\ in\ bp}$$

*Where*

The FL TME signature determined for the subject includes a first gene expression signature and a second gene expression signature. The first gene expression signature includes scores for gene groups obtained using ssGSEA. The gene groups for the first gene expression signature were selected based on relevance to follicular lymphoma (FL) and the correlation of the genes in connection with different aspects of the lymph nodes, tumors and their microenvironment. The second gene expression signature includes scores for gene groups associated with B cells. These scores were produced using vectors of coefficients for each gene set of the B cell associated gene groups.

The gene group scores in the first and second signatures were calculated from log-transformed RNA expression values. After calculation, the scores were scaled using median-scaling, which was important for removing undesirable batch effects and to enable all the datasets to be combined together.

Median scaling consisted of estimating median and MAD (median absolute deviation) for each signature within each dataset, and applying the formula xi-median(x)/MAD(x).

In certain examples, the FL TME signature includes other one or more other signatures. In some examples, PROGENy signatures (e.g., NFKB or PI3K) were used to create a third gene expression signature.

In certain examples, the FL TME signature includes ratios of gene scores for one or more gene groups in the first gene expression signature. Initially ratios were selected based on biology of a normal lymph node; for example CD4/CD8 ratio is approximately 2:1 normally, and bias towards CD8 may indicate disruption of normal microenvironment structure. The score of ratio between signature A and signature B is defined as score(A)-score(B), these values are than scaled in the same way as all other scores. Thus, in some examples, the FL TME signature includes the ratio of scores for the CD4+ gene group and the CD8+ gene group. However, the inclusion of these ratios is optional and not necessary for the generation of FL TME signatures.

As described above, the second gene expression signature was produced using gene groups associated with B cells. Multiple different approaches were tried.

In one example, previously-described B cell associated gene set model (BAGS), which uses pre-defined gene sets, was used. The BAGS gene sets are described in Dybkær K et al., Diffuse large B-cell lymphoma classification system that associates normal B-cell subset phenotypes with prognosis. J Clin Oncol. 2015;33(12):1379-1388, which is incorporated by reference herein in its entirety. A BAGS gene set score was calculated by taking a dot product between log-normalized expression values for genes in the BAGS gene set and coefficients of a corresponding multinomial regression model, which is also described in Dybkær K et al., Diffuse large B-cell lymphoma classification system that associates normal B-cell subset phenotypes with prognosis. J Clin Oncol. 2015;33(12):1379-1388.

In another example, the BAGS gene sets were not used. Instead, new gene sets were identified using machine learning feature selection techniques. For this, a large dataset of different types of sorted B-cells was collected. Using the "shap" and gradient boosting techniques (e.g., as implemented using the Light GBM software package) for each of B-cell subtypes (naive, centrocyte, centroblast, memory, and plasmacyte) genes that best separate each B cell subtype from all others were selected. The resulting gene set, organized into gene groups, was significantly smaller (e.g., see Table 2) than the gene sets used for the BAGS classifier. These genes were then used as features in logistic regression models which were trained to distinguish a particular cell type from the others. Coefficients of these models were then used to calculate scores in FL samples by taking dot product of coefficient vector and expression vectors. Resulting values were then scaled.

The "shap" technique is described in Lundberg, Scott M., and Su-In Lee. "A unified approach to interpreting model predictions." Advances in Neural Information Processing Systems. 2017, which is incorporated by reference herein in its entirety. The "lgbm" technique is described in Ke, G., Meng, Q., Finley, T., Wang, T., Chen, W., Ma, W., ... Liu, T.-Y. (2017). Lightgbm: A highly efficient gradient boosting decision tree. Advances in Neural Information Processing Systems, 30, 3146-3154, which is incorporated by reference herein in its entirety.

After FL TME signatures were calculated according to the above for multiple patients, unsupervised clustering was performed to generate FL TME clusters. Dense clustering and spectral clustering algorithms were the most appropriate. To classify a new sample, it is grouped together with the dataset used to get the subtypes. Scores are calculated for the sample and scaled together with the selected cohort. After that the sample subtype, can be predicted by applying a machine learning model (e.g., K-nearest neighbor, "knn") trained on the scaled metacohort.

*Characteristics of Follicular Lymphoma Types*

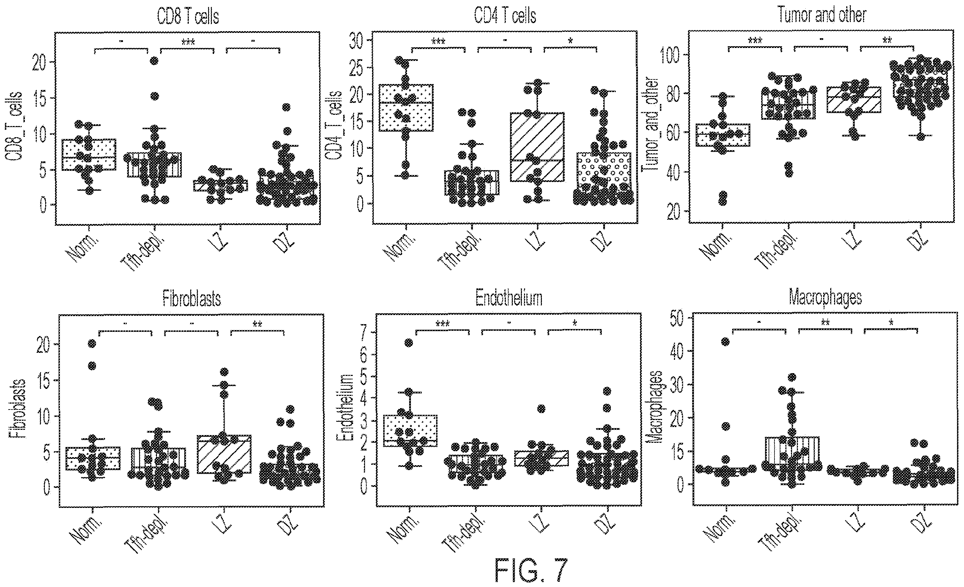

Using the aforementioned approach on several publicly available cancer data sets, four distinct types of FL were observed (FIG. 7):

- **Normal-like type.** This type is most similar to a normal lymph node in the selected signature space. In terms of a microenvironment, it is characterized by the highest stromal signal and high effector cell signal. It also is characterized by high Memory cell, Plasma cell, and Naive cell group signatures, as determined from the B cell associated gene groups. This FL TME type is also characterized by the highest signal of NFkB PROGENy signature relative to other FL TME types. Most normal lymph node and tonsil samples fall into this type if they are classified according to this model. Transformed FL (tFL) samples do not fall into this FL TME type. Subjects have an intermediate prognosis on R-CHOP.
- **Plasma cell (PC)-like (or T Helper (TH)-depleted) type.** This type has the lowest CD4 to CD8[+] T-cell signal ratio and the highest T-reg group to T follicular helper group ratio. It is also characterized by a high Effector cell group signal in the first gene expression signature. A high Plasma cell group B cell associated gene group signal is also present. Subjects have an intermediate prognosis on R-CHOP.
- **Light Zone (LZ)-like type.** This FL TME type has the highest Centrocyte group and MHC-II group signals (light zone phenotype). It has a low Effector cell group signal. Subjects have the best prognosis on R-CHOP.
- **Dark Zone (DZ)-like type.** This FL TME type has the highest Centroblast group and Proliferation Rate group signals (e.g., a dark zone phenotype), high PI3K signal. It has a low Effector cell group signal. Subjects have the worst prognosis on R-CHOP.

For RNA-seq samples, different cell content of each type was also supported by a cell deconvolution algorithm. This algorithm allows for the reconstruction of cell composition from bulk RNA-seq data and estimating the percentage of different cell types (fibroblasts, B cells, T cells, macrophages, etc.). In some embodiments, cell deconvolution algorithms may be used as a control to confirm that the cell types identified by FL TME type agree with cell types identified by other phenotype-based methods. The differences between the FL TME types are presented in FIG. 7. Values for each signature in each subtype are provided below in Tables 3A-3C.

Table 3A: Median score values for FL TME types

|  | Normal-like | PC-like | LZ-like | DZ-like |
|---|---|---|---|---|
| Effector_cells | 0.53 | 0.72 | -0.63 | -0.56 |
| Memory | 0.56 | 0.45 | -0.44 | -0.55 |
| Follicular dendritic cells | 0.62 | 0.72 | -0.53 | -0.69 |
| Treg | 0.66 | 0.34 | -0.48 | -0.61 |
| Follicular_helper_T_cells | 0.67 | 0.31 | -0.61 | -0.66 |
| Lymphatic_endothelium | 0.09 | 0.82 | -0.52 | -0.39 |
| Naive | 0.57 | -0.58 | 0.64 | -0.55 |
| Plasma | 0.11 | 0.71 | -0.63 | -0.14 |
| M2_signature | 0.37 | 0.8 | -0.51 | -0.7 |
| Centrocyte | 0.67 | -0.84 | 0.76 | -0.73 |
| MHCII | 0.55 | -0.71 | 0.78 | -0.8 |
| Proliferation_rate | 0.52 | -0.86 | 0.55 | -0.33 |
| Centroblast | -0.32 | -0.38 | 0.53 | 0.39 |

Table 3B: 25th percentile score values for FL TME types

|  | Normal-like | PC-like | LZ-like | DZ-like |
|---|---|---|---|---|
| Effector_cells | -0.05 | 0.18 | -1.18 | -1.08 |
| Memory | -0.27 | -0.3 | -0.85 | -1.06 |
| Follicular_dendritic_cells | 0.25 | 0.29 | -1.13 | -1.24 |
| Treg | 0.17 | -0.36 | -0.94 | -1.12 |
| Follicular_helper_T_cells | -0.11 | -0.33 | -1.28 | -1.31 |
| Lymphatic_endothelium | -0.33 | 0.12 | -1.24 | -0.86 |
| Naive | 0.19 | -1.13 | -0.36 | -1.33 |
| Plasma | -0.63 | 0.25 | -1.25 | -0.84 |
| M2_signature | 0.1 | 0.2 | -0.84 | -1.1 |
| Centrocyte | 0.36 | -1.26 | 0.27 | -1.16 |
| MHCII | 0.11 | -1.22 | 0.32 | -1.34 |
| Proliferation_rate | 0.06 | -1.29 | -0.07 | -1.15 |
| Centroblast | -0.87 | -1.02 | -0.32 | -0.14 |

Table 3C: 75th percentile score values for FL TME types

|  | Normal-like | PC-like | LZ-like | DZ-like |
|---|---|---|---|---|
| Effector_cells | 1 | 1.29 | -0.09 | -0.24 |
| Memory | 1.25 | 1.02 | 0.2 | 0.05 |
| Follicular dendritic cells | 1.04 | 1.26 | -0.07 | -0.29 |
| Treg | 1.32 | 0.98 | -0.05 | -0.12 |
| Follicular_helper_T_cells | 1.32 | 0.84 | -0.04 | 0.02 |

(continued)

|  | Normal-like | PC-like | LZ-like | DZ-like |
|---|---|---|---|---|
| Lymphatic_endothelium | 0.74 | 1.43 | 0.15 | 0.13 |
| Naive | 1.09 | 0.03 | 1.13 | 0.07 |
| Plasma | 0.65 | 1.17 | 0.08 | 0.61 |
| M2 signature | 1.03 | 1.6 | -0.04 | -0.16 |
| Centrocyte | 1.08 | -0.41 | 1.22 | -0.23 |
| MHCII | 0.92 | -0.23 | 1.12 | -0.27 |
| Proliferation_rate | 0.92 | -0.24 | 1.04 | 0.61 |
| Centroblast | 0.15 | 0.19 | 0.95 | 0.96 |

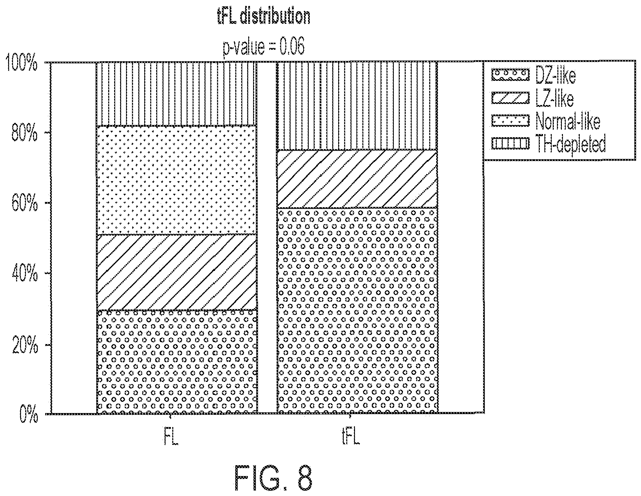

Understanding the mechanism of FL transformation is a question of key importance in lymphomagenesis. TME FL type analysis determined an enrichment of transformed FL (tFL) in the DZ-like type, while no tFL was observed in Normal type (FIG. 8).

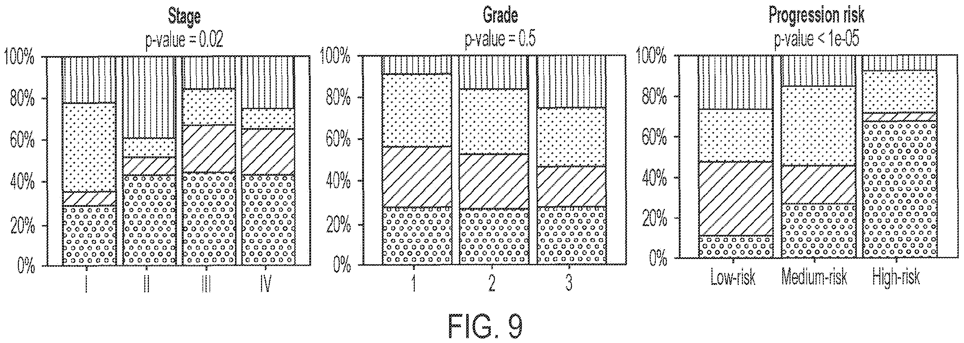

While stages and grades of FL were distributed similarly across TME FL types (FIG. 9), it was observed that DZ-like type was enriched in samples with a high risk of progression, as calculated by a previously-described risk assessment algorithm using previously published gene signatures (FIG. 9).

In addition to the progression probability insights, TME FL types were demonstrated to have prognostic and predictive power. Using the public cohort Pastore [*PMID: 26256760*] it was determined that LZ-like type had better survival, and DZ-like type showed the worst overall survival (OS) and failure free survival (FFS) (FIG. 10).

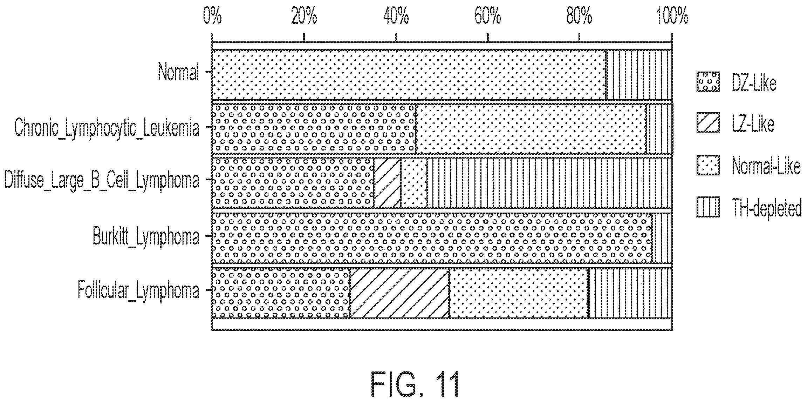

Using this approach, TME FL types were identified for normal samples from Lymph node (LN) and for samples with more aggressive B-cell lymphoma. Interestingly, the most aggressive, Burkitt lymphoma (BL), was mostly classified as DZ. On the other hand, Normal LN samples were mostly classified as normal-like and less than 20% as Th-depleted (FIG. 11).

Thus, TME FL typing based on the combination of gene expression signatures, GES ratios, B cell phenotype prediction, and pathway scoring is a promising and applicable method for FL itself and also for other lymphoma types. The developed approach provides valuable insights into lymphomagenesis, biology of tumors and TMEs, prognosis and drug response prediction.

*Example 3: Additional embodiments*

FIG. 12 schematically provides an exemplary workflow of processing gene expression data from the datasets and determining various signature scores based on the use of the selected algorithms. The expression data was preprocessed. The preprocessing of expression data included normalization and log-transformation. For microarray assays, normalization was performed automatically using gcrma (GC Robust Multi-array Average) package. Gcrma was used to perform background adjustment, quantile normalization, and median-polish summarization on microarray data.

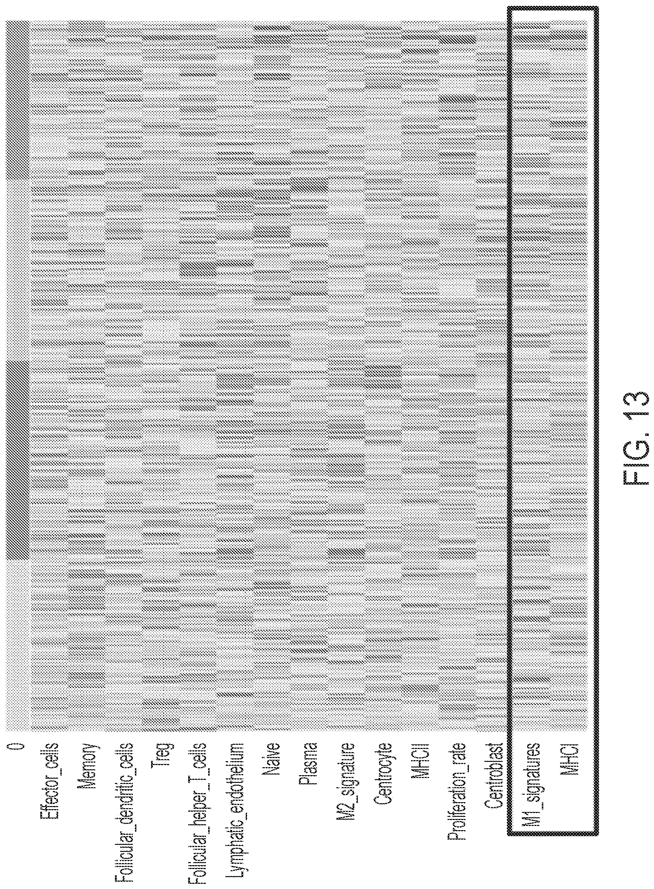

The clustered gene signatures and the classified FL samples were demonstrated in heatmaps. Gene signatures that appeared to introduce noise and therefore were inconclusive were identified and excluded. FIG. 13 provides an exemplary illustration of a heatmap where the addition of the M1 and MHC-I gene signatures represented noisy gene signatures.

Table 4: Exemplary NCBI Accession Numbers for genes listed in Table 1.

| Gene | Accession Number(s) |
|---|---|
| ADAMDEC1 | NM_001145271, NM_001145272, NM_014479 |
| ADAP2 | XM_024450832, NM_001346714, XM_024450835, XM_024450834, XM_024450831, NM_001346712, NM_018404, NR_144488, XM_024450833, NM_001346716 |
| ADORA3 | NM_000677, NM_001302678, NM_001302679 |
| APOC2 | NM_000483 |
| APOE | NM_000041, NM_001302689, NM_001302690, NM_001302691, NM_001302688 |

(continued)

| Gene | Accession Number(s) |
|------|---------------------|
| AURKA | NM_001323303, NM_001323304, NM_001323305, NM_003600, NM_198433, NM_198434, NM_198435, NM_198436, NM_198437, XM_017028035 |
| AURKB | NM_001256834, NM_001284526, NM_001313950, NM_001313951, NM_001313952, NM_001313953, NM_001313954, NM_001313955, NM_004217, XM_017025310, XM_017025311, XM_017025309, XM_017025307, NR_132730, NR_132731, XM_011524072, XM_017025308 |
| BCL6 | NM_001706.5, NM_001130845.2, NM_001134738.1, XM_005247694.4, XM_011513062.3 |
| BST1 | NM_004334, XM_011513881 |
| BUB1 | NM_001278617, NM_004336 |
| C1QA | NM_015991, NM_001347465, NM_001347466 |
| C1QC | NM_001347619, NM_001347620, NM_001114101, NM_172369 |
| C1S | NM_001346850, NM_001734, NM_201442, XM_005253760 |
| C3AR1 | NM_001326477, NM_004054, NM_001326475 |
| C4A | NM_001002029, NM_001252204, NM_007293 |
| C5AR1 | XM_005259190, NM_001736 |
| CCL21 | NM_002989 |
| CCL7 | NM_006273 |
| CCNB1 | NM_031966 |
| CCND1 | NM_053056 |
| CCNE1 | NM_001238, NM_001322262, NM_001322259, NM_001322261 |
| CCR1 | NM_001295 |
| CCR8 | NM_005201 |
| CD14 | NM_001174104, NM_001040021, NM_000591, NM_001174105 |
| CD160 | NM_007053, XM_011509104, XM_005272929, NR_103845 |
| CD163 | XR_002957389, NM_004244, NM_203416, XM_024449278, NM_001370146, NM_001370145, NR_163255 |
| CD28 | NM_001243078, XM_011512195, NM_001243077, XM_011512194, XM_011512197, NM_006139 |
| CD33 | XM_017027509, XM_011527531, XM_011527532, NM_001177608, XM_017027510, NM_001082618, XM_017027508, NM_001772 |
| CD4 | NM_001195017, NM_001382707, NM_001382705, NM_001382706, NM_001195015, NR_036545, NM_001195016, NM_000616, NM_001195014, NM_001382714 |
| CD40LG | NM_000074 |
| CD68 | NM_001040059, NM_001251 |
| CD84 | NM_001184882.2, NM_003874.4, NM_001184879.2, NM_001330742.2, NM_001184881.2, XR_002957960.1, XR_921991.3, XM_011510095.2 |
| CD8A | NR_168478, NM_001145873, NM_001382698, NR_168480, NM_001768, NM_171827, NR_027353, NR_168481, NR_168479 |
| CD8B | NM_172101, NM_172213, NM_172102, NM_001178100, NM_004931, NM_172099, XM_011533164 |
| CDK2 | NM_052827, NM_001798, NM_001290230, XM_011537732 |

(continued)

| Gene | Accession Number(s) |
|------|---------------------|
| CETN3 | NM_001297768, NM_001297765, NM_004365 |
| CIITA | NM_001286402, XM_006720880, XM_011522485, XM_011522487, XM_011522489, XR_932842, XR_932846, NM_001286403, NR_104444, XM_011522491, NM_000246, XM_011522484, XM_011522486, XM_024450280, NM_001379330, XR_932847, XM_011522494, NM_001379333, XM_024450281, XM_011522490, XR_001751904, NM_001379332, NM_001379334, XR_932841, NM_001379331 |
| CLEC10A | NM_001330070, NM_006344, NM_182906 |
| CLEC5A | NM_001301167, XM_017011916, NM_013252, XM_017011915, XM_017011917, XM_011515995 |
| CLU | NM_001831 |
| CMKLR1 | NM_001142345, NM_004072, NM_001142343, NM_001142344, XM_017018820 |
| CSF1R | NM_001288705, NM_001349736, NM_001375320, NR_109969, NR_164679, NM_001375321, NM_005211 |
| CTLA4 | NM_001037631, NM_005214 |
| CXADR | NM_001207063, NM_001207064, NM_001207065, NM_001207066, NM_001338, XM_011529479 |
| CXCL12 | NM_000609, NM_001033886, NM_001178134, NM_001277990, NM_199168 |
| CXCR5 | NM_001716, NM_032966 |
| CYBB | NM_000397 |
| E2F1 | NM_005225 |
| EDNRB | NM_001201397, NM_003991, NM_000115, NM_001122659 |
| EOMES | NM_005442, NM_001278182, XM_005265510, NM_001278183 |
| ESCO2 | NM_001017420, XM_011544421 |
| FASLG | NM_001302746, NM_000639 |
| FDCSP | NM_152997 |
| FLT4 | NM_002020, NM_182925 |
| FOXC2 | NM_005251 |
| FOXP3 | XM_006724533, XM_017029567, NM_001114377, NM_014009 |
| FPR3 | NM_002030, XM_011526687 |
| GNLY | NM_001302758, NM_006433, NM_012483 |
| GZMA | NM_006144 |
| GZMB | NM_001346011, NR_144343, NM_004131 |
| GZMK | NM_002104 |
| HLA-DMA | NM_006120 |
| HLA-DMB | NM_002118 |
| HLA-DPA1 | NM_001242524, NM_001242525, NM_033554 |
| HLA-DPB1 | NM_002121 |
| HLA-DQA1 | NM_002122 |
| HLA-DQB1 | NM_001243962, NM_002123, NM_001243961 |
| HLA-DRA | NM_019111 |

(continued)

| Gene | Accession Number(s) |
|---|---|
| HLA-DRB1 | NM_002124, NM_001359193, XM_024452553, NM_001359194, XR_002958969, NM_001243965, XR_002958970 |
| ICOS | NM_012092 |
| IFI30 | NM_006332 |
| IFNG | NM_000619 |
| IKZF2 | XM_005246385, XM_011510818, NM_001371277, XM_011510809, XM_005246386, XM_011510810, XM_011510803, XM_011510804, XM_011510812, XM_011510815, XM_011510817, XM_017003592, NM_001371275, XM_011510808, NM_001371274, NM_016260, XM_011510802, XM_011510807, XM_011510819, NM_001371276, XM_005246384, XM_011510805, XM_011510811, XM_017003591, XM_011510816, NM_001079526 |
| IKZF4 | XM_005269089, XM_017019813, XM_017019815, XM_024449128, XM_024449129, NM_001351090, XM_017019807, XM_017019812, XM_024449131, NM_001351089, XM_011538664, XM_011538669, XM_017019814, XM_017019808, XM_024449130, NM_001351092, XM_017019806, XM_017019809, XM_017019810, NM_022465, XM_005269086, XM_017019811, XM_017019816, NM_001351091 |
| IL10 | NM_001382624, NM_000572, NR_168466, NR_168467 |
| IL21 | NM_021803.4, NM_001207006.3 |
| IL4 | NM_000589, NM_172348 |
| IL4I1 | NM_001258017, NM_001258018, NM_152899, NR_047577, NM_172374 |
| IL6 | NM_000600.5, NM_001318095.2, NM_001371096.1, XM_005249745.5, XM_011515390.2 |
| JAM2 | NM_001270407, NM_001270408, NM_021219 |
| JAM3 | NM_001205329, NM_032801 |
| KLRC2 | NM_002260 |
| KLRK1 | NM_007360 |
| KMO | NM_003679 |
| LILRB4 | XR_002958246, XM_017026217, XM_017026215, NM_001278428, XM_024451331, NM_001278426, NM_001278429, NM_001278430, NM_001278427, NM_006847, XM_017026216, NM_001081438 |
| LTBR | NM_001270987, NM_002342 |
| LYVE1 | NM_006691 |
| MAF | XM_017023233, XM_017023234, XM_017023235, NM_001031804, NM_005360 |
| MCM2 | NM_001278595, NM_005916, NM_182776, NM_004526 |
| MCM6 | NM_005915 |
| MKI67 | NM_001145966, NM_002417 |
| MMP9 | NM_004994 |
| MRC1 | NM_002438, NM_001009567 |
| MS4A4A | NM_024021, NM_001243266, NM_148975, XM_017017909 |
| MS4A7 | NM_206938, NM_206939, NM_206940, NM_021201 |
| MSR1 | NM_138715, NM_001363744, NM_002445, XM_024447161, NM_138716 |
| MYBL2 | NM_001278610, NM_002466 |

(continued)

| Gene | Accession Number(s) |
|---|---|
| NKG7 | XM_005258955, NM_005601, XM_006723228, NM_001363693 |
| OLR1 | NM_002543, NM_001172632, NM_001172633 |
| PDPN | NM_001006624, NM_001006625, NM_006474, NM_198389, XM_006710295 |
| PLA2G7 | NM_001168357, XR_001743639, NM_005084, XR_002956305, XM_005249408 |
| PLK1 | NM_005030 |
| PPP1R13B | NM_015316 |
| PRF1 | NM_001083116, NM_005041 |
| PRNP | NM_001271561, NM_000311, NM_001080121, NM_001080122, NM_001080123, NM_183079 |
| PROX1 | NM_001270616, NM_002763, XM_017001833 |
| RAB7B | NM_177403, NM_001164522, NM_001304839, XM_006711288 |
| SERPINE2 | NM_006216, XM_017004330, XM_017004332, NR_073116, XM_005246641, NM_001136528, NM_001136530 |
| SH2D1A | NM_002351, NM_001114937 |
| SIGLEC1 | NM_001367089, NM_023068 |
| SLAMF8 | NM_001330741, NM_020125 |
| SOX18 | NM_018419 |
| SPP1 | NM_001040058, NM_000582, NM_001040060, NM_001251830, NM_001251829, NM_030791 |
| TBX21 | NM_013351 |
| TNFRSF18 | NM_148902, NM_148901, XM_017002722, NM_004195 |
| TNFRSF1A | NM_001346091, NM_001065, NM_001346092 |
| TRAC | NG_001332.3 |
| TRAT1 | NM_016388, NM_001317747 |
| TREM2 | NM_001271821, NM_018965 |
| VSIG4 | NM_001184831, NM_001257403, XM_017029251, NM_007268, NM_001100431, NM_001184830 |
| ZAP70 | XM_017004868, XR_001738926, XR_001738927, NM_001378594, NM_207519, XM_017004867, XR_001738925, NM_001079, XM_017004869, XM_017004870 |
| VEGFA | NM_001171623.2, NM_001171629.2, NM_001171627.2, NM_001171624.2, NM_001171630.2, NM_001171628.2, NM_001171626.2, NM_001204384.2, NM_001171625.2, NM_001025366.3, NM_001025368.3, NM_001204385.2, NM_001287044.2, NM_001025370.3, NM_001171622.2, NM_003376.6, NM_001033756.3, NM_001025369.3, NM_001025367.3, NM_001317010.1 |
| TGFB1 | NM_000660.7, XM_011527242.2 |
| IDO1 | NM_002164.6 |
| PTGES | NM_004878.5 |
| CSF1 | NM_000757.6, NM_172210.3, NM_172211.4, NM_172212.3, XM_017000369.1 |
| LRP1 | NM_002332.3, XM_017019303.1 |
| ARG1 | NM_000045.4, NM_001244438.2, NM_001369020.1, NR_160934.1 |

(continued)

| Gene | Accession Number(s) |
|---|---|
| PTGS1 | NM_000962.4, NM_080591.3, NM_001271164.2, NM_001271165.2, NM_001271166.2, NM_001271367.2, NM_001271368.2, XM_005252105.3, XM_011518875.2, XM_011518876.2, XM_024447614.1, XM_024447615.1 |

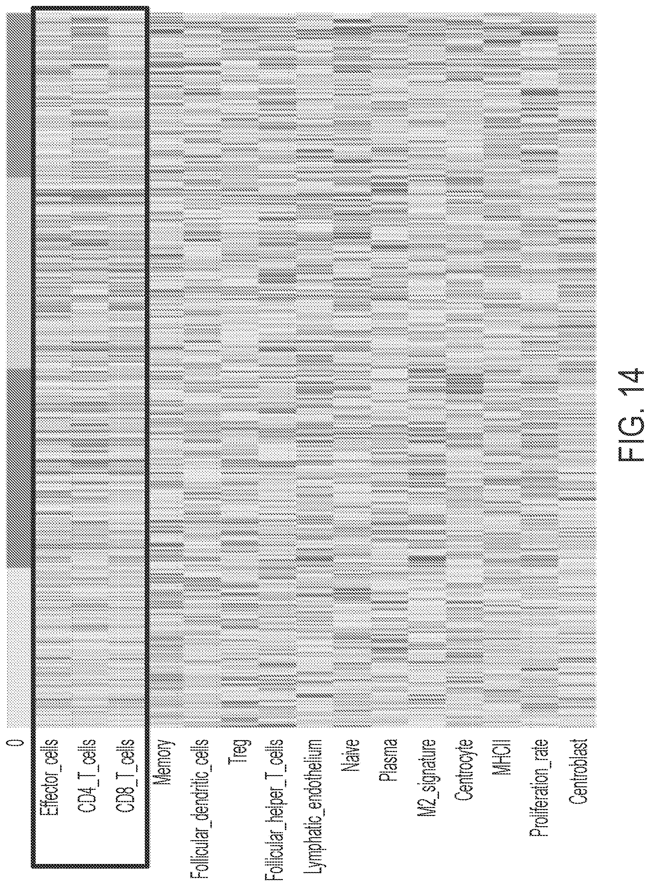

FIG. 14 shows the correlation of the gene groups and the distinct FL subtypes (DZ-like, PC-like, LZ-like, or Normal-like), and the CD4 gene group and CD8 gene group can be used as separate signatures, but they strongly correlate with Effector cells group and are thus redundant.

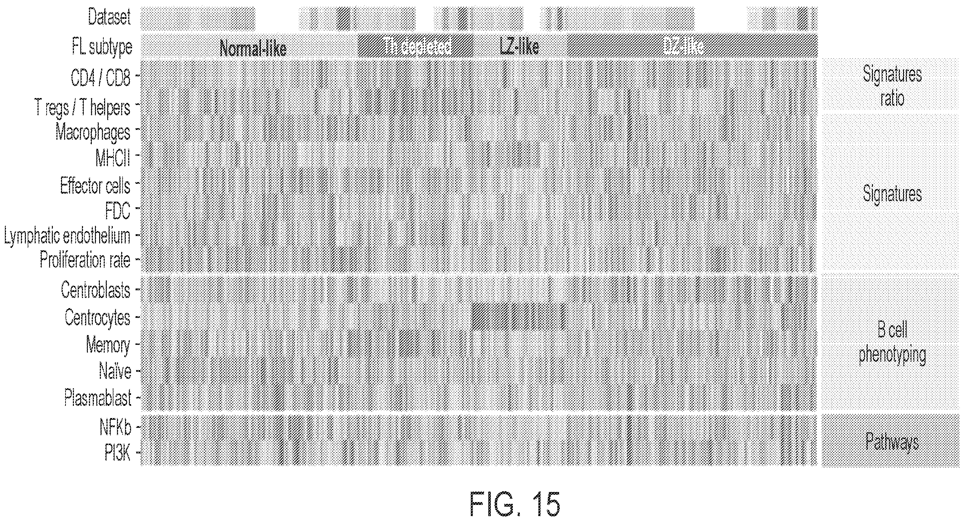

The clustered gene signatures and the classified FL samples were demonstrated in heatmaps (FIG. 15) to show the correlation inclusion of PROGENy signatures ("Pathways") to the FL TME signature.

*Computer Implementation*

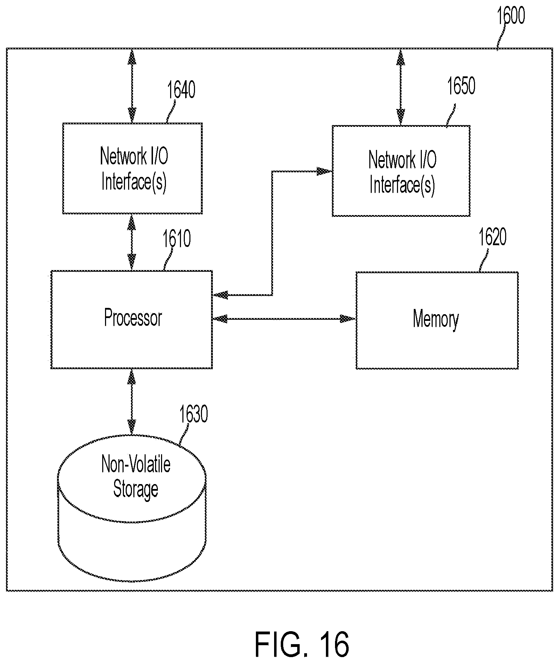

An illustrative implementation of a computer system 1600 that may be used in connection with any of the embodiments of the technology described herein (e.g., such as the method of FIG. 1) is shown in FIG. 16. The computer system 1600 includes one or more processors 1610 and one or more articles of manufacture that comprise non-transitory computer-readable storage media (e.g., memory 1620 and one or more non-volatile storage media 1630). The processor 1610 may control writing data to and reading data from the memory 1020 and the non-volatile storage device 1630 in any suitable manner, as the aspects of the technology described herein are not limited to any particular techniques for writing or reading data. To perform any of the functionality described herein, the processor 1610 may execute one or more processor-executable instructions stored in one or more non-transitory computer-readable storage media (e.g., the memory 1620), which may serve as non-transitory computer-readable storage media storing processor-executable instructions for execution by the processor 1610.

Computing device 1600 may also include a network input/output (I/O) interface 1640 via which the computing device may communicate with other computing devices (e.g., over a network), and may also include one or more user I/O interfaces 1050, via which the computing device may provide output to and receive input from a user. The user I/O interfaces may include devices such as a keyboard, a mouse, a microphone, a display device (e.g., a monitor or touch screen), speakers, a camera, and/or various other types of I/O devices.

The above-described embodiments can be implemented in any of numerous ways. For example, the embodiments may be implemented using hardware, software, or a combination thereof. When implemented in software, the software code can be executed on any suitable processor (e.g., a microprocessor) or collection of processors, whether provided in a single computing device or distributed among multiple computing devices. It should be appreciated that any component or collection of components that perform the functions described above can be generically considered as one or more controllers that control the above-discussed functions. The one or more controllers can be implemented in numerous ways, such as with dedicated hardware, or with general purpose hardware (e.g., one or more processors) that is programmed using microcode or software to perform the functions recited above.

In this respect, it should be appreciated that one implementation of the embodiments described herein comprises at least one computer-readable storage medium (e.g., RAM, ROM, EEPROM, flash memory or other memory technology, CD-ROM, digital versatile disks (DVD) or other optical disk storage, magnetic cassettes, magnetic tape, magnetic disk storage or other magnetic storage devices, or other tangible, non-transitory computer-readable storage medium) encoded with a computer program (i.e., a plurality of executable instructions) that, when executed on one or more processors, performs the above-discussed functions of one or more embodiments. The computer-readable medium may be transportable such that the program stored thereon can be loaded onto any computing device to implement aspects of the techniques discussed herein. In addition, it should be appreciated that the reference to a computer program which, when executed, performs any of the above-discussed functions, is not limited to an application program running on a host computer. Rather, the terms computer program and software are used herein in a generic sense to reference any type of computer code (e.g., application software, firmware, microcode, or any other form of computer instruction) that can be employed to program one or more processors to implement aspects of the techniques discussed herein.

The foregoing description of implementations provides illustration and description but is not intended to be exhaustive or to limit the implementations to the precise form disclosed. Modifications and variations are possible in light of the above teachings or may be acquired from practice of the implementations. In other implementations the methods depicted in these figures may include fewer operations, different operations, differently ordered operations, and/or additional operations. Further, non-dependent blocks may be performed in parallel.

It will be apparent that example aspects, as described above, may be implemented in many different forms of software, firmware, and hardware in the implementations illustrated in the figures. Further, certain portions of the implementations may be implemented as a "module" that performs one or more functions. This module may include hardware, such as a processor, an application-specific integrated circuit (ASIC), or a field-programmable gate array (FPGA), or a combination of hardware and software.

Having thus described several aspects and embodiments of the technology set forth in the disclosure, it is to be appreciated that various alterations, modifications, and improvements will readily occur to those skilled in the art. Such alterations, modifications, and improvements are intended to be within the spirit and scope of the technology described herein. For example, those of ordinary skill in the art will readily envision a variety of other means and/or structures for performing the function and/or obtaining the results and/or one or more of the advantages described herein, and each of such variations and/or modifications is deemed to be within the scope of the embodiments described herein. Those skilled in the art will recognize or be able to ascertain using no more than routine experimentation many equivalents to the specific embodiments described herein. It is, therefore, to be understood that the foregoing embodiments are presented by way of example only and that, within the scope of the appended claims and equivalents thereto, inventive embodiments may be practiced otherwise than as specifically described. In addition, any combination of two or more features, systems, articles, materials, kits, and/or methods described herein, if such features, systems, articles, materials, kits, and/or methods are not mutually inconsistent, is included within the scope of the present disclosure.

The above-described embodiments can be implemented in any of numerous ways. One or more aspects and embodiments of the present disclosure involving the performance of processes or methods may utilize program instructions executable by a device (e.g., a computer, a processor, or other device) to perform, or control performance of, the processes or methods. In this respect, various inventive concepts may be embodied as a computer readable storage medium (or multiple computer readable storage media) (e.g., a computer memory, one or more floppy discs, compact discs, optical discs, magnetic tapes, flash memories, circuit configurations in Field Programmable Gate Arrays or other semiconductor devices, or other tangible computer storage medium) encoded with one or more programs that, when executed on one or more computers or other processors, perform methods that implement one or more of the various embodiments described above. The computer readable medium or media can be transportable, such that the program or programs stored thereon can be loaded onto one or more different computers or other processors to implement various ones of the aspects described above. In some embodiments, computer readable media may be non-transitory media.

The terms "program" or "software" are used herein in a generic sense to refer to any type of computer code or set of computer-executable instructions that can be employed to program a computer or other processor to implement various aspects as described above. Additionally, it should be appreciated that according to one aspect, one or more computer programs that when executed perform methods of the present disclosure need not reside on a single computer or processor, but may be distributed in a modular fashion among a number of different computers or processors to implement various aspects of the present disclosure.

Computer-executable instructions may be in many forms, such as program modules, executed by one or more computers or other devices. Generally, program modules include routines, programs, objects, components, data structures, etc. that perform particular tasks or implement particular abstract data types. Typically the functionality of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

When implemented in software, the software code can be executed on any suitable processor or collection of processors, whether provided in a single computer or distributed among multiple computers.

Further, it should be appreciated that a computer may be embodied in any of a number of forms, such as a rack-mounted computer, a desktop computer, a laptop computer, or a tablet computer, as non-limiting examples. Additionally, a computer may be embedded in a device not generally regarded as a computer but with suitable processing capabilities, including a Personal Digital Assistant (PDA), a smartphone, a tablet, or any other suitable portable or fixed electronic device.

Also, a computer may have one or more input and output devices. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computer may receive input information through speech recognition or in other audible formats.

Such computers may be interconnected by one or more networks in any suitable form, including a local area network or a wide area network, such as an enterprise network, and intelligent network (IN) or the Internet. Such networks may be based on any suitable technology and may operate according to any suitable protocol and may include wireless networks, wired networks or fiber optic networks.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

The terms "approximately," "substantially," and "about" may be used to mean within ±20% of a target value in some embodiments, within ±10% of a target value in some embodiments, within ±5% of a target value in some embodiments, within ±2% of a target value in some embodiments. The terms "approximately," "substantially," and "about" may include the target value.

Alternative expressions of the inventive concept are set out in the following numbered clauses.

1. A method for determining a follicular lymphoma (FL) tumor microenvironment (TME) type for a subject having, suspected of having, or at risk of having a follicular lymphoma (FL), the method comprising:

using at least one computer hardware processor to perform:

(a) obtaining RNA expression data for the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells;
(b) generating an FL TME signature for the subject using the RNA expression data, the FL TME signature comprising:

a first gene expression signature comprising first gene group expression scores for respective gene groups in the first plurality of gene groups, and
a second gene expression signature comprising second gene group expression scores for respective gene groups in the second plurality of gene groups associated with B cells,

the generating comprising:

determining the first gene expression signature by determining the first gene group expression scores using the first RNA expression levels, and

determining the second gene expression signature by determining the second gene group expression scores using the second RNA expression levels; and

(c) identifying, using the FL TME signature and from among a plurality of FL TME types, an FL TME type for the subject.

2. The method of clause 1, wherein obtaining the RNA expression data for the subject comprises obtaining bulk sequencing RNA data previously obtained by sequencing a biological sample obtained from the subject.

3. The method of clause 2, wherein the bulk sequencing data comprises at least 1 million reads, at least 5 million reads, at least 10 million reads, at least 20 million reads, at least 50 million reads, or at least 100 million reads.

4. The method of any one of clauses 1 to 3, wherein the sequencing data comprises bulk RNA sequencing (RNA-seq) data, single cell RNA sequencing (scRNA-seq) data, or next generation sequencing (NGS) data.

5. The method of any one of clauses 1 to 3, wherein the sequencing data comprises microarray data.

6. The method of any one of clauses 1 to 5, further comprising:
normalizing the RNA expression data to transcripts per million (TPM) units prior to generating the FL TME signature.

7. The method of any one of clauses 1 to 6, wherein obtaining the RNA expression for the subject comprises sequencing a biological sample obtained from the subject.

8. The method of clause 7, wherein the biological sample comprises lymph node tissue of the subject, optionally wherein the sample comprises tumor tissue of the subject.

9. The method of any one of clauses 1 to 8, wherein the first RNA expression levels for genes in the first plurality of gene groups comprise RNA expression levels for at least three genes from each of at least two of the following gene groups:

(a) MHC II group: *HLA-DRA, HLA-DRB1, HLA-DMA, HLA-DPA1, HLA-DPB1, HLA-DMB, HLA-DQB1, HLA-DQA1, CIITA;*
(b) Effector cells group: *IFNG, GZMA, GZMB, PRF1, GZMK, ZAP70,* GNLY, *FASLG, TBX21, EOMES, CD8A, CD8B;* and
(c) Follicular Dendritic Cells (FDC) group: *PDPN, LTBR, FDCSP, CLU, PRNP, C4A, BST1, SERPINE2, C1S, TNFRSF1A.*

10. The method of any one of clauses 1 to 8, wherein the first RNA expression levels for genes in the first plurality of gene groups further comprise RNA expression levels for at least three genes from each of at least two of the following gene groups:

(d) Treg cells group: *FOXP3, CTLA4, IL10, TNFRSF18, CCR8, IKZF4, IKZF2;*
(e) T helper cells (Follicular B Helper T cells) group: *CXCR5, IL6, ICOS, CD40LG, CD84, IL21, BCL6, MAF, SH2D1A, IL4;*
(f) Effector cells group: *IFNG, GZMA, GZMB, PRF1, GZMK, ZAP70,* GNLY, *FASLG, TBX21, EOMES, CD8A, CD8B;*
(g) Follicular Dendritic Cells (FDC) group: *PDPN, LTBR, FDCSP, CLU, PRNP, C4A, BST1, SERPINE2, CIS, TNFRSF1A;*
(h) Lymphatic endothelial cells group: *CCL21, CXCL12, SOX18, PPPIR13B, FLT4, PROX1, PDPN, LYVE1, FOXC2, CXADR, EDNRB, JAM2, JAM3;*
(i) Proliferation rate group: *MKI67, ESCO2, CETN3, CDK2, CCND1, CCNE1, AURKA, AURKB, E2F1, MYBL2, BUB1, PLK1, CCNB1, MCM2, MCM6;*
(j) M2 group: *IL10, VEGFA, TGFB1, IDO1 , PTGES, MRC1, CSF1, LRP1, ARG1, PTGS1, MSR1, CD163, CSFIR;* and

(k) MHC II group: *HLA-DRA, HLA-DRB1, HLA-DMA, HLA-DPA1, HLA-DPB1, HLA-DMB, HLA-DQB1, HLA-DQA1, CIITA.*

11. The method of clause 9 or 10, wherein the first RNA expression levels for genes in the first plurality of gene groups further comprise RNA expression levels for at least three genes from each of at least two of the following gene groups:

(l) CD4+ T cells group: *CD4, TRAT1, CD40LG, TRAC, CD28;*

(m) CD8+ T cells group: *PRF1, GZMA, CD8B, KLRK1, CD8A, ZAP70, GZMK, TBX21, GZMB, NKG7, EOMES, CD160, KLRC2, TRAT1;* and

(n) Macrophages group: *CMKLR1, IL4I1, OLR1, ADAMDEC1, FPR3, CSFIR, MRC1, SIGLEC1, MS4A7, APOC2, APOE, CD163, SPP1, CCL7, LILRB4, C3AR1, SLAMF8, C1QC, MS4A4A, CLEC10A, C5AR1, RAB7B, CLECSA, CD14, KMO, VSIG4, ADORA3, IL10, CD4, TREM2, ADAP2, CD68, IFI30, MMP9, PLA2G7, MSR1, C1QA, CYBB, CCR1, CD33.*

12. The method of any one of clauses 1 to 11, wherein the second RNA expression levels for genes in the second plurality of gene groups comprises RNA expression levels for at least three genes from each of at least two of the following gene groups associated with B cells:

(a) Naive B cells group: *CD200, CD27, DPPA4, NAAA, XBP1, MNS1, SIGLEC6, PDE8B, BCL2, IRF4, RHOBTB3, CD1A, ENTPD1,* and *KIF18A;*

(b) Centrocyte group: *DHRS9, EGR3, FCER2, DPPA4, ENTPD1, FGD6, DNAJB9, ELL2, ERN1, EIF4E3, AHNAK, and FEZ1;*

(c) Centroblast group: *KANK2, POU2AF1, PDE8B, SLAMF7, TCL1A, RBM47, MNS1, UEVLD, RASGRF1, NDE1, KIF13A, JUN, and NEK2;*

(d) Memory B cells group: *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1;* and/or

(e) Plasmacyte group: *FKBP11, EGR3, EIF4E3, DPPA4, DNER, ELL2, ELOVL6, FNDC3A, DNAJB9, PRDM1, DLGAPS, FGD6, DHRS9, FNDC3B,* and *ZNF677.*

13. The method of any one of clauses 1 to 12, wherein determining the first gene group expression scores comprises: determining a respective gene expression score for each of at least two of the three following gene groups, using, for a particular gene group, first RNA expression levels for at least three genes in the particular gene group to determine the gene expression score for the particular group, the three gene groups including:

(a) MHC II group: *HLA-DRA, HLA-DRB1, HLA-DMA, HLA-DPA1, HLA-DPB1, HLA-DMB, HLA-DQB1, HLA-DQA1, CIITA;*

(b) Effector cells group: *IFNG, GZMA, GZMB, PRF1, GZMK, ZAP70,* GNLY, *FASLG, TBX21, EOMES, CD8A, CD8B;* and

(c) Follicular Dendritic Cells (FDC) group: *PDPN, LTBR, FDCSP, CLU, PRNP, C4A, BST1, SERPINE2, C1S, TNFRSF1A.*

14. The method of cany one of clauses 1 to 12, wherein determining the first gene expression signature further comprises determining a respective gene expression score for each of at least two of the following gene groups, using, for a particular gene group, first RNA expression levels for at least three genes in the particular gene group to determine the gene expression score for the particular group, the gene groups including:

(d) Treg cells group: *FOXP3, CTLA4, IL10, TNFRSF18, CCR8, IKZF4, IKZF2;*

(e) T helper cells (Follicular B Helper T cells) group: *CXCR5, IL6, ICOS, CD40LG, CD84, IL21, BCL6, MAF, SH2D1A, IL4;*

(f) Effector cells group: *IFNG, GZMA, GZMB, PRF1, GZMK, ZAP70,* GNLY, *FASLG, TBX21, EOMES, CD8A, CD8B;*

(g) Follicular Dendritic Cells (FDC) group: *PDPN, LTBR, FDCSP, CLU, PRNP, C4A, BST1, SERPINE2, CIS, TNFRSF1A;*

(h) Lymphatic endothelial cells group: *CCL21, CXCL12, SOX18, PPP1R13B, FLT4, PROX1, PDPN, LYVE1, FOXC2, CXADR, EDNRB, JAM2, JAM3;*

(i) Proliferation rate group: *MKI67, ESCO2, CETN3, CDK2, CCND1, CCNE1, AURKA, AURKB, E2F1, MYBL2, BUB1, PLK1, CCNB1, MCM2, MCM6;*

(j) M2 group: *IL10, VEGFA, TGFB1, IDO1 , PTGES, MRC1, CSF1, LRP1, ARG1, PTGS1, MSR1, CD163, CSFIR;* and
(k) MHC II group: *HLA-DRA, HLA-DRB1, HLA-DMA, HLA-DPA1, HLA-DPB1, HLA-DMB, HLA-DQB1, HLA-DQA1, CIITA.*

15. The method of clause 14, wherein determining the first gene expression signature further comprises determining a respective gene expression score for each of at least two of the following gene groups, using, for a particular gene group, first RNA expression levels for at least three genes in the particular gene group to determine the gene expression score for the particular group, the gene groups including:

(l) CD4$^+$ T cells group: *CD4, TRAT1, CD40LG, TRAC, CD28;*
(m) CD8$^+$ T cells group: *PRF1, GZMA, CD8B, KLRK1, CD8A, ZAP70, GZMK, TBX21, GZMB, NKG7, EOMES, CD160, KLRC2, TRAT1;* and
(n) Macrophages group: *CMKLR1, IL4I1, OLR1, ADAMDEC1, FPR3, CSF1R, MRC1, SIGLEC1, MS4A7, APOC2, APOE, CD163, SPP1, CCL7, LILRB4, C3AR1, SLAMF8, C1QC, MS4A4A, CLEC10A, C5AR1, RAB7B, CLECSA, CD14, KMO, VSIG4, ADORA3, IL10, CD4, TREM2, ADAP2, CD68, IFI30, MMP9, PLA2G7, MSR1, C1QA, CYBB, CCR1, CD33.*

16. The method of any one of clauses 1 to 15,

wherein the first gene group expression scores include a first score for a first gene group in the first plurality of gene groups,
wherein determining the first gene group expression scores comprises determining the first score, using a gene set enrichment analysis (GSEA) technique, from RNA expression levels of at least some genes in the first gene group.

17. The method of clause 16, wherein the first score of the first gene group in the first gene expression signature is determined using a single-sample GSEA (ssGSEA) technique from RNA expression levels for at least some of the genes in one of the following gene groups:

(a) MHC II group: *HLA-DRA, HLA-DRB1*, *HLA-DMA, HLA-DPA1, HLA-DPB1*, *HLA-DMB, HLA-DQB1, HLA-DQA1, CIITA*;
(b) Effector cells group: *IFNG, GZMA, GZMB, PRF1, GZMK, ZAP70,* GNLY, *FASLG, TBX21, EOMES, CD8A, CD8B;* or
(c) Follicular Dendritic Cells (FDC) group: *PDPN, LTBR, FDCSP, CLU, PRNP, C4A, BST1, SERPINE2, CIS, TNFRSF1A.*

18. The method of any one of clauses 1 to 17, wherein determining the second gene expression signature comprises determining a respective gene expression score for each of at least two of the following gene groups associated with B cells including, using, for a particular gene group associated with B cells, second RNA expression levels for at least three genes in the particular gene group associated with B cells to determine the gene expression score for the particular group, the gene groups associated with B cells including:

(a) Naive B cells group: *CD200, CD27, DPPA4, NAAA, XBP1, MNS1, SIGLEC6, PDE8B, BCL2, IRF4, RHOBTB3, CD1A, ENTPD1,* and *KIF18A;*
(b) Centrocyte group: *DHRS9, EGR3, FCER2, DPPA4, ENTPD1, FGD6, DNAJB9, ELL2, ERN1, EIF4E3, AHNAK,* and *FEZ1;*
(c) Centroblast group: *KANK2, POU2AF1, PDE8B, SLAMF7, TCL1A, RBM47, MNS1, UEVLD, RASGRF1, NDE1, KIF13A, JUN, and NEK2;*
(d) Memory B cells group: *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1;* and
(e) Plasmacyte group: *FKBP11, EGR3, EIF4E3, DPPA4, DNER, ELL2, ELOVL6, FNDC3A, DNAJB9, PRDM1, DLGAPS, FGD6, DHRS9, FNDC3B,* and *ZNF677.*

19. The method of any one of clauses 1 to 18, wherein the second plurality of gene groups associated with B cells comprises a first B-cell gene group, wherein determining the second gene expression scores comprises:

determining, using RNA expression levels of at least some genes in the first B-cell gene group and coefficients of a first statistical model associated with the first B-cell gene group, a first score for the first B-cell gene group in the second gene expression signature,

wherein the coefficients of the first statistical model were previously estimated by training the first statistical model to generate, from the RNA expression levels of the at least some genes in the first B-cell gene group, an output indicative of whether the subject is to be associated with the first B-cell gene group.

20. The method of clause 19, wherein determining the first score for the first B-cell gene group comprises:

determining an initial score as a dot product between a vector of the coefficients of the first statistical model and a vector of the RNA expression levels of the at least some of the genes in the first B-cell gene group; and determining the score by adjusting the initial score to compensate for batch effects in a process used to obtain the RNA expression levels from the biological sample.

21. The method of clause 20, wherein adjusting the initial score is performed by median scaling.

22. The method of any one of clauses 1 to 21, wherein the second plurality of gene groups associated with B cells comprises a second B-cell gene group, wherein determining the second gene expression scores comprises:

determining, using RNA expression levels of at least some genes in the second B-cell gene group and coefficients of a second statistical model associated with the second B-cell gene group, a second score for the second B-cell gene group in the second gene expression signature,

wherein the coefficients of the second statistical model were previously estimated by training the second statistical model to generate, from the RNA expression levels of the at least some genes in the second B-cell gene group, an output indicative of whether the subject is to be associated with the second B-cell gene group.

23. The method of any one of clauses 1 to 22, wherein the second plurality of gene groups associated with B cells comprises a third B-cell gene group, wherein determining the second gene expression scores comprises:

determining, using RNA expression levels of at least some genes in the third B-cell gene group and coefficients of a third statistical model associated with the second B-cell gene group, a third score for the third B-cell gene group in the second gene expression signature,

wherein the coefficients of the third statistical model were previously estimated by training the third statistical model to generate, from the RNA expression levels of the at least some genes in the third B-cell gene group, an output indicative of whether the subject is to be associated with the third B-cell gene group.

24. The method of any one of clauses 1 to 23, wherein the second plurality of gene groups associated with B cells comprises a fourth B-cell gene group, wherein determining the second gene expression scores comprises:

determining, using RNA expression levels of at least some genes in the fourth B-cell gene group and coefficients of a fourth statistical model associated with the fourth B-cell gene group, a fourth score for the fourth B-cell gene group in the second gene expression signature,

wherein the coefficients of the fourth statistical model were previously estimated by training the fourth statistical model to generate, from the RNA expression levels of the at least some genes in the fourth B-cell gene group, an output indicative of whether the subject is to be associated with the fourth B-cell gene group.

25. The method of any one of clauses 1 to 24, wherein the second plurality of gene groups associated with B cells comprises a fifth B-cell gene group, wherein determining the second gene expression scores comprises:

determining, using RNA expression levels of at least some genes in the fifth B-cell gene group and coefficients of a fifth statistical model associated with the fifth B-cell gene group, a fifth score for the fifth B-cell gene group in the second gene expression signature,

wherein the coefficients of the fifth statistical model were previously estimated by training the fifth statistical model to generate, from the RNA expression levels of the at least some genes in the fifth B-cell gene group, an output indicative of whether the subject is to be associated with the fifth B-cell gene group.

26. The method of clause any one of clauses 19 to 25,

wherein the first B-cell gene group is the Naive B cells group: *CD200, CD27, DPPA4, NAAA, XBP1, MNS1,*

*SIGLEC6, PDE8B, BCL2, IRF4, RHOBTB3, CD1A, ENTPD1,* and *KIF18A;*
wherein the second B-cell gene group is the Centrocyte group: *DHRS9, EGR3, FCER2, DPPA4, ENTPD1, FGD6, DNAJB9, ELL2, ERN1, EIF4E3, AHNAK,* and *FEZ1;*
wherein the third B-cell gene group is the Centroblast group: *KANK2, POU2AF1, PDE8B, SLAMF7, TCL1A, RBM47, MNS1, UEVLD, RASGRF1, NDE1, KIF13A, JUN,* and *NEK2;*
wherein the fourth B-cell gene group is the Memory B cells group: *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1;* and
wherein the fifth B-cell gene group is the Plasmacyte group: *FKBP 11, EGR3, EIF4E3, DPPA4, DNER, ELL2, ELOVL6, FNDC3A, DNAJB9, PRDM1, DLGAPS, FGD6, DHRS9, FNDC3B,* and *ZNF677.*

27. The method of any one of clauses 19 to 26, wherein each of the first, second, third, fourth, and fifth B-cell gene groups of the second plurality of gene groups is selected from the B-cell gene groups listed in Table 2.

28. The method of any one of clauses 19 to 27, wherein the first statistical model comprises a generalized linear model.

29. The method of clause 28, wherein the generalized linear model comprises a logistic regression model.

30. The method of any one of clauses 19-26, wherein each of the first statistical model, second statistical model, third statistical model, fourth statistical model, and fifth statistical model is a logistic regression model with a respective set of coefficients.

31. The method of any one of clauses 1 to 30, wherein determining the second gene expression scores comprises, for each particular B-cell gene group in the second plurality of gene groups:
determining, using RNA expression levels of genes in the particular B-cell gene group and coefficients of a respective statistical model associated with the particular B-cell gene group, a respective score for the respective B-cell gene group in the second gene expression signature.

32. The method of clause 31, wherein the statistical model comprises a generalized linear model.

33. The method of clause 32, wherein the generalized linear model comprises a logistic regression model.

34. The method of any one of clauses 1 to 33, wherein generating the FL TME signature further comprises performing median scaling on the first gene expression signature and the second gene expression signature.

35. The method of any one of clauses 1 to 17, wherein the second gene expression signature comprises a plurality of BAGS scores for a respective plurality of gene groups, wherein generating the second gene expression signature comprises determining a first BAGS score for a first of the plurality of gene groups, wherein determining the first BAGS score is performed using RNA gene expression levels of at least some of the genes in the first gene group and coefficients of a BAGS classifier associated with the first group.

36. The method of any one of clauses 1 to 35, wherein the plurality of FL TME types is associated with a respective plurality of FL TME signature clusters,
wherein identifying, using the FL TME signature and from among a plurality of FL TME types, the FL TME type for the subject comprises:

associating the FL TME signature of the subject with a particular one of the plurality of FL TME signature clusters; and,
identifying the FL TME type for the subject as the FL TME type corresponding to the particular one of the plurality of FL TME signature clusters to which the FL TME signature of the subject is associated.

37. The method of clause 31, further comprising generating the plurality of FL TME signature clusters, the generating comprising:

obtaining multiple sets of RNA expression data obtained by sequencing biological samples from multiple respective subjects, each of the multiple sets of RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells;

generating multiple FL TME signatures from the multiple sets of RNA expression data, each of the multiple FL TME signatures comprising first gene group expression scores for respective gene groups in the first plurality of gene groups and second gene group expression scores for respective gene groups in the second plurality of gene groups associated with B cells,

the generating comprising, for each particular one of the multiple TME signatures:

determining the first gene group expression scores using the first RNA expression levels in the particular set of RNA expression data from which the particular one TME signature is being generated, and determining the second gene group expression scores using the second RNA expression levels in the particular set of RNA expression data form which the particular one TME signature is being generated; and

clustering the multiple TME signatures to obtain the plurality of FL TME signature clusters.

38. The method of clause 37, wherein the clustering is performed using a clustering algorithm.

39. The method of clause 38, wherein the clustering algorithm is a dense clustering algorithm, spectral clustering algorithm, k-means clustering algorithm, hierarchical clustering algorithm, and/or an agglomerative clustering algorithm.

40. The method of any one of clauses 36 to 39, further comprising:
updating the plurality of FL TME signature clusters using the FL TME signature of the subject, wherein the FL TME signature of the subject is one of a threshold number FL TME signatures for a threshold number of subjects, wherein when the threshold number of FL TME signatures is generated the FL TME signature clusters are updated.

41. The method of clause 40, wherein the threshold number of FL TME signatures is at least 50, at least 75, at least 100, at least 200, at least 500, at least 1000, or at least 5000 FL TME signatures.

42. The method of clause 41, wherein the updating is performed using a clustering algorithm.

43. The method of clause 42, wherein the clustering algorithm is a dense clustering algorithm, spectral clustering algorithm, k-means clustering algorithm, hierarchical clustering algorithm, and/or an agglomerative clustering algorithm.

44. The method of any one of clauses 40 to 43, further comprising:
determining an FL TME type of a second subject, wherein the FL TME type of the second subject is identified using the updated FL TME signature clusters, wherein the identifying comprises:

determining an FL TME signature of the second subject from RNA expression data obtained by sequencing a biological sample obtained from the second subject;
associating the FL TME signature of the second subject with a particular one of the plurality of the updated FL TME signature clusters; and
identifying the FL TME type for the second subject as the FL TME type corresponding to the particular one of the plurality of updated FL TME signature clusters to which the FL TME signature of the second subject is associated.

45. The method of any one of clauses 1 to 44, wherein the plurality of a plurality of FL TME types comprises a Normal-like type, a Plasma-cell (PC)-like type, a Light Zone (LZ)-like type, and a Dark Zone (DZ)-like type.

46. The method of any one of clauses 1 to 45, wherein the FL TME signature further comprises a third gene expression signature, wherein the third gene expression signature comprises one or more PROGENy signatures.

47. The method of clause 46, wherein the one or more PROGENy signatures comprise NF-kB and/or PI3K PROGENy signatures.

48. The method of any one of clauses 1 to 47, further comprising identifying the subject as not having transformed follicular lymphoma (tFL) when the identified FL-TME type for the subject is the Normal-like type.

49. The method of any one of clauses 1 to 48, further comprising:

identifying the subject as having a high risk of progression and/or an increased risk of lacking response to R-CHOP when the identified FL-TME type for the subject is the DZ-like type.

50. The method of any one of clauses 1 to 49, further comprising:

identifying one or more anti-cancer therapies for the subject based upon the identified FL-TME type for the subject; and
administering the one or more identified anti-cancer therapies to the subject.

51. The method of clause 50, wherein the one or more anti-cancer therapies comprises rituximab, cyclophosphamide, doxorubicin hydrochloride, vincristine sulfate, and prednisone (R-CHOP) when the subject is identified as having an FL TME type other than DZ-like type.

52. A system, comprising:

at least one computer hardware processor; and
at least one computer-readable storage medium storing processor-executable instructions that, when executed by the at least one computer hardware processor, cause the at least one computer hardware processor to perform a method for determining a follicular lymphoma (FL) tumor microenvironment (TME) type for a subject having, suspected of having, or at risk of having a follicular lymphoma (FL), the method comprising:

(a) obtaining RNA expression data for the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells;
(b) generating an FL TME signature for the subject using the RNA expression data, the FL TME signature comprising:

a first gene expression signature comprising first gene group expression scores for respective gene groups in the first plurality of gene groups, and
a second gene expression signature comprising second gene group expression scores for respective gene groups in the second plurality of gene groups associated with B cells,

the generating comprising:

determining the first gene expression signature by determining the first gene group expression scores using the first RNA expression levels, and
determining the second gene expression signature by determining the second gene group expression scores using the second RNA expression levels; and

(c) identifying, using the FL TME signature and from among a plurality of FL TME types, an FL TME type for the subject.

53. At least one computer-readable storage medium storing processor-executable instructions that, when executed by at least one computer hardware processor, cause the at least one computer hardware processor to perform a method for determining a follicular lymphoma (FL) tumor microenvironment (TME) type for a subject having, suspected of having, or at risk of having a follicular lymphoma (FL), the method comprising:

(a) obtaining RNA expression data for the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells;
(b) generating an FL TME signature for the subject using the RNA expression data, the FL TME signature comprising:

a first gene expression signature comprising first gene group expression scores for respective gene groups in the first plurality of gene groups, and
a second gene expression signature comprising second gene group expression scores for respective gene groups in the second plurality of gene groups associated with B cells,

the generating comprising:

determining the first gene expression signature by determining the first gene group expression scores using the first RNA expression levels, and
determining the second gene expression signature by determining the second gene group expression scores using the second RNA expression levels; and

(c) identifying, using the FL TME signature and from among a plurality of FL TME types, an FL TME type for the subject.

54. A method for treating follicular lymphoma, the method comprising administering one or more therapeutic agents to a subject identified as having a particular FL TME type, wherein the FL TME type of the subject has been identified by method comprising:
using at least one computer hardware processor to perform:

(a) obtaining RNA expression data for the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells;
(b) generating an FL TME signature for the subject using the RNA expression data, the FL TME signature comprising:

a first gene expression signature comprising first gene group expression scores for respective gene groups in the first plurality of gene groups, and
a second gene expression signature comprising second gene group expression scores for respective gene groups in the second plurality of gene groups associated with B cells,

the generating comprising:

determining the first gene expression signature by determining the first gene group expression scores using the first RNA expression levels, and
determining the second gene expression signature by determining the second gene group expression scores using the second RNA expression levels; and

(c) identifying, using the FL TME signature and from among a plurality of FL TME types, an FL TME type for the subject.

55. The method of clause 54, wherein the subject has been identified as having an FL TME type selected from a Normal-like type, a PC-like type, a Light Zone (LZ)-like type, and a Dark Zone (DZ)-like type.

56. The method of clause 54 or 55, wherein the therapeutic agent comprises R-CHOP when the subject has been identified as having a Normal-like type, a PC-like type, or a Light Zone (LZ)-like type.

57. The method of clause 56, wherein the R-CHOP is administered to the subject on more than one occasion, optionally wherein the R-CHOP is administered to the subject on between 3 and 6 occasions.

58. The method of clause 56, wherein the therapeutic agent is not R-CHOP when the subject has been identified as having a Dark zone-like type.

**Claims**

1. A method for determining a follicular lymphoma (FL) tumor microenvironment (TME) type for a subject having, suspected of having, or at risk of having a follicular lymphoma (FL), the method comprising:
using at least one computer hardware processor to perform:

(a) obtaining RNA expression data for the subject, wherein the RNA expression data has been obtained from a biological sample comprising lymph node tissue of the subject or a biological sample comprising tumor cells of the subject, the RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells;

(b) generating an FL TME signature for the subject using the RNA expression data, the FL TME signature comprising:

a first gene expression signature comprising first gene group expression scores for respective gene groups in the first plurality of gene groups, and

a second gene expression signature comprising second gene group expression scores for respective gene groups in the second plurality of gene groups associated with B cells,

the generating comprising:

determining the first gene expression signature by determining the first gene group expression scores using the first RNA expression levels, and

determining the second gene expression signature by determining the second gene group expression scores using the second RNA expression levels; and

(c) identifying, using the FL TME signature and from among a plurality of FL TME types, an FL TME type for the subject, wherein the plurality of FL TME types is associated with a respective plurality of FL TME signature clusters, and wherein identifying, using the FL TME signature and from among a plurality of FL TME types, the FL TME type for the subject comprises:

associating the FL TME signature of the subject with a particular one of the plurality of FL TME signature clusters; and,

identifying the FL TME type for the subject as the FL TME type corresponding to the particular one of the plurality of FL TME signature clusters to which the FL TME signature of the subject is associated;

wherein the plurality of FL TME signature clusters have been generated using a method comprising:

obtaining multiple sets of RNA expression data obtained from samples from multiple respective subjects, each sample selected from a lymph node tissue sample or sample comprising tumor cells, each of the multiple sets of RNA expression data indicating first RNA expression levels for genes in a first plurality of gene groups and second RNA expression levels for genes in a second plurality of gene groups different from the first plurality of gene groups, wherein genes in the second plurality of gene groups are associated with B cells;

generating multiple FL TME signatures from the multiple sets of RNA expression data, each of the multiple FL TME signatures comprising first gene group expression scores for respective gene groups in the first plurality of gene groups and second gene group expression scores for respective gene groups in the second plurality of gene groups associated with B cells,

the generating comprising, for each particular one of the multiple TME signatures:

determining the first gene group expression scores using the first RNA expression levels in the particular set of RNA expression data from which the particular one TME signature is being generated, and

determining the second gene group expression scores using the second RNA expression levels in the particular set of RNA expression data form which the particular one TME signature is being generated; and

clustering the multiple TME signatures to obtain the plurality of FL TME signature clusters.

2. The method of claim 1, wherein obtaining the RNA expression data for the subject comprises obtaining bulk sequencing RNA data previously obtained by sequencing a biological sample obtained from the subject, or wherein the sequencing data comprises microarray data, optionally wherein the bulk sequencing data comprises at least 1 million reads, at least 5 million reads, at least 10 million reads, at least 20 million reads, at least 50 million reads, or at least 100 million reads, and/or wherein the sequencing data comprises bulk RNA sequencing (RNA-seq) data, single cell RNA sequencing (scRNA-seq) data, or next generation sequencing (NGS) data.

3. The method of claim 1 or claim 2, wherein the method further comprises normalizing the RNA expression data to transcripts per million (TPM) units prior to generating the FL TME signature; and/or

wherein generating the FL TME signature further comprises performing median scaling on the first gene expression signature and the second gene expression signature and/or
wherein determining the first score for the first B-cell gene group comprises:

determining an initial score as a dot product between a vector of the coefficients of the first statistical model and a vector of the RNA expression levels of the at least some of the genes in the first B-cell gene group; and determining the score by adjusting, optionally by median scaling, the initial score to compensate for batch effects in a process used to obtain the RNA expression levels from the biological sample; and/or

wherein the first statistical model comprises a generalized linear model, optionally a logistic regression model.

4. The method of any one of claims 1 to 3, wherein obtaining the RNA expression for the subject comprises sequencing a biological sample obtained from the subject, and/or wherein the biological sample comprises tumor tissue of the subject, and/or wherein the plurality of a plurality of FL TME types comprises a Normal-like type, a Plasma-cell (PC)-like type, a Light Zone (LZ)-like type, and a Dark Zone (DZ)-like type.

5. The method of any one of claims 1 to 4, wherein the first RNA expression levels for genes in the first plurality of gene groups comprise RNA expression levels for:

at least three genes from each of at least two of the following gene groups:

(a) MHC II group: *HLA-DRA, HLA-DRB1, HLA-DMA, HLA-DPA1, HLA-DPBI, HLA-DMB, HLA-DQB1, HLA-DQA1, CIITA*;
(b) Effector cells group: *IFNG, GZMA, GZMB, PRF1, GZMK, ZAP70,* GNLY, *FASLG, TBX21, EOMES, CD8A, CD8B;* and
(c) Follicular Dendritic Cells (FDC) group: *PDPN, LTBR, FDCSP, CLU, PRNP, C4A, BST1, SERPINE2, CIS, TNFRSF1A;* and/or

at least three genes from each of at least two of the following gene groups:

(d) Treg cells group: *FOXP3, CTLA4, IL10, TNFRSF18, CCR8, IKZF4, IKZF2;*
(e) T helper cells (Follicular B Helper T cells) group: *CXCRS, IL6, ICOS, CD40LG, CD84, IL21, BCL6, MAF, SH2D1A, IL4;*
(f) Effector cells group: *IFNG, GZMA, GZMB, PRF1, GZMK, ZAP70,* GNLY, *FASLG, TBX21, EOMES, CD8A, CD8B;*
(g) Follicular Dendritic Cells (FDC) group: *PDPN, LTBR, FDCSP, CLU, PRNP, C4A, BST1, SERPINE2, CIS, TNFRSF1A;*
(h) Lymphatic endothelial cells group: *CCL21, CXCL12, SOX18, PPP1R13B, FLT4, PROX1, PDPN, LYVE1, FOXC2, CXADR, EDNRB, JAM2, JAM3;*
(i) Proliferation rate group: *MKI67, ESCO2, CETN3, CDK2, CCND1, CCNE1, AURKA, A URKB, E2F1, MYBL2, BUB1, PLK1, CCNB1, MCM2, MCM6;*
(j) M2 group: *IL10, VEGFA, TGFB1, IDO1, PTGES, MRC1, CSF1, LRP1, ARG1, PTGS1, MSR1, CD163, CSFIR;* and
(k) MHC II group: *HLA-DRA, HLA-DRB1, HLA-DMA, HLA-DPA1, HLA-DPB1, HLA-DMB, HLA-DQB1, HLA-DQA1, CIITA;* and/or

at least three genes from each of at least two of the following gene groups:

(l) CD4⁺ T cells group: *CD4, TRAT1, CD40LG, TRAC, CD28;*
(m) CD8⁺ T cells group: *PRF1, GZMA, CD8B, KLRK1, CD8A, ZAP70, GZMK, TBX21, GZMB, NKG7, EOMES, CD160, KLRC2, TRAT1;* and
(n) Macrophages group: *CMKLR1, IL4I1, OLR1, ADAMDEC1, FPR3, CSF1R, MRC1, SIGLEC1, MS4A7, APOC2, APOE, CD163, SPP1, CCL7, LILRB4, C3AR1, SLAMF8, C1QC, MS4A4A, CLEC10A, C5AR1, RAB7B, CLECSA, CD14, KMO, VSIG4, ADORA3, IL10, CD4, TREM2, ADAP2, CD68, IFI30, MMP9, PLA2G7, MSR1, C1QA, CYBB, CCR1, CD33.*

**6.** The method of any one of claims 1 to 5, wherein the second RNA expression levels for genes in the second plurality of gene groups comprises RNA expression levels for at least three genes from each of at least two of the following gene groups associated with B cells:

(a) Naive B cells group: *CD200, CD27, DPPA4, NAAA, XBP1, MNS1, SIGLEC6, PDE8B, BCL2, IRF4, RHOBTB3, CD1A, ENTPD1,* and *KIF18A;*
(b) Centrocyte group: *DHRS9, EGR3, FCER2, DPPA4, ENTPD1, FGD6, DNAJB9, ELL2, ERN1, EIF4E3, AHNAK, and FEZ1;*
(c) Centroblast group: *KANK2, POU2AF1, PDE8B, SLAMF7, TCL1A, RBM47, MNS1, UEVLD, RASGRF1, NDE1, KIF13A, JUN, and NEK2;*
(d) Memory B cells group: *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1;* and/or
(e) Plasmacyte group: *FKBP11, EGR3, EIF4E3, DPPA4, DNER, ELL2, ELOVL6, FNDC3A, DNAJB9, PRDM1, DLGAPS, FGD6, DHRS9, FNDC3B,* and *ZNF677.*

**7.** The method of any one of claims 1 to 6, wherein determining the first gene group expression scores comprises: determining a respective gene expression score for each of at least two of the three following gene groups, using, for a particular gene group, first RNA expression levels for at least three genes in the particular gene group to determine the gene expression score for the particular group, the three gene groups including:

(a) MHC II group: *HLA-DRA, HLA-DRB1, HLA-DMA, HLA-DPA1, HLA-DPBI, HLA-DMB, HLA-DQB1, HLA-DQA1, CIITA;*
(b) Effector cells group: *IFNG, GZMA, GZMB, PRF1, GZMK, ZAP70,* GNLY, *FASLG, TBX21, EOMES, CD8A, CD8B;* and
(c) Follicular Dendritic Cells (FDC) group: *PDPN, LTBR, FDCSP, CLU, PRNP, C4A, BST1, SERPINE2, C1S, TNFRSF1A.*

**8.** The method of cany one of claims 1 to 7, wherein determining the first gene expression signature further comprises determining a respective gene expression score for each of at least two of the following gene groups, using, for a particular gene group, first RNA expression levels for at least three genes in the particular gene group to determine the gene expression score for the particular group, the gene groups including:

(d) Treg cells group: *FOXP3, CTLA4, IL10, TNFRSF18, CCR8, IKZF4, IKZF2;*
(e) T helper cells (Follicular B Helper T cells) group: *CXCRS, IL6, ICOS, CD40LG, CD84, IL21, BCL6, MAF, SH2D1A, IL4;*
(f) Effector cells group: *IFNG, GZMA, GZMB, PRF1, GZMK, ZAP70,* GNLY, *FASLG, TBX21, EOMES, CD8A, CD8B;*
(g) Follicular Dendritic Cells (FDC) group: *PDPN, LTBR, FDCSP, CLU, PRNP, C4A, BST1, SERPINE2, CIS, TNFRSF1A;*
(h) Lymphatic endothelial cells group: *CCL21, CXCL12, SOX18, PPP1R13B, FLT4, PROX1, PDPN, LYVE1, FOXC2, CXADR, EDNRB, JAM2, JAM3;*
(i) Proliferation rate group: *MKI67, ESCO2, CETN3, CDK2, CCND1, CCNE1, AURKA, AURKB, E2F1, MYBL2, BUB1, PLK1, CCNB1, MCM2, MCM6;*
(j) M2 group: *IL10, VEGFA, TGFB 1, IDO1, PTGES, MRC1, CSF1, LRP 1, ARG 1, PTGS1, MSR1, CD163, CSF1R;* and
(k) MHC II group: *HLA-DRA, HLA-DRB1, HLA-DMA, HLA-DPA1, HLA-DPB1, HLA-DMB, HLA-DQB1, HLA-DQA1, CIITA;* optionally wherein determining the first gene expression signature further comprises determining a respective gene expression score for each of at least two of the following gene groups, using, for a particular gene group, first RNA expression levels for at least three genes in the particular gene group to determine the gene expression score for the particular group, the gene groups including:
(l) CD4⁺ T cells group: *CD4, TRAT1, CD40LG, TRAC, CD28;*
(m) CD8⁺ T cells group: *PRF1, GZMA, CD8B, KLRK1, CD8A, ZAP70, GZMK, TBX21, GZMB, NKG7, EOMES, CD160, KLRC2, TRAT1;* and
(n) Macrophages group: *CMKLR1, IL4I1, OLR1, ADAMDEC1, FPR3, CSF1R, MRC1, SIGLEC1, MS4A7, APOC2, APOE, CD163, SPP1, CCL7, LILRB4, C3AR1, SLAMF8, C1QC, MS4A4A, CLEC10A, C5AR1, RAB7B, CLECSA, CD14, KMO, VSIG4, ADORA3, IL10, CD4, TREM2, ADAP2, CD68, IFI30, MMP9, PLA2G7, MSR1, C1QA, CYBB, CCR1, CD33.*

9. The method of any one of claims 1 to 8,

wherein the first gene group expression scores include a first score for a first gene group in the first plurality of gene groups,
wherein determining the first gene group expression scores comprises determining the first score, using a gene set enrichment analysis (GSEA) technique, from RNA expression levels of at least some genes in the first gene group, optionally wherein the first score of the first gene group in the first gene expression signature is determined using a single-sample GSEA (ssGSEA) technique from RNA expression levels for at least some of the genes in one of the following gene groups:

(a) MHC II group: *HLA-DRA, HLA-DRB1, HLA-DMA, HLA-DPA1, HLA-DPB1, HLA-DMB, HLA-DQB1, HLA-DQA1, CIITA*;
(b) Effector cells group: *IFNG, GZMA, GZMB, PRF1, GZMK, ZAP70,* GNLY, *FASLG, TBX21, EOMES, CD8A, CD8B;* or
(c) Follicular Dendritic Cells (FDC) group: *PDPN, LTBR, FDCSP, CLU, PRNP, C4A, BST1, SERPINE2, C1S, TNFRSF1A.*

10. The method of any one of claims 1 to 9, wherein determining the second gene expression signature comprises determining a respective gene expression score for each of at least two of the following gene groups associated with B cells including, using, for a particular gene group associated with B cells, second RNA expression levels for at least three genes in the particular gene group associated with B cells to determine the gene expression score for the particular group, the gene groups associated with B cells including:

(a) Naive B cells group: *CD200, CD27, DPPA4, NAAA, XBP1, MNS1, SIGLEC6, PDE8B, BCL2, IRF4, RHOBTB3, CD1A, ENTPD1,* and *KIF18A;*
(b) Centrocyte group: *DHRS9, EGR3, FCER2, DPPA4, ENTPD1, FGD6, DNAJB9, ELL2, ERN1, EIF4E3, AHNAK, and FEZ1;*
(c) Centroblast group: *KANK2, POU2AF1, PDE8B, SLAMF7, TCL1A, RBM47, MNS1, UEVLD, RASGRF1, NDE1, KIF13A, JUN, and NEK2;*
(d) Memory B cells group: *SLC39A8, IL21R, CCR1, TCL1A, BHLHE41, NAAA, ITGAM, EGR3, FCGR2A, RHOBTB3, DPPA4, CD27, RCBTB2, ELOVL6,* and *ABCB1;* and
(e) Plasmacyte group: *FKBP11, EGR3, EIF4E3, DPPA4, DNER, ELL2, ELOVL6, FNDC3A, DNAJB9, PRDM1, DLGAPS, FGD6, DHRS9, FNDC3B,* and *ZNF677.*

11. The method of any one of claims 1 to 10, wherein the second plurality of gene groups associated with B cells comprises:

(i) a first B-cell gene group, wherein determining the second gene expression scores comprises:

determining, using RNA expression levels of at least some genes in the first B-cell gene group and coefficients of a first statistical model associated with the first B-cell gene group, a first score for the first B-cell gene group in the second gene expression signature,
wherein the coefficients of the first statistical model were previously estimated by training the first statistical model to generate, from the RNA expression levels of the at least some genes in the first B-cell gene group, an output indicative of whether the subject is to be associated with the first B-cell gene group; and/or

(ii) a second B-cell gene group, wherein determining the second gene expression scores comprises:
determining, using RNA expression levels of at least some genes in the second B-cell gene group and coefficients of a second statistical model associated with the second B-cell gene group, a second score for the second B-cell gene group in the second gene expression signature, wherein the coefficients of the second statistical model were previously estimated by training the second statistical model to generate, from the RNA expression levels of the at least some genes in the second B-cell gene group, an output indicative of whether the subject is to be associated with the second B-cell gene group; and/or
(iii) a third B-cell gene group, wherein determining the second gene expression scores comprises:
determining, using RNA expression levels of at least some genes in the third B-cell gene group and coefficients of a third statistical model associated with the second B-cell gene group, a third score for the third B-cell gene group in the second gene expression signature,
wherein the coefficients of the third statistical model were previously estimated by training the third statistical model to generate, from the RNA expression levels of the at least some genes in the third B-cell gene group, an output indicative of whether the subject is to be associated with the third B-cell gene group; and/or

(iv) a fourth B-cell gene group, wherein determining the second gene expression scores comprises: determining, using RNA expression levels of at least some genes in the fourth B-cell gene group and coefficients of a fourth statistical model associated with the fourth B-cell gene group, a fourth score for the fourth B-cell gene group in the second gene expression signature, wherein the coefficients of the fourth statistical model were previously estimated by training the fourth statistical model to generate, from the RNA expression levels of the at least some genes in the fourth B-cell gene group, an output indicative of whether the subject is to be associated with the fourth B-cell gene group; and/or

(v) a fifth B-cell gene group, wherein determining the second gene expression scores comprises: determining, using RNA expression levels of at least some genes in the fifth B-cell gene group and coefficients of a fifth statistical model associated with the fifth B-cell gene group, a fifth score for the fifth B-cell gene group in the second gene expression signature,

wherein the coefficients of the fifth statistical model were previously estimated by training the fifth statistical model to generate, from the RNA expression levels of the at least some genes in the fifth B-cell gene group, an output indicative of whether the subject is to be associated with the fifth B-cell gene group.

12. The method of any one of claims 1 to 11, further comprising:
identifying the subject as not having transformed follicular lymphoma (tFL) when the identified FL-TME type for the subject is the Normal-like type; and/or

identifying the subject as having a high risk of progression and/or an increased risk of lacking response to R-CHOP when the identified FL-TME type for the subject is the DZ-like type; and/or
identifying one or more anti-cancer therapies for the subject based upon the identified FL-TME type for the subject, optionally wherein the one or more anti-cancer therapies comprises rituximab, cyclophosphamide, doxorubicin hydrochloride, vincristine sulfate, and prednisone (R-CHOP) when the subject is identified as having an FL TME type other than DZ-like type and/or wherein the one or more anti-cancer therapies is not R-CHOP when the subject has been identified as having a Dark zone-like type.

13. A system, comprising:

at least one computer hardware processor; and
at least one computer-readable storage medium storing processor-executable instructions that, when executed by the at least one computer hardware processor, cause the at least one computer hardware processor to perform a method for determining a follicular lymphoma (FL) tumor microenvironment (TME) type for a subject having, suspected of having, or at risk of having a follicular lymphoma (FL), as described in any of claims 1 to 13.

14. At least one computer-readable storage medium storing processor-executable instructions that, when executed by at least one computer hardware processor, cause the at least one computer hardware processor to perform a method for determining a follicular lymphoma (FL) tumor microenvironment (TME) type for a subject having, suspected of having, or at risk of having a follicular lymphoma (FL), as described in any of claims 1 to 13.

15. An anti-cancer therapy comprising rituximab, cyclophosphamide, doxorubicin hydrochloride, vincristine sulfate, and prednisone (R-CHOP) for use in a method of treating follicular lymphoma, the method comprising administering one or more therapeutic agents to a subject identified as having a particular FL TME type, wherein the FL TME type of the subject has been identified using the method of any of claims 1 to 19 and is selected from a Normal-like, a PC-like, or a Light Zone (LZ)-like FL TME type., optionally wherein the R-CHOP is administered to the subject on more than one occasion, optionally wherein the R-CHOP is administered to the subject on between 3 and 6 occasions.

100

Obtaining Sequencing Data — 102

↓

Processing Sequencing Data to Obtain RNA Expression Data — 104

↓

Generating Follicular Lymphoma (FL) Tumor Microenvironment (TME) Signature using RNA Expression Levels — 106

↓

Determining First Gene Expression Signature — 108

↓

Determining Second Gene Expression Signature Associated with B Cells — 110

↓

Generating FL TME Signature — 112

↓

Identifying FL TME Type using FL TME Signature — 114

↓

Identifying one or more anti-cancer therapies for the subject based upon the identified FL-TME type for the subject — 116

↓

Administering the identified one or more anti-cancer therapies to the subject — 118

FIG. 1

104

| Obtaining bulk sequencing data | 200 |

↓

| Normalizing bulk sequencing data to TPM units | 202 |

↓

| Log transformation of TPM units | 204 |

FIG. 2

108

## Determining First Gene Expression Signature

| 300 | 310 | 320 |
|---|---|---|

RNA Expression Data for Gene Group 1 → GSEA → Gene Enrichment Score 1

RNA Expression Data for Gene Group 2 → GSEA → Gene Enrichment Score 2

RNA Expression Data for Gene Group 3 → GSEA → Gene Enrichment Score 3

RNA Expression Data for Gene Group 4 → GSEA → Gene Enrichment Score 4

RNA Expression Data for Gene Group 5 → GSEA → Gene Enrichment Score 5

RNA Expression Data for Gene Group 6 → GSEA → Gene Enrichment Score 6

RNA Expression Data for Gene Group 7 → GSEA → Gene Enrichment Score 7

RNA Expression Data for Gene Group 8 → GSEA → Gene Enrichment Score 8

## FIG. 3

110

Determining Second Gene Expression Signature for Gene Groups Associated with B cells

400

410

420

| RNA Expression Data for Gene Group 1 | → | Coefficients of Statistical Model 1 | → | Score 1 |

| RNA Expression Data for Gene Group 2 | → | Coefficients of Statistical Model 2 | → | Score 2 |

| RNA Expression Data for Gene Group 3 | → | Coefficients of Statistical Model 3 | → | Score 3 |

| RNA Expression Data for Gene Group 4 | → | Coefficients of Statistical Model 4 | → | Score 4 |

| RNA Expression Data for Gene Group 5 | → | Coefficients of Statistical Model 5 | → | Score 5 |

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63124617
- US 20200273543 A

**Non-patent literature cited in the description**

- **LIU et al.** *Annals of Lymphoma,* June 2021, vol. 5, 11
- **DYBKAER et al.** *J Clin Oncol.,* 20 April 2015, vol. 33 (12), 1379-1388
- **VAUGHT et al.** *Cancer Epidemiol Biomarkers Prev,* February 2012, vol. 21 (2), 253-5
- **VAUGHT ; HENDERSON.** *IARC Sci Publ,* 2011, (163), 23-42
- **NICOLAS L BRAY ; HAROLD PIMENTEL ; PÁLL MELSTED ; LIOR PACHTER.** Near-optimal probabilistic RNA-seq quantification. *Nature Biotechnology,* 2016, vol. 34, 525-527
- *Bioinformatics.,* 12 February 2004, vol. 20 (3), 307-15
- **RITCHIE ME ; PHIPSON B ; WU D ; HU Y ; LAW CW ; SHI W ; SMYTH GK.** limma powers differential expression analyses for RNA-sequencing and microarray studies. *Nucleic Acids Res.,* 20 April 2015, vol. 43 (7), e47.20, https://doi.org/10.1093/nar/gkv007
- **WAGNER et al.** *Theory Biosci.,* 2012, vol. 131, 281-285
- **WU J.** *Gentry RlwcfJMJ,* 2021
- **BARBIE et al.** *Nature,* 05 November 2009, vol. 462 (7269), 108-112
- **DYBKÆR K et al.** Diffuse large B-cell lymphoma classification system that associates normal B-cell subset phenotypes with prognosis. *J Clin Oncol.,* 2015, vol. 33 (12), 1379-1388
- **CUNNINGHAM et al.** *Lancet,* 25 May 2013, vol. 381 (9880), 1817-26
- **WU J.** gcrma: Background Adjustment Using Sequence Information. R package version 2.66.0. *Gentry RlwcfJMJ,* 2021
- **DYBKÆR K et al.** Diffuse large B-cell lymphoma classification system that associates normal B-cell subset phenotypes with prognosis. *J Clin Oncol.,* 2015, vol. 33 (12), 1379-1388
- **KE, G ; MENG, Q. ; FINLEY, T. ; WANG, T. ; CHEN, W ; MA, W. ; LIU, T.-Y.** Lightgbm: A highly efficient gradient boosting decision tree. *Advances in Neural Information Processing Systems,* 2017, vol. 30, 3146-3154